# EUROPEAN PATENT APPLICATION

(11) **EP 4 847 502 A1**
(43) Date of publication of application: **02.12.2026**
(21) Application number: 25745282.1
(22) Date of filing: 21.01.2025
(51) Int. Cl.: C07D 403/10, H10K 85/60, H10K 50/11, C09K 11/06, H10K 101/00

(54) **COMPOUND FOR ORGANIC OPTOELECTRONIC DIODE, COMPOSITION FOR ORGANIC OPTOELECTRONIC DIODE, ORGANIC OPTOELECTRONIC DIODE, AND DISPLAY DEVICE**

(30) Priority: 25.01.2024 KR 20240011822
(71) Applicant: SAMSUNG SDI CO., LTD., Yongin-si, Gyeonggi-do 17084 (KR)
(72) Inventor: LEE, Byoungkwan, Suwon-si, Gyeonggi-do 16678 (KR); KANG, Dong Min, Suwon-si, Gyeonggi-do 16678 (KR); YANG, Yongtak, Suwon-si, Gyeonggi-do 16678 (KR); JUNG, Ho Kuk, Suwon-si, Gyeonggi-do 16678 (KR); KIM, Hyung Sun, Suwon-si, Gyeonggi-do 16678 (KR); LEE, Yoonman, Suwon-si, Gyeonggi-do 16678 (KR); KIM, Changwoo, Suwon-si, Gyeonggi-do 16678 (KR); KIM, Byungku, Suwon-si, Gyeonggi-do 16678 (KR); SEO, Hansol, Suwon-si, Gyeonggi-do 16678 (KR); NOH, Wonho, Suwon-si, Gyeonggi-do 16678 (KR); LEE, Jihee, Suwon-si, Gyeonggi-do 16678 (KR); LEE, Hyungyu, Suwon-si, Gyeonggi-do 16678 (KR); JANG, Kipo, Suwon-si, Gyeonggi-do 16678 (KR)
(74) Representative: Michalski Hüttermann & Partner mbB
(86) International application number: PCT/KR2025/099108
(87) International publication number: WO 2025/159617

(57) **Abstract**

Disclosed are a compound for an organic optoelectronic device represented by Chemical Formula 1, an organic optoelectronic device including the same, an organic optoelectronic device, and a display device.

Details of Chemical Formula 1 are as defined in the specification.

## Description

### [Technical Field]

A compound for an organic optoelectronic device, a composition for an organic optoelectronic device, an organic optoelectronic device, and a display device are disclosed.

### [Background Art]

An organic optoelectronic device (organic optoelectronic diode) is a device capable of converting electrical energy and optical energy to each other.

Organic optoelectronic devices may be largely divided into two types according to a principle of operation. One is a photoelectric device that generates electrical energy by separating excitons formed by light energy into electrons and holes, and transferring the electrons and holes to different electrodes, respectively and the other is light emitting device that generates light energy from electrical energy by supplying voltage or current to the electrodes.

Examples of the organic optoelectronic device include an organic photoelectric device, an organic light emitting diode, an organic solar cell, and an organic photoconductor drum.

Among them, organic light emitting diodes (OLEDs) are attracting much attention in recent years due to increasing demands for flat panel display devices. The organic light emitting diode is a device that converts electrical energy into light, and the performance of the organic light emitting diode is greatly influenced by an organic material between electrodes.

### [Disclosure]

### [Technical Problem]

An embodiment provides a compound for an organic optoelectronic device capable of lowering the driving voltage and implementing high-efficiency and long life-span organic optoelectronic device.

Another embodiment provides a composition for an organic optoelectronic device including the compound for the organic optoelectronic device.

Another embodiment provides an organic optoelectronic device including the compound for the organic optoelectronic device or composition for the organic optoelectronic device.

Another embodiment provides a display device including the organic optoelectronic device.

### [Technical Solution]

According to an embodiment, a compound for an organic optoelectronic device represented by Chemical Formula 1 is provided.

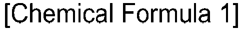

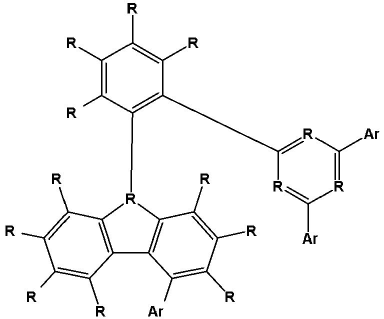

In Chemical Formula 1,
Ar¹ and Ar² are each independently a substituted or unsubstituted C6 to C30 aryl group,
Ar³ is a substituted or unsubstituted C6 to C30 aryl group,
at least one of Ar¹ to Ar³ is a substituted or unsubstituted C10 to C30 aryl group,
R¹ to R⁷ are each independently hydrogen, deuterium, a cyano group, a halogen, a substituted or unsubstituted C1 to C30 alkyl group, or a substituted or unsubstituted C6 to C30 aryl group, and
R⁸ to R¹¹ are each independently hydrogen, deuterium, a cyano group, a halogen, or a substituted or unsubstituted C1 to C30 alkyl group.

According to another embodiment, a composition for an organic optoelectronic device including a first compound and a second compound is provided.

The first compound may be the aforementioned compound for the organic optoelectronic device, and the second compound may be represented by Chemical Formula 2; a combination of Chemical Formula 3 and Chemical Formula 4; or Chemical Formula 5.

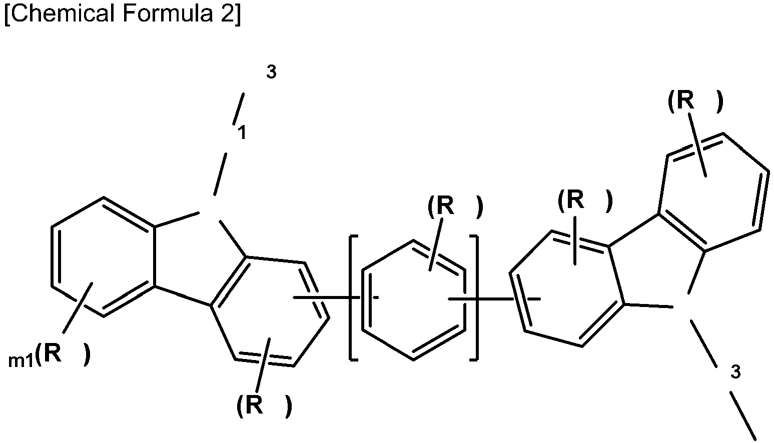

In Chemical Formula 2,
R¹² to R¹⁶ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar³ and Ar⁴ are each independently a substituted or unsubstituted C6 to C20 aryl group or a substituted or unsubstituted C2 to C30 heterocyclic group,
L¹ and L² are each independently a single bond or a substituted or unsubstituted C6 to C20 arylene group,
m1, m4, and m5 are each independently one of integers from 1 to 4,
m2 and m3 are each independently one of integers from 1 to 3, and
n is one of integers from 0 to 2; 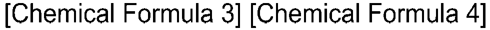 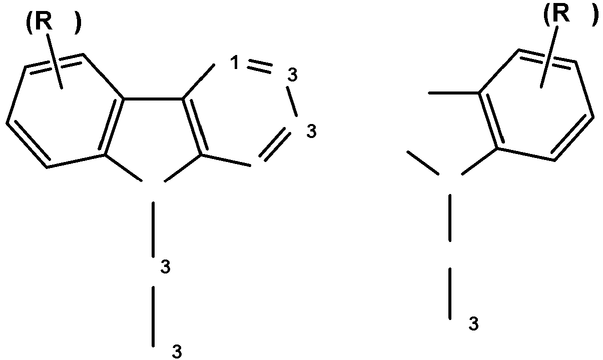
wherein, in Chemical Formula 3 and Chemical Formula 4,
among a₁* to a₄* in Chemical Formula 3, two adjacent ones are each a linking carbon (C) that is linked to *s in Chemical Formula 4,
among a₁* to a₄* of Chemical Formula 3, the remaining two that are not linked to Chemical Formula 4 are C-L^{a}-R^{a},
L^{a}, L³, and L⁴ are each independently a single bond or a substituted or unsubstituted C6 to C20 arylene group,
R^{a}, R¹⁷, and R¹⁸ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group, and
m6 and m7 are each independently one of integers from 1 to 4; 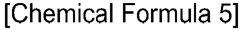 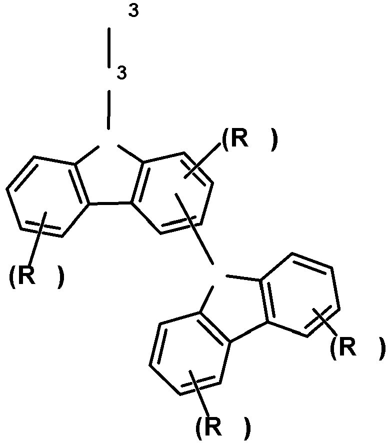
wherein, in Chemical Formula 5,
L⁵ is each independently a single bond or a substituted or unsubstituted C6 to C20 arylene group,
R¹⁹ to R²² are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁷ is a substituted or unsubstituted C6 to C20 aryl group or a substituted or unsubstituted C2 to C30 heterocyclic group,
m8, m10, and m11 are each independently one of integers from 1 to 4, and
m9 is one of integers from 1 to 3.

According to another embodiment, an organic optoelectronic device includes an anode and a cathode facing each other, and at least one organic layer between the anode and the cathode, and the organic layer includes the compound for the organic optoelectronic device.

According to another embodiment, a display device including the organic optoelectronic device is provided.

### [Advantageous Effects]

High-efficiency, long life-span organic optoelectronic devices may be realized while lowering the driving voltage.

### [Description of the Drawings]

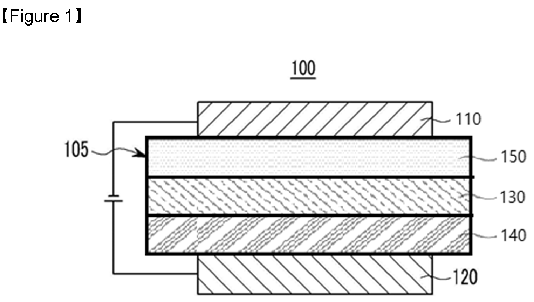

FIG. 1 is a cross-sectional view showing an organic light emitting diode according to an embodiment.

### <Description of Symbols>

100: organic light emitting diode
105: organic layer
110: cathode
120: anode
130: light emitting layer
140: hole transport region
150: electron transport region

### [Best Mode]

Hereinafter, embodiments of the present invention are described in detail. However, these are presented as examples, and the present invention is not limited thereto, and the present invention is only defined by the scope of the claims to be described later.

In the present specification, when a definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a cyano group, or a combination thereof.

In one example of the present invention, the "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, or a cyano group. In a specific example of the present invention, the "substituted" refers to replacement of at least one hydrogen of a substituent or a compound by deuterium, a C1 to C20 alkyl group, a C1 to C5 alkylsilyl group, a C6 to C20 aryl group, a C2 to C20 heteroaryl group, or a cyano group. In a specific example of the present invention, the "substituted" refers to replacement of at least one hydrogen of a substituent or a compound by deuterium, a C1 to C5 alkyl group, a C1 to C5 alkylsilyl group, a C6 to C18 aryl group, a C2 to C18 heteroaryl group, or a cyano group. In a specific example of the present invention, the "substituted" refers to replacement of at least one hydrogen of a substituent or a compound by deuterium, a cyano group, a methyl group, an ethyl group, an n-propyl group, an iso-propyl group, an n-butyl group, an iso-butyl group, a sec-butyl group, a tert-butyl group, a trimethylsilyl group, a phenyl group, a biphenyl group, a terphenyl group, or a naphthyl group.

In the present specification, "unsubstituted" refers to non-replacement of a hydrogen atom by another substituent and remaining of the hydrogen atom.

In the present specification, hydrogen (-H)" may include "deuterium substitution (-D)" or "tritium substitution (-T).".

In the present specification, when a definition is not otherwise provided, "hetero" refers to one including one to three heteroatoms selected from N, O, S, P, and Si, and remaining carbons in one functional group.

In the present specification, "aryl group" refers to a group including at least one hydrocarbon aromatic moiety, and all elements of the hydrocarbon aromatic moiety have p-orbitals which form conjugation, for example a phenyl group, a naphthyl group, and the like, two or more hydrocarbon aromatic moieties may be linked by a sigma bond and may be, for example a biphenyl group, a terphenyl group, a quaterphenyl group, and the like, and two or more hydrocarbon aromatic moieties are fused directly or indirectly to provide a non-aromatic fused ring, for example a fluorenyl group.

The aryl group may include a monocyclic, polycyclic, or fused ring polycyclic (i.e., rings sharing adjacent pairs of carbon atoms) functional group.

In the present specification, "heterocyclic group" is a generic concept of a heteroaryl group, and may include at least one heteroatom selected from N, O, S, P, and Si instead of carbon (C) in a cyclic compound such as aryl group, a cycloalkyl group, a fused ring thereof, or a combination thereof. When the heterocyclic group is a fused ring, the entire ring or each ring of the heterocyclic group may include one or more heteroatoms.

For example, "heteroaryl group" may refer to aryl group including at least one heteroatom selected from N, O, S, P, and Si. Two or more heteroaryl groups are linked by a sigma bond directly, or when the heteroaryl group includes two or more rings, the two or more rings may be fused. When the heteroaryl group is a fused ring, each ring may include one to three heteroatoms.

More specifically, the substituted or unsubstituted C6 to C30 aryl group may be a substituted or unsubstituted phenyl group, a substituted or unsubstituted naphthyl group, a substituted or unsubstituted anthracenyl group, a substituted or unsubstituted phenanthrenyl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted o-terphenyl group, a substituted or unsubstituted chrysenyl group, a substituted or unsubstituted benzophenanthrenyl group, a substituted or unsubstituted triphenylene group, a substituted or unsubstituted perylenyl group, a substituted or unsubstituted fluorenyl group, a substituted or unsubstituted indenyl group, or a combination thereof, but is not limited thereto.

More specifically, the substituted or unsubstituted C2 to C30 heterocyclic group may be a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted carbazolyl group, a substituted or unsubstituted dibenzofuranyl group, or a substituted or unsubstituted dibenzothiophenyl group, a substituted or unsubstituted benzonaphthol furanyl group, a substituted or unsubstituted benzonaphthothiophenyl group, a substituted or unsubstituted benzofuranofluorenyl group, a substituted or unsubstituted benzothiophenefluorenyl group, or a combination thereof, but is not limited thereto.

In the present specification, hole characteristics refer to an ability to donate an electron to form a hole when an electric field is applied and that a hole formed in the anode may be easily injected into the light emitting layer and transported in the light emitting layer due to conductive characteristics according to a highest occupied molecular orbital (HOMO) level.

In addition, electron characteristics refer to an ability to accept an electron when an electric field is applied and that electron formed in the cathode may be easily injected into the light emitting layer and transported in the light emitting layer due to conductive characteristics according to a lowest unoccupied molecular orbital (LUMO) level.

Hereinafter, a compound for an organic optoelectronic device according to an embodiment is described.

A compound for an organic optoelectronic device according to an embodiment is represented by Chemical Formula 1.

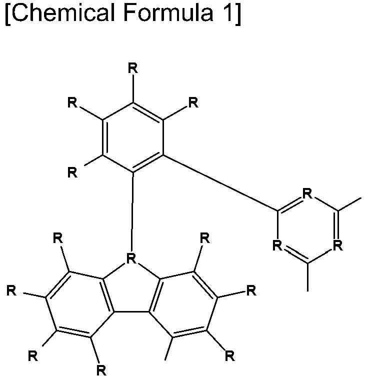

In Chemical Formula 1,
Ar¹ and Ar² are each independently a substituted or unsubstituted C6 to C30 aryl group,
Ar³ is a substituted or unsubstituted C6 to C30 aryl group,
at least one of Ar¹ to Ar³ is a substituted or unsubstituted C10 to C30 aryl group,
R¹ to R⁷ are each independently hydrogen, deuterium, a cyano group, a halogen, a substituted or unsubstituted C1 to C30 alkyl group, or a substituted or unsubstituted C6 to C30 aryl group,
R⁸ to R¹¹ are each independently hydrogen, deuterium, a cyano group, a halogen, or a substituted or unsubstituted C1 to C30 alkyl group.

The compound represented by Chemical Formula 1 has a structure in which N-carbazole and triazine are linked by ortho-phenylene.

The structure has a high glass transition temperature (Tg) relative to the molecular weight and a shallow LUMO energy level, whereby a high-efficiency organic light emitting diode may be realized.

In addition, because at least one of Ar¹ to Ar³ becomes a C10 to C30 aryl group, characteristics of the organic light emitting diode are improved. When an aryl group of C10 or higher is introduced to triazine, an effect of decreasing the driving voltage may be obtained, and because Ar³ is substituted at the 4th position of carbazole, the efficiency may be further improved. In addition, if an aryl group of C10 or higher is introduced at the 4th position of carbazole, the life span can be improved compared to phenyl.

For example, Ar¹ and Ar² may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, or a substituted or unsubstituted triphenylene group, and at least one of Ar¹ and Ar² may be a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, or a substituted or unsubstituted triphenylene group.

As another example, Ar¹ and Ar² may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, or a substituted or unsubstituted triphenylene group, and Ar³ may be a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, or a substituted or unsubstituted triphenylene group.

As a specific example, Ar¹ and Ar² may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, or a substituted or unsubstituted triphenylene group, and at least one of Ar¹ and Ar² may be a substituted or unsubstituted biphenyl group, or a substituted or unsubstituted terphenyl group.

As another specific example, Ar¹ and Ar² may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, or a substituted or unsubstituted triphenylene group, and Ar³ may be a substituted or unsubstituted biphenyl group, or a substituted or unsubstituted terphenyl group.

In a specific embodiment, the compound represented by Chemical Formula 1 may be one selected from compounds listed in Group 1, but is not limited thereto.

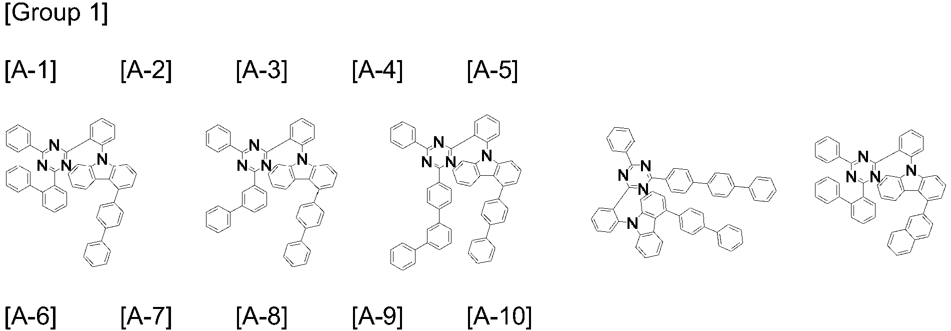

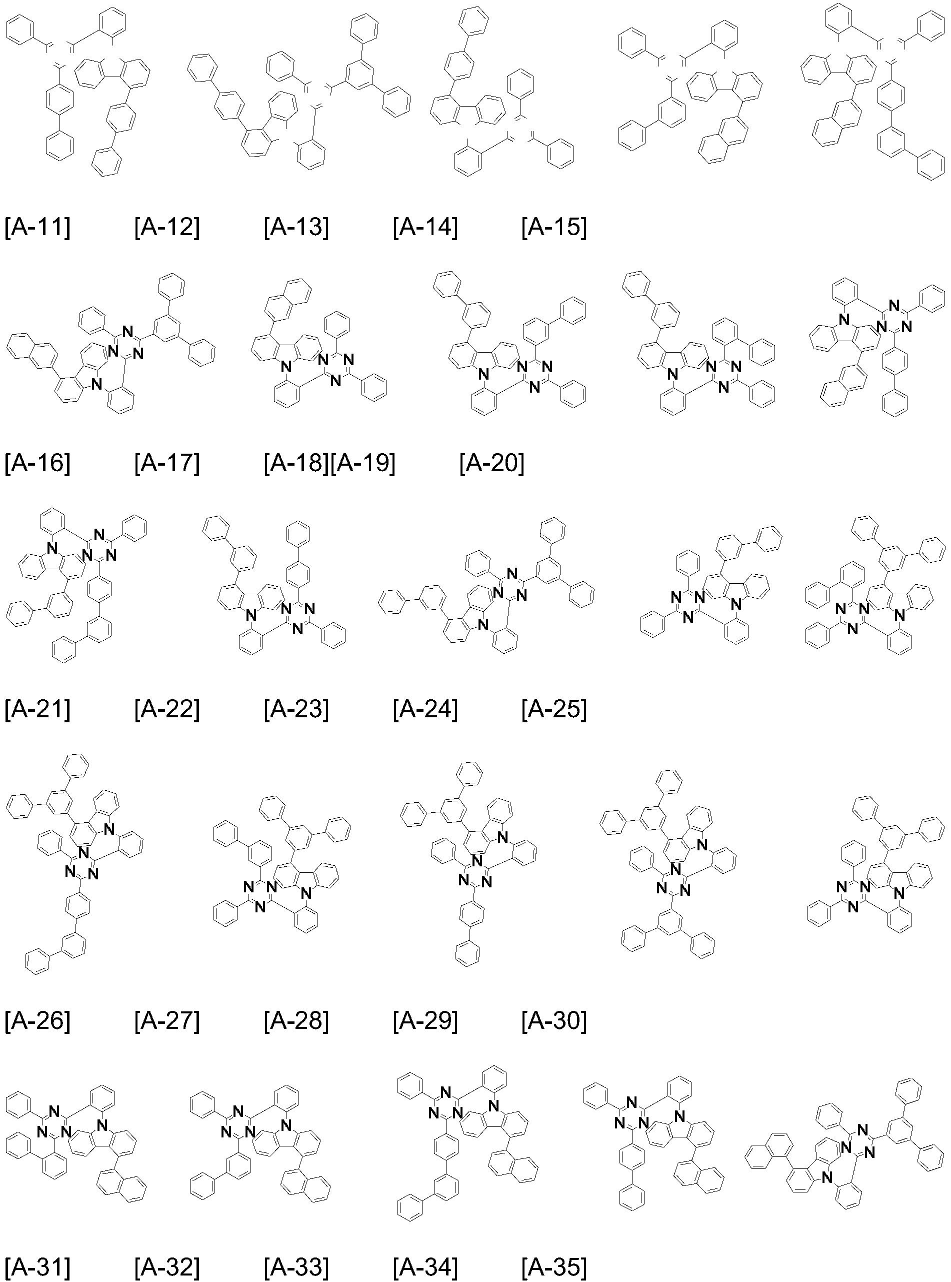

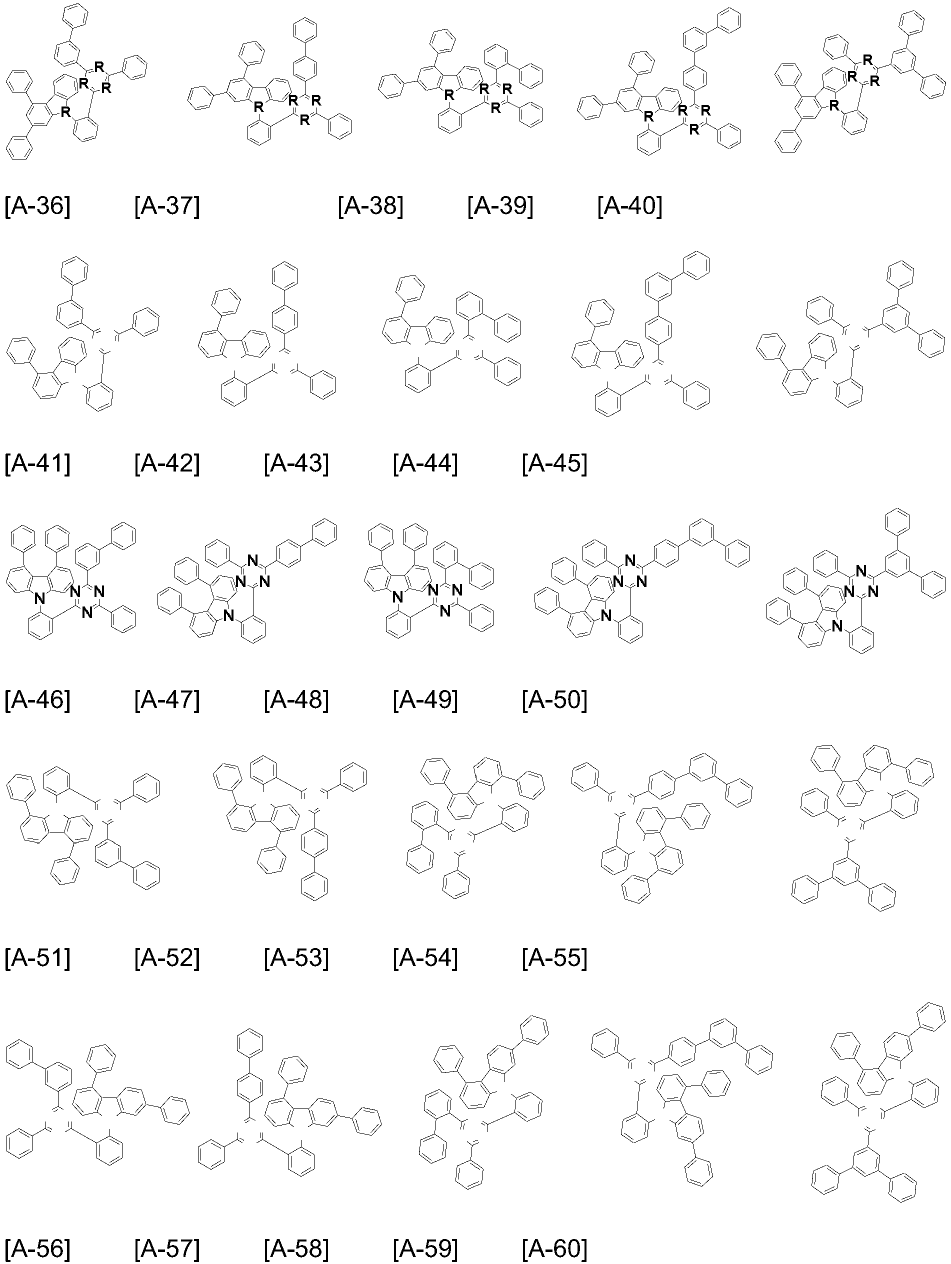

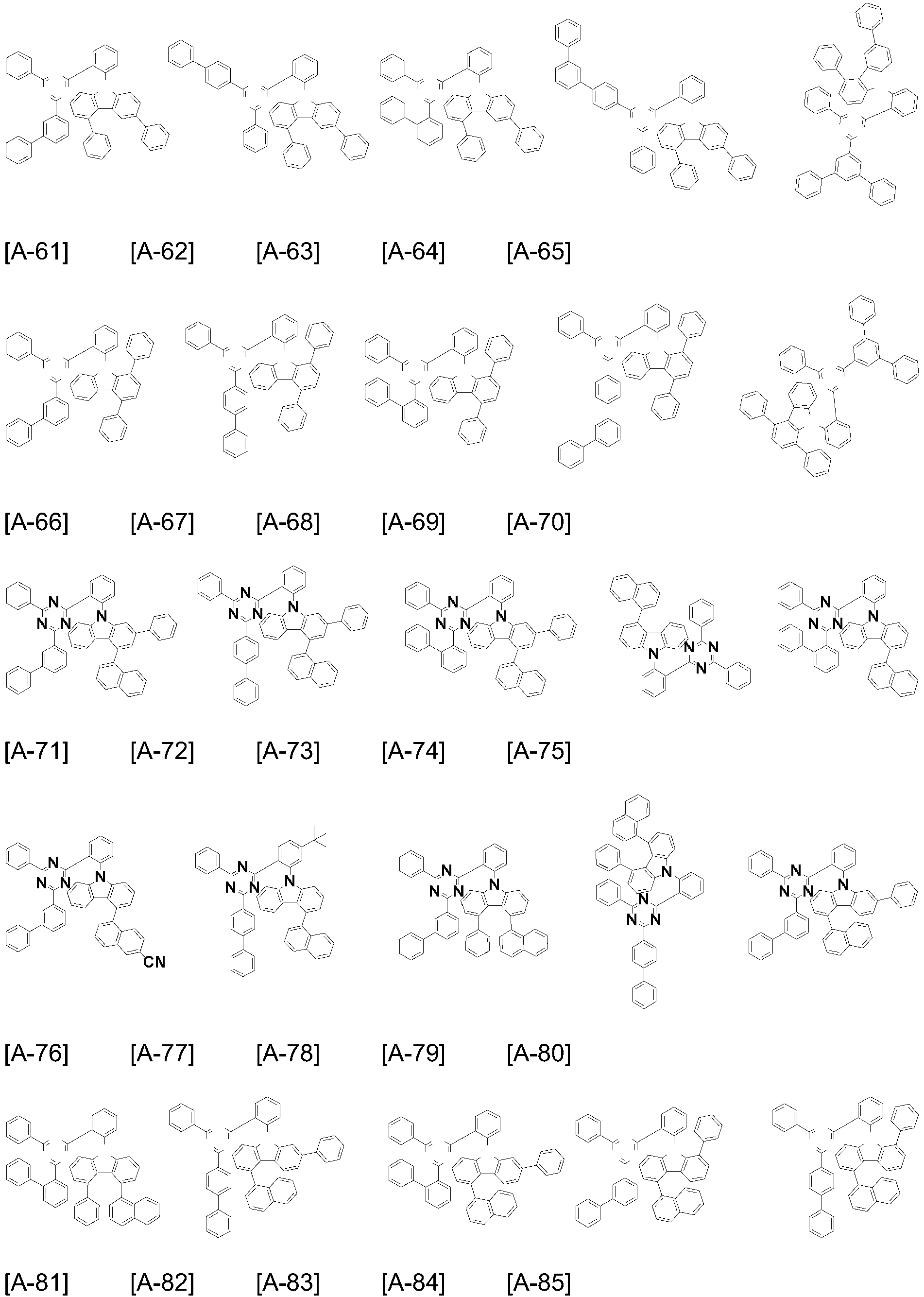

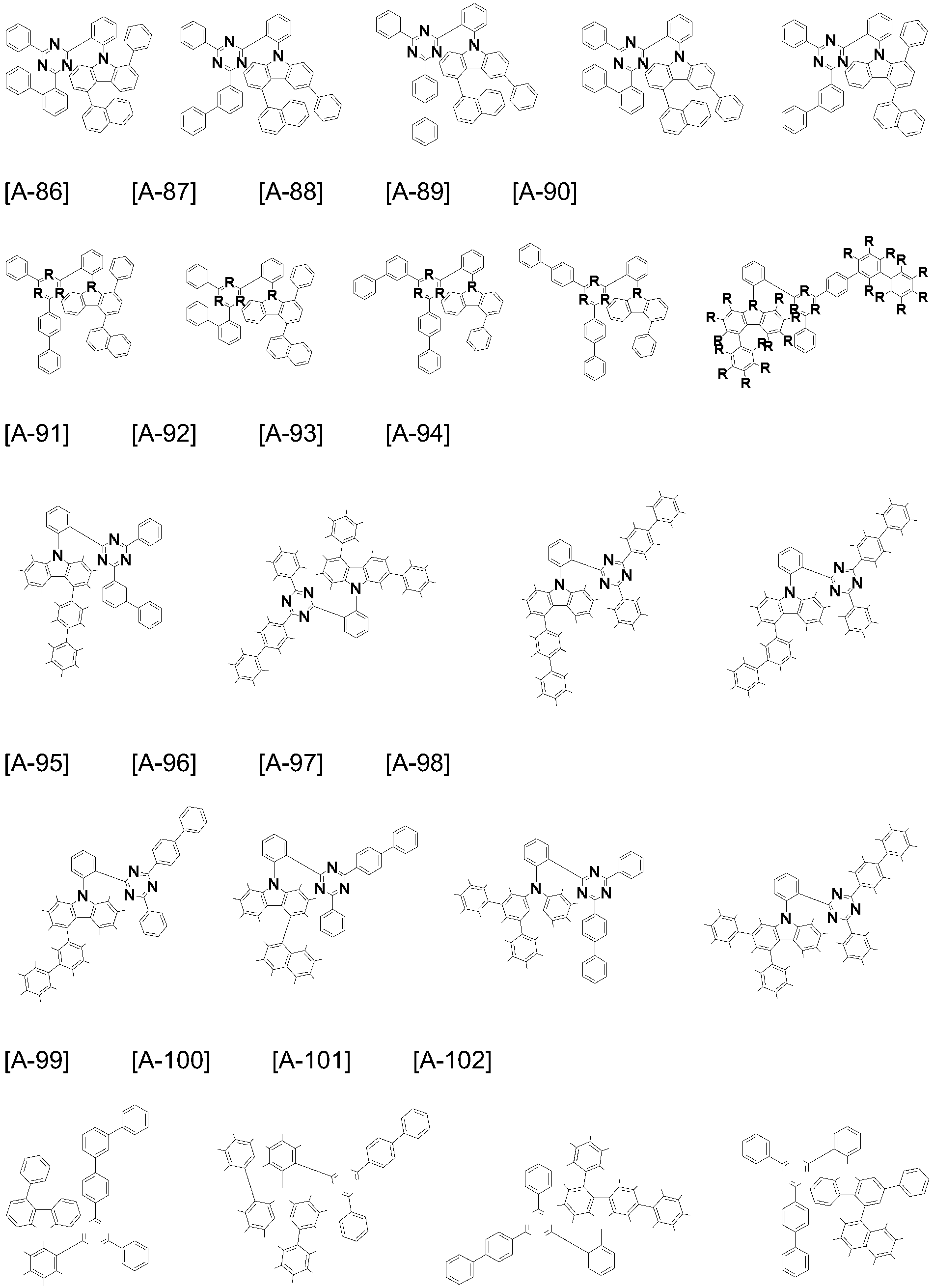

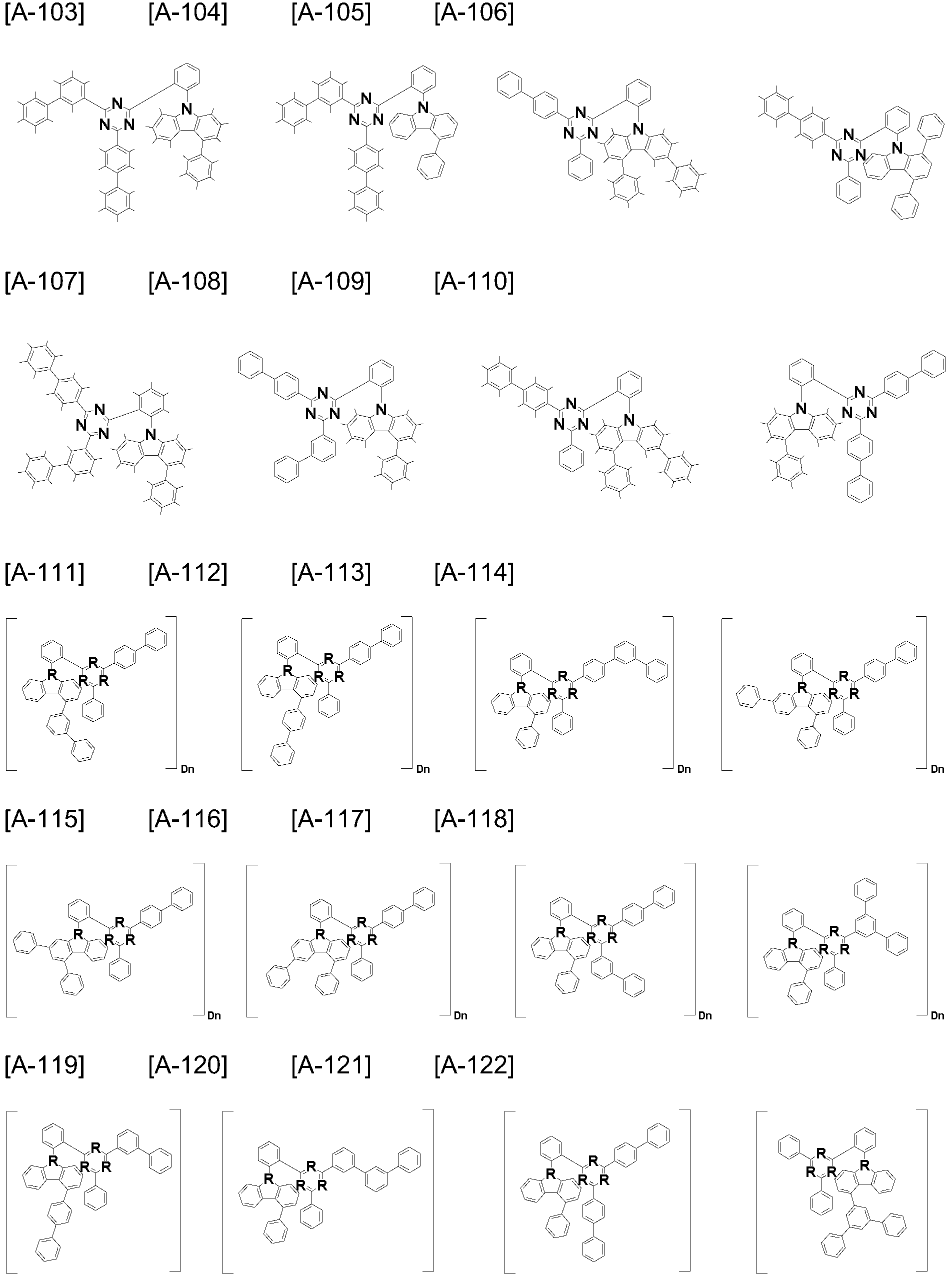

(Dn refers to the number of deuterium substitutions and indicates a structure substituted with one or more deuteriums)

A composition for an organic optoelectronic device according to another embodiment includes a first compound and a second compound, wherein the first compound is the aforementioned compound for the organic optoelectronic device, and the second compound may be represented by Chemical Formula 2; a combination of Chemical Formula 3 and Chemical Formula 4; or Chemical Formula 5.

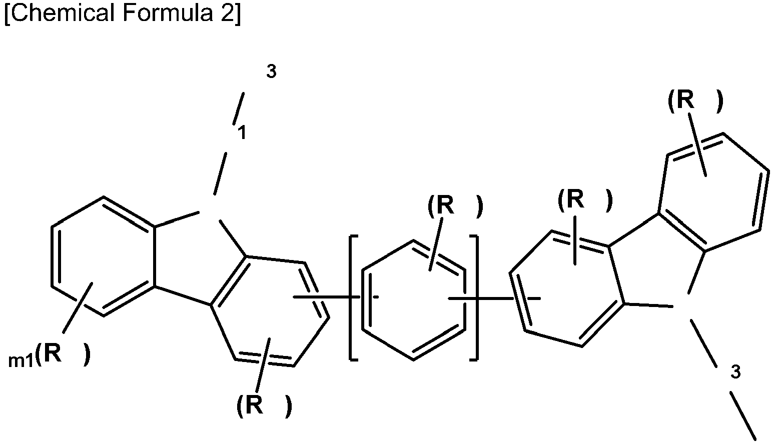

In Chemical Formula 2,
R¹² to R¹⁶ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar³ and Ar⁴ are each independently a substituted or unsubstituted C6 to C20 aryl group or a substituted or unsubstituted C2 to C30 heterocyclic group,
L¹ and L² are each independently a single bond or a substituted or unsubstituted C6 to C20 arylene group,
m1, m4, and m5 are each independently one of integers from 1 to 4,
m2 and m3 are each independently one of integers from 1 to 3, and
n is one of integers from 0 to 2; 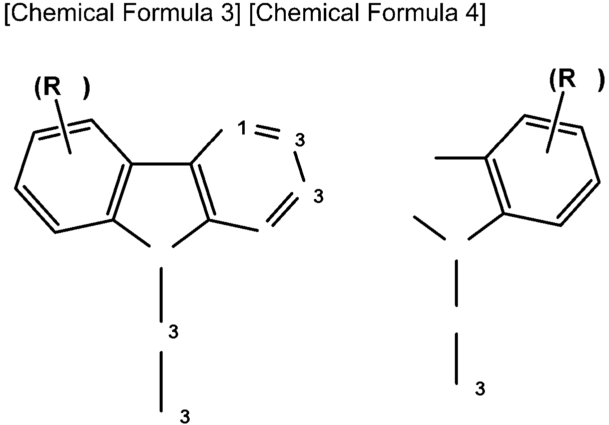
wherein, in Chemical Formula 3 and Chemical Formula 4,
among a₁* to a₄* in Chemical Formula 3, two adjacent ones are each a linking carbon (C) that is linked to *s in Chemical Formula 4,
among a₁* to a₄* of Chemical Formula 3, the remaining two that are not linked to Chemical Formula 4 are C-L^{a}-R^{a},
L^{a}, L³, and L⁴ are each independently a single bond or a substituted or unsubstituted C6 to C20 arylene group,
R^{a}, R¹⁷, and R¹⁸ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group, and
m6 and m7 are each independently one of integers from 1 to 4; 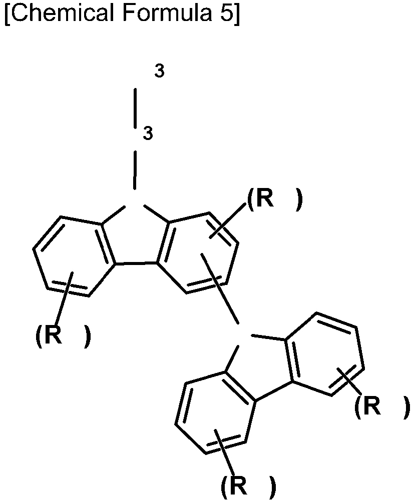
wherein, in Chemical Formula 5,
L⁵ is each independently a single bond or a substituted or unsubstituted C6 to C20 arylene group,
R¹⁹ to R²² are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁷ is a substituted or unsubstituted C6 to C20 aryl group or a substituted or unsubstituted C2 to C30 heterocyclic group,
m8, m10, and m11 are each independently one of integers from 1 to 4, and
m9 is one of integers from 1 to 3.

The second compound is used in the light emitting layer together with the first compound to increase charge mobility and increase stability, thereby improving luminous efficiency and life-span characteristics.

In Chemical Formula 2, when m1 is 2 or more, each R¹² may be the same or different from each other.

In Chemical Formula 2, when m2 is 2 or more, each R¹³ may be the same or different from each other.

In Chemical Formula 2, when m3 is 2 or more, each R¹⁴ may be the same or different from each other.

In Chemical Formula 2, when m4 is 2 or more, each R¹⁵ may be the same or different from each other.

In Chemical Formula 2, when m5 is 2 or more, each R¹⁶ may be the same or different from each other.

In Chemical Formula 3 and Chemical Formula 4, when m6 is 2 or more, each R¹⁷ may be the same or different from each other.

In Chemical Formula 3 and Chemical Formula 4, when m7 is 2 or more, each R¹⁸ may be the same or different from each other.

In Chemical Formula 3 and Chemical Formula 4, when two or more R^{a}s are substituted, each R^{a} may be the same or different from each other.

In Chemical Formula 5, when m8 is 2 or more, each R¹⁹ may be the same or different from each other.

In Chemical Formula 5, when m9 is 2 or more, each R²⁰ may be the same or different from each other.

In Chemical Formula 5, when m10 is 2 or more, each R²¹ may be the same or different from each other.

In Chemical Formula 5, when m11 is 2 or more, each R²² may be the same or different from each other.

As an example, in Chemical Formula 2, Ar³ and Ar⁴ may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group,
in Chemical Formula 2, L¹ and L² may each independently be a single bond, a substituted or unsubstituted phenylene group, or a substituted or unsubstituted biphenylene group,
in Chemical Formula 2, R¹² to R¹⁶ may each independently be hydrogen, deuterium, or a substituted or unsubstituted C6 to C12 aryl group, and
n may be 0 or 1.

As an example, in Chemical Formula 2, "substituted" refers to replacement of at least one hydrogen by deuterium, a C1 to C4 alkyl group, a C6 to C18 aryl group, or a C2 to C30 heteroaryl group.

For example, in Chemical Formula 2, Ar³ and Ar⁴ may each independently be a substituted or unsubstituted carbazolyl group, a substituted or unsubstituted dibenzothiophenyl group, a substituted or unsubstituted dibenzofuranyl group, or a substituted or unsubstituted fluorenyl group.

In another specific embodiment of the present invention, Chemical Formula 2 may be represented by one of Chemical Formula 2-1 to Chemical Formula 2-15.

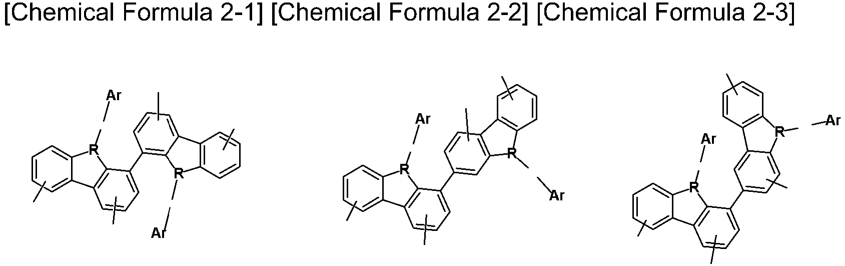

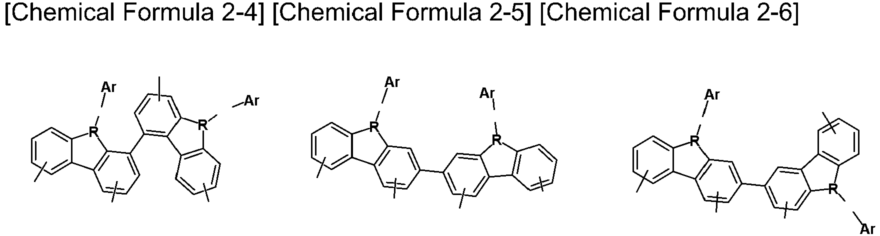

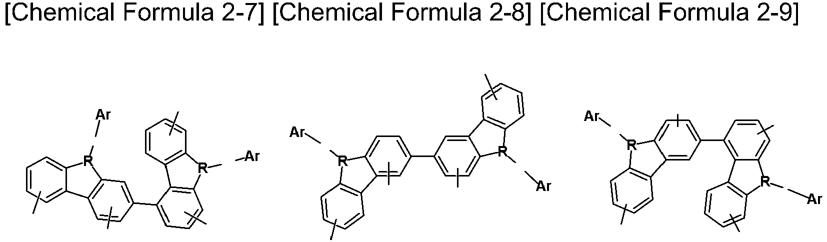

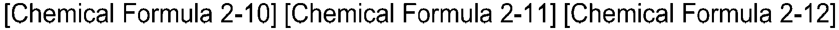

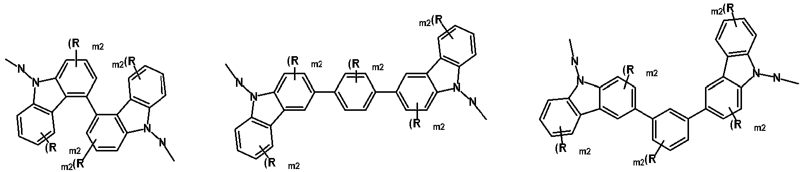

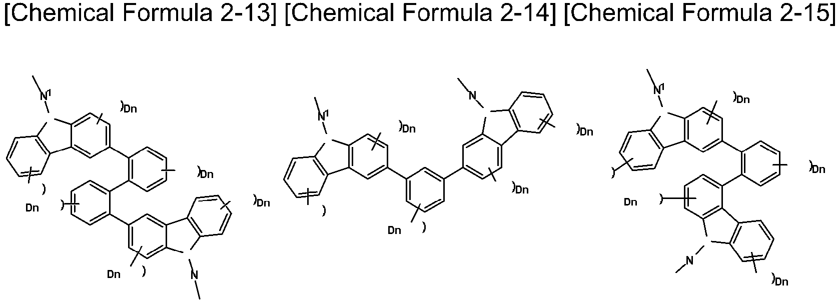

In Chemical Formula 2-1 to Chemical Formula 2-15, R¹² to R¹⁶ may each independently be hydrogen, deuterium, or a substituted or unsubstituted C6 to C12 aryl group, and L¹-Ar³ and L²-Ar⁴ may each independently be one of substituents listed in Group I.

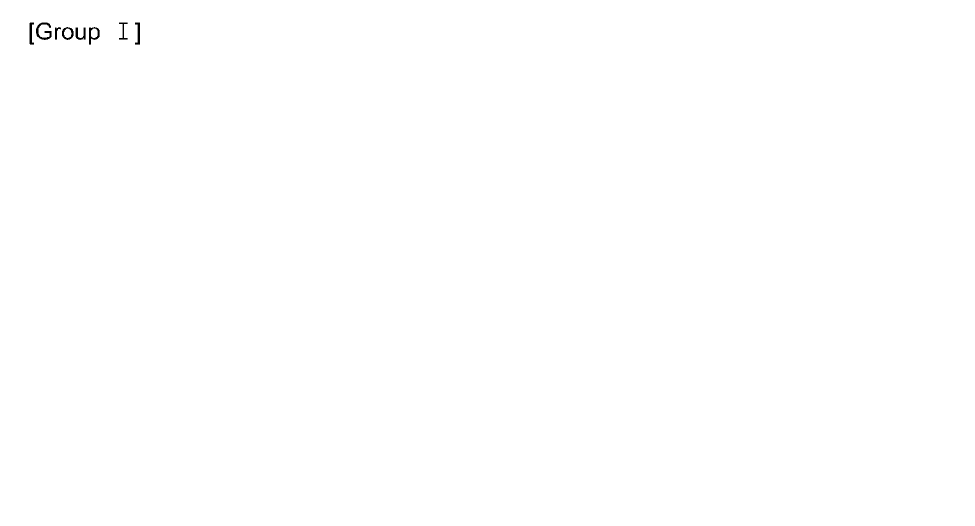

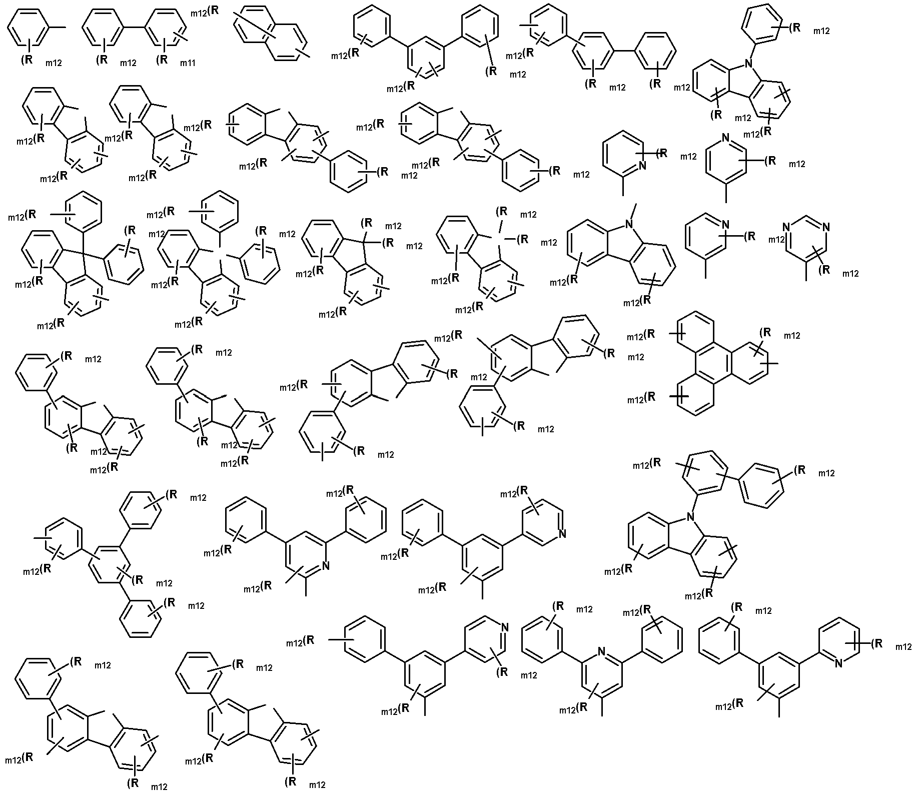

In Group I,
R²³ to R²⁷ are each independently hydrogen, deuterium, a cyano group, a C1 to C10 alkyl group, or a C6 to C12 aryl group,
m12 is one of integers from 1 to 5,
m13 is one of integers from 1 to 4,
m14 is one of integers from 1 to 3,
m15 is an integer of 1 or 2,
m16 is one of integers from 1 to 7, and
* is a linking point.

In Group I, when m12 is 2 or more, each R²³ may be the same or different from each other.

In Group I, when m13 is 2 or more, each R²⁴ may be the same or different from each other.

In Group I, when m14 is 2 or more, each R²⁵ may be the same or different from each other.

In Group I, when m15 is 2, each R²⁶ may be the same or different from each other.

In Group I, when m16 is 2 or more, each R²⁷ may be the same or different from each other.

The combination of Chemical Formula 3 and Chemical Formula 4 may be represented by, for example, any one of Chemical Formula 3A, Chemical Formula 3B, Chemical Formula 3C, Chemical Formula 3D, and Chemical Formula 3E.

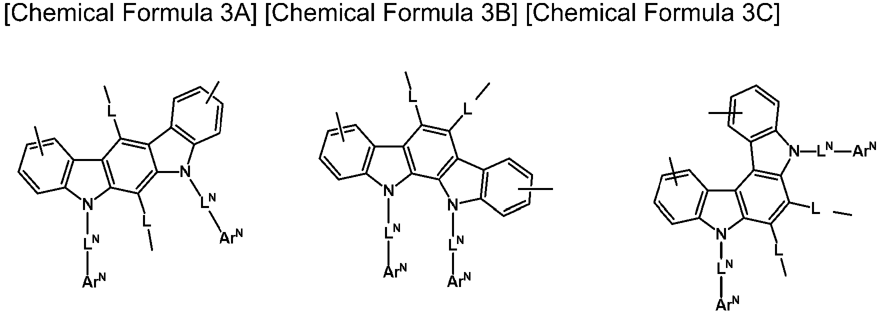

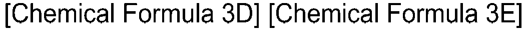

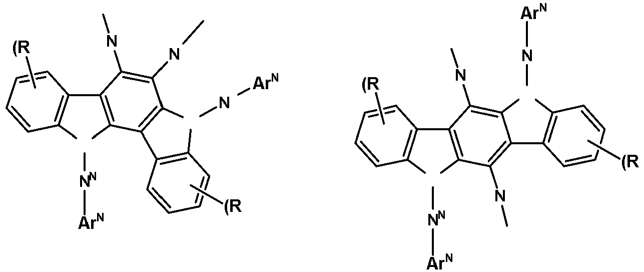

In Chemical Formula 3A to Chemical Formula 3E, L³, L⁴, Ar⁵, Ar⁶, R¹⁷, R¹⁸, m6, and m7 are the same as described above,
L^{a1} to L^{a4} are the same as the definitions of L³ and L⁴ described above, and
R^{a1} to R^{a4} are the same as the definitions of R¹⁷ and R¹⁸ described above.

For example, in Chemical Formulas 3 and 4, Ar⁵ and Ar⁶ may each independently be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group, and
R^{a1} to R^{a4}, R¹⁷, and R¹⁸ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In another specific embodiment of the present invention, in Chemical Formulas 3 and 4, L³-Ar⁵ and L⁴-Ar⁶ may each independently be selected from substituents listed in Group I .

In an embodiment, R^{a1} to R^{a4}, R¹⁷, and R¹⁸ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, R^{a1} to R^{a4}, R¹⁷, and R¹⁸ may each independently be hydrogen, deuterium, a cyano group, or a substituted or unsubstituted phenyl group, and
in a specific embodiment, R^{a1} to R^{a4}, R¹⁷, and R¹⁸ may each independently be hydrogen, deuterium, or a substituted or unsubstituted phenyl group.

In another specific embodiment of the present invention, the second compound may be represented by Chemical Formula 2-8, and in Chemical Formula 2-8, Ar³ and Ar⁴ may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group, L¹ and L² may each independently be a single bond, or a substituted or unsubstituted C6 to C20 arylene group, and R¹² to R¹⁶ may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, in Chemical Formula 2-8, R¹² to R¹⁵ may each independently be hydrogen, deuterium, or a substituted or unsubstituted C6 to C12 aryl group, and L¹-Ar³ and L²-Ar⁴ may each independently be one of substituents listed in Group I .

In another specific embodiment of the present invention, the second compound may be represented by Chemical Formula 3C, and in Chemical Formula 3C, L^{a3} and L^{a4} may be a single bond, L³ and L⁴ may each independently be a single bond or a substituted or unsubstituted C6 to C12 arylene group, R¹⁷ R¹⁸, R^{a3}, and R^{a4} may each be hydrogen, deuterium, or a phenyl group, and Ar⁵ and Ar⁶ may each independently be a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

For example, in Chemical Formula 3C, L^{a3} and L^{a4} may be a single bond, R¹⁷, R¹⁸, R^{a3}, and R^{a4} may each independently be hydrogen, deuterium, or a C6 to C12 aryl group, and L³-Ar⁵ and L⁴-Ar⁶ may each independently be one of the substituents listed in Group I.

Chemical Formula 5 may be, for example, represented by any one of Chemical Formula 5-1 to Chemical Formula 5-4.

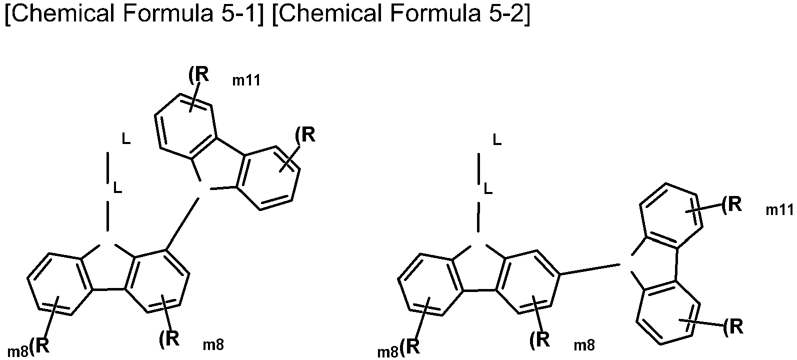

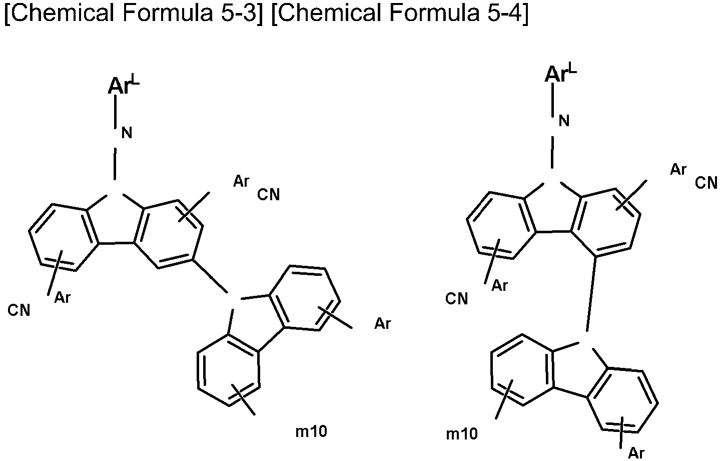

In Chemical Formula 5-1 to Chemical Formula 5-4, L⁵, Ar⁷, R¹⁹ to R²², and m8 to m11 are the same as described above.

For example, in Chemical Formula 5, Ar⁷ may be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted triphenylenyl group, a substituted or unsubstituted carbazolyl group, a substituted or unsubstituted dibenzothiophenyl group, a substituted or unsubstituted dibenzofuranyl group, or a substituted or unsubstituted fluorenyl group, and
R¹⁹ to R²² may each independently be hydrogen, deuterium, a cyano group, a substituted or unsubstituted phenyl group, a substituted or unsubstituted biphenyl group, a substituted or unsubstituted pyridinyl group, a substituted or unsubstituted carbazolyl group, a substituted or unsubstituted dibenzofuranyl group, or a substituted or unsubstituted dibenzothiophenyl group.

In a specific embodiment of the present invention, in Chemical Formula 5, L⁵-Ar⁷ may be selected from the substituents listed in Group I .

For example, R¹⁹ to R²² may each independently be hydrogen, deuterium, a cyano group, or a substituted or unsubstituted phenyl group.

For example, the second compound for the organic optoelectronic device may be one selected from the compounds listed in Group 2, but is not limited thereto.

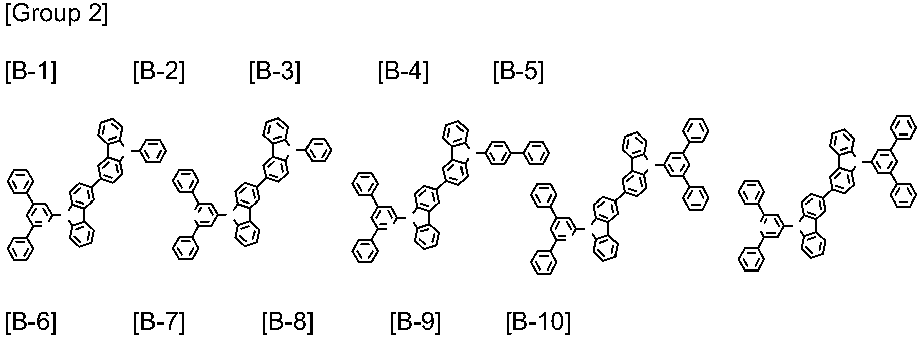

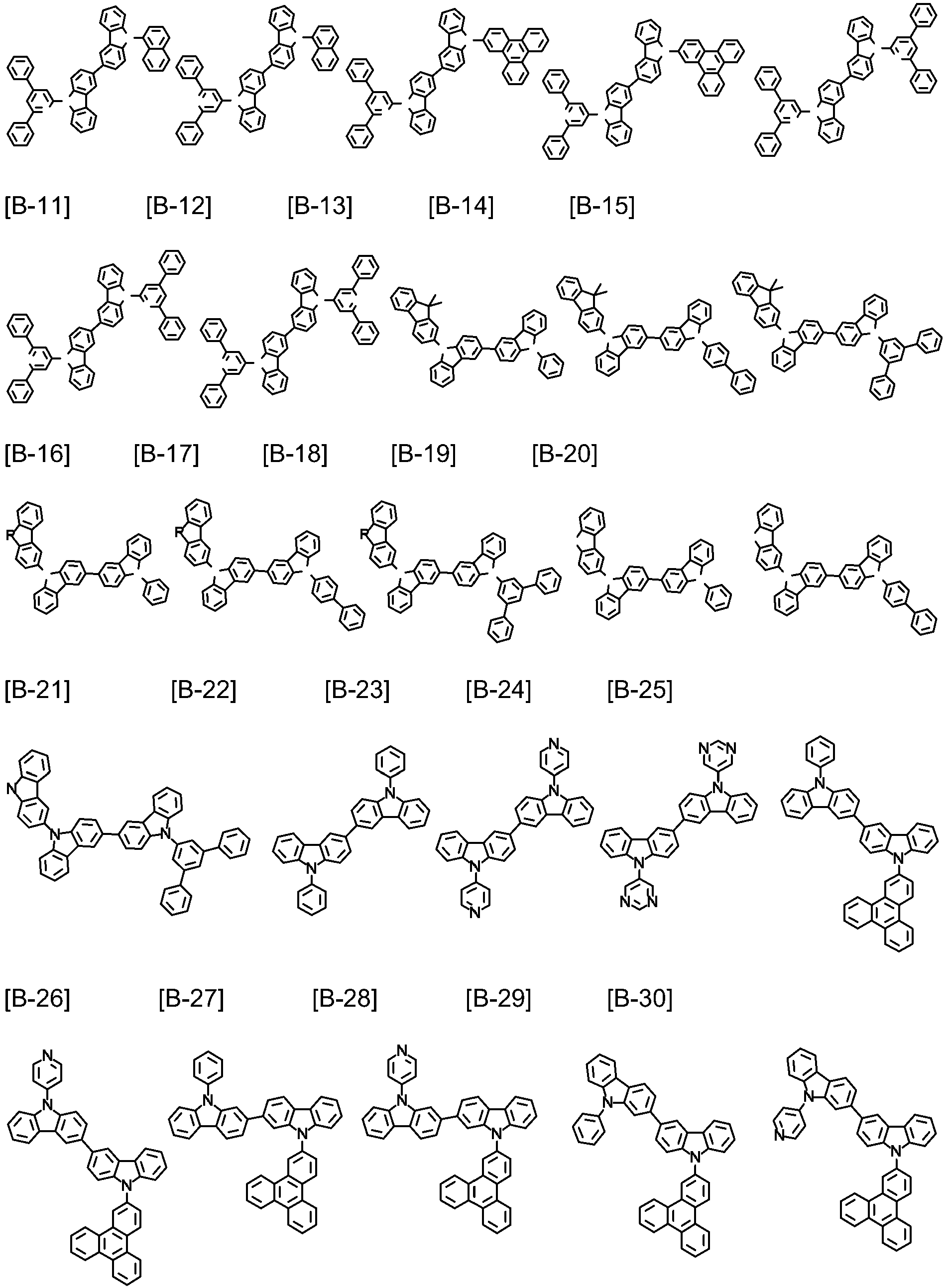

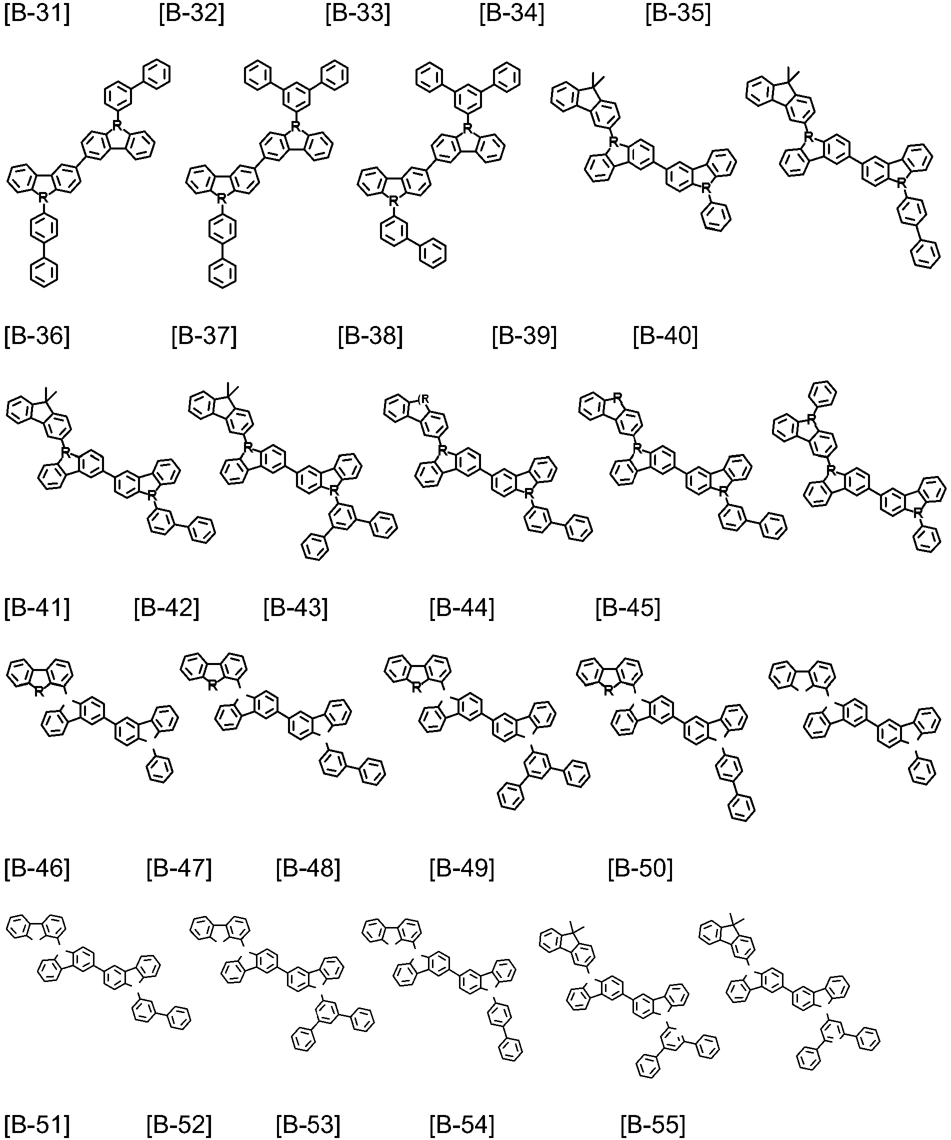

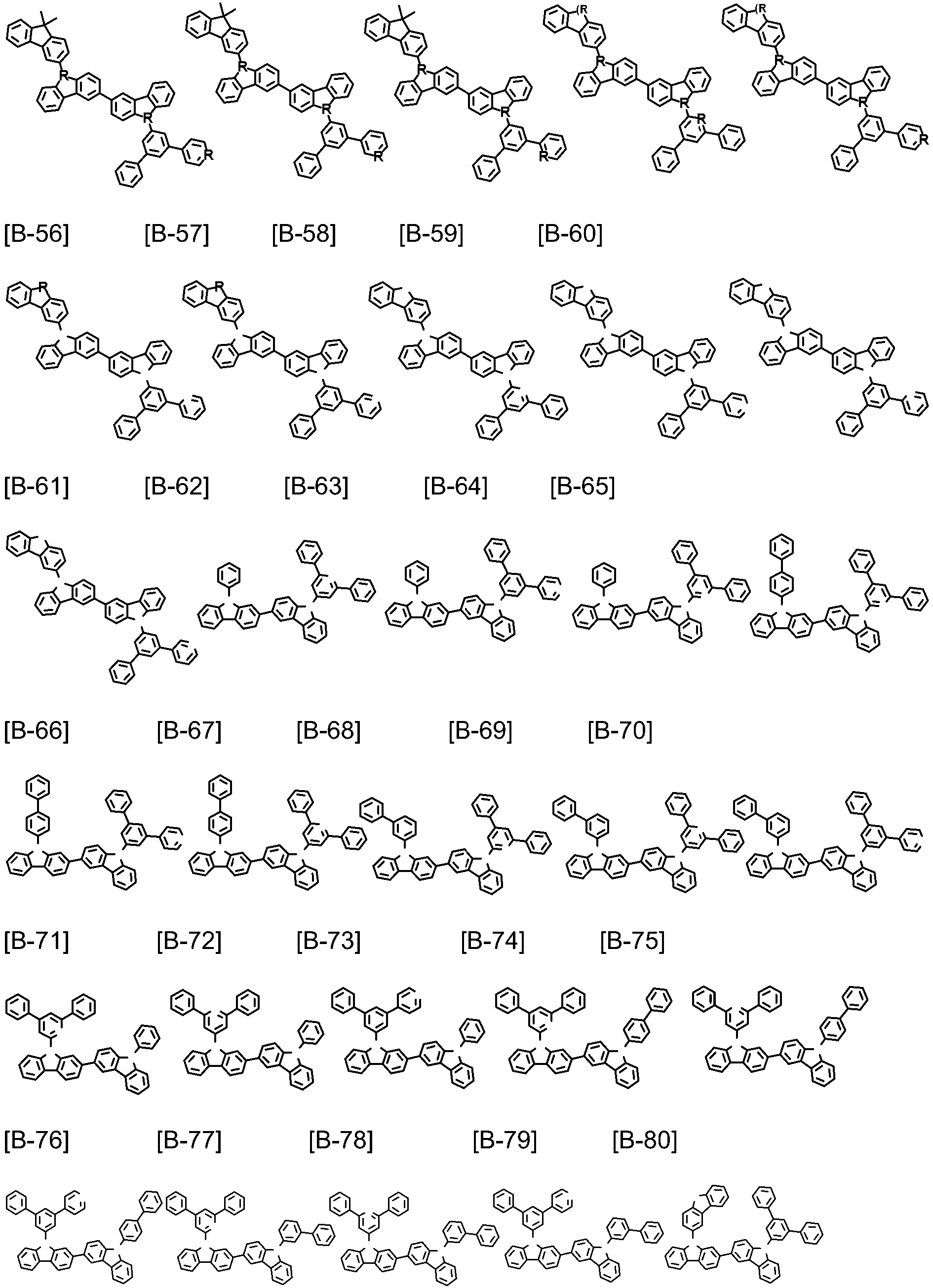

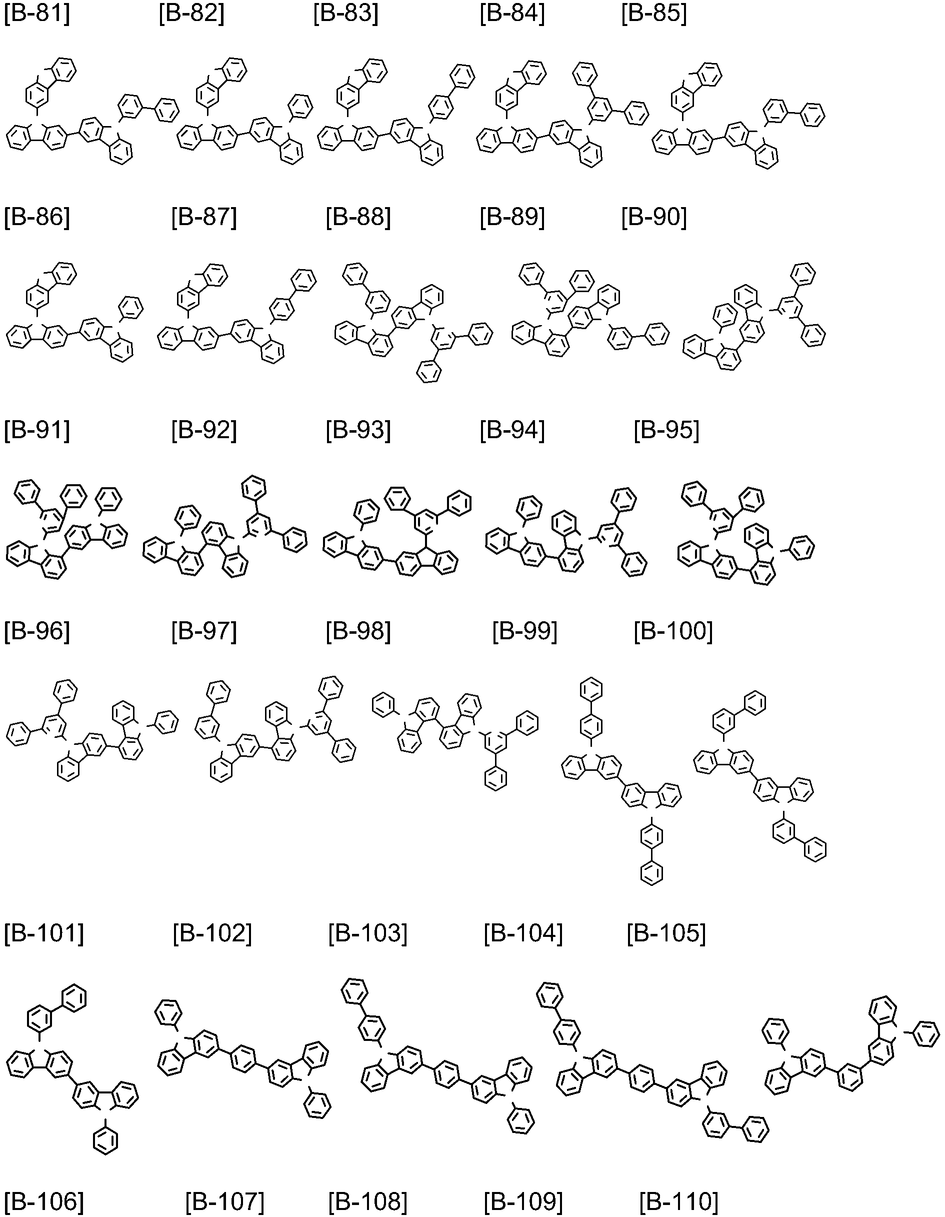

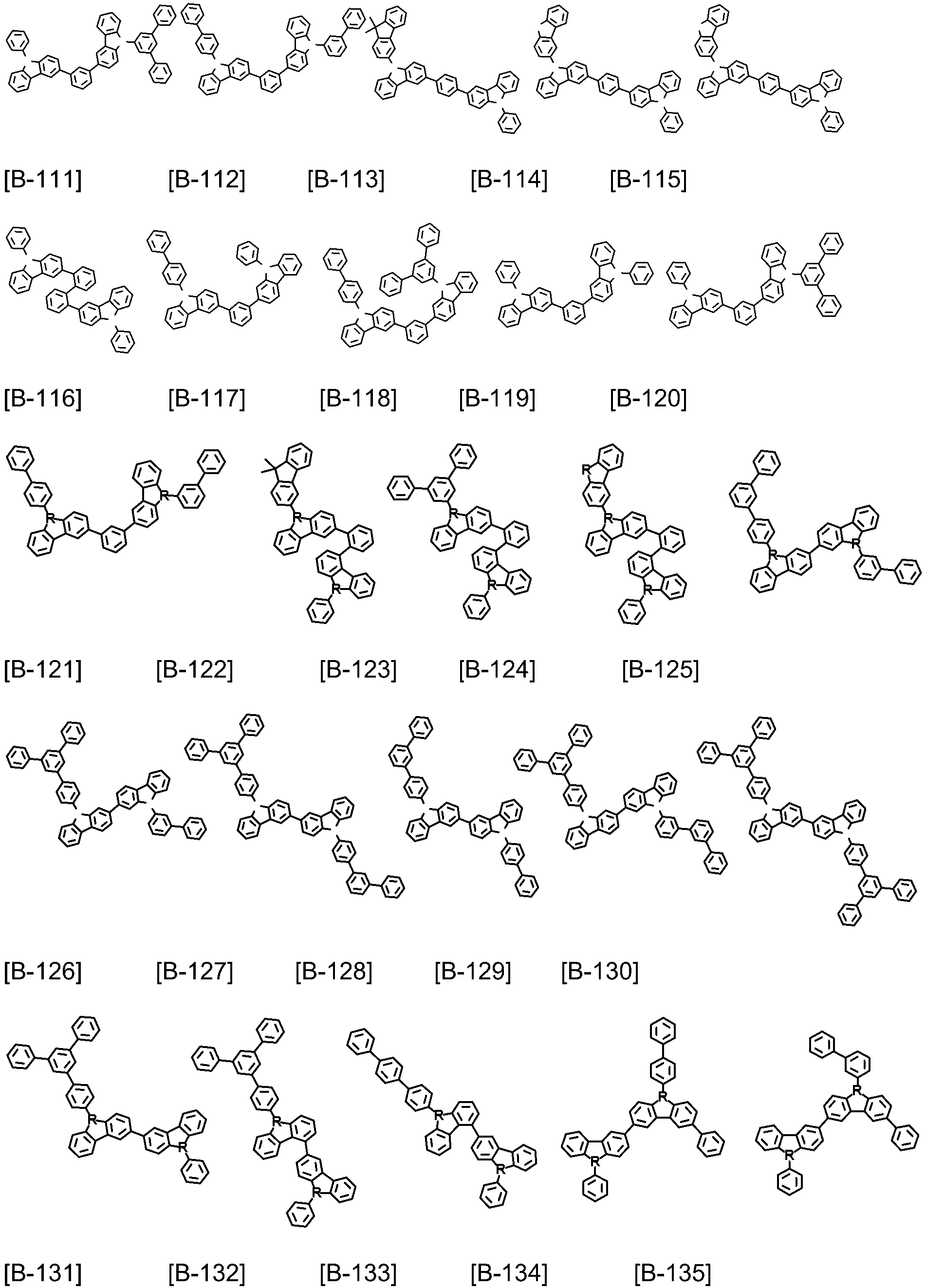

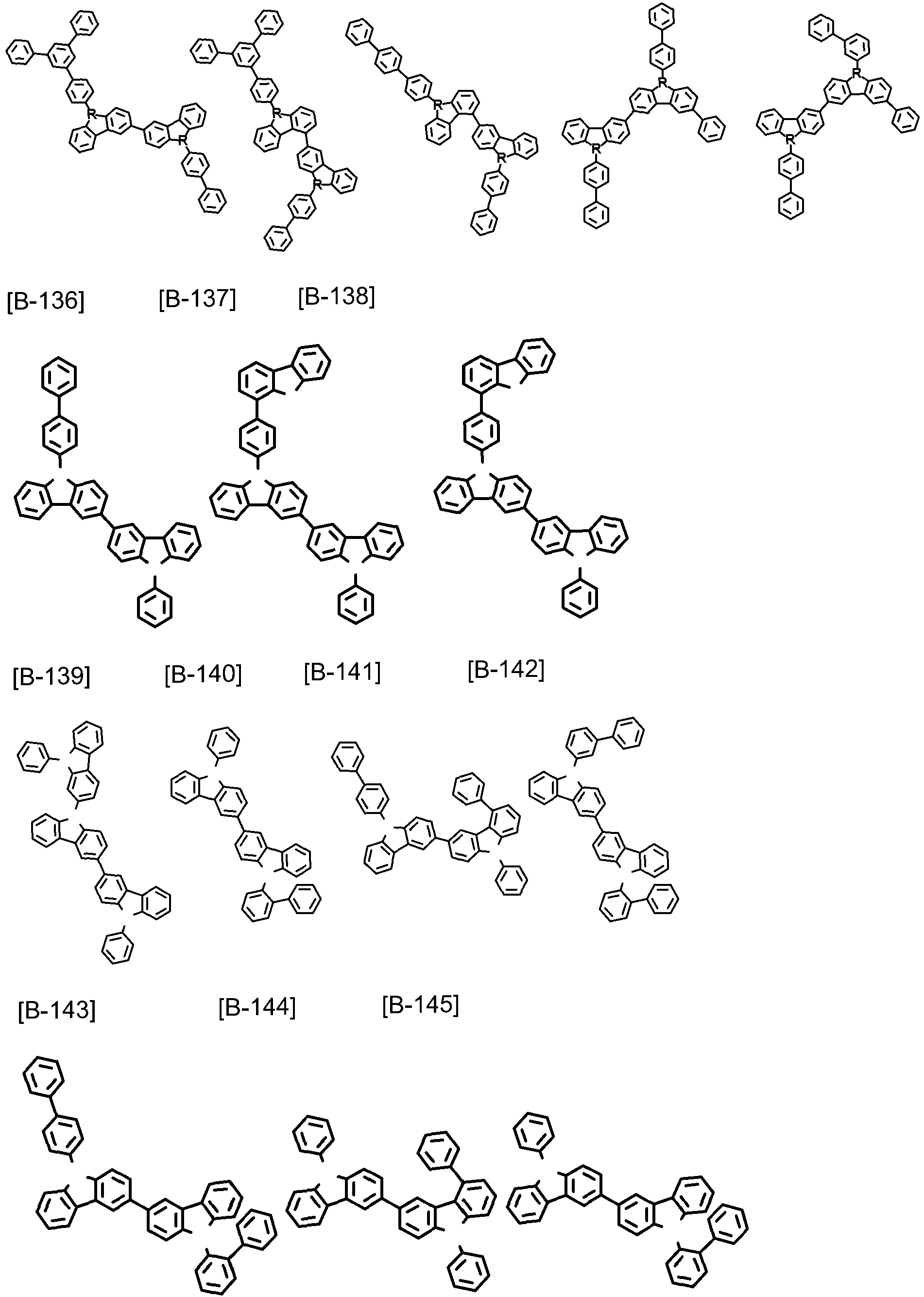

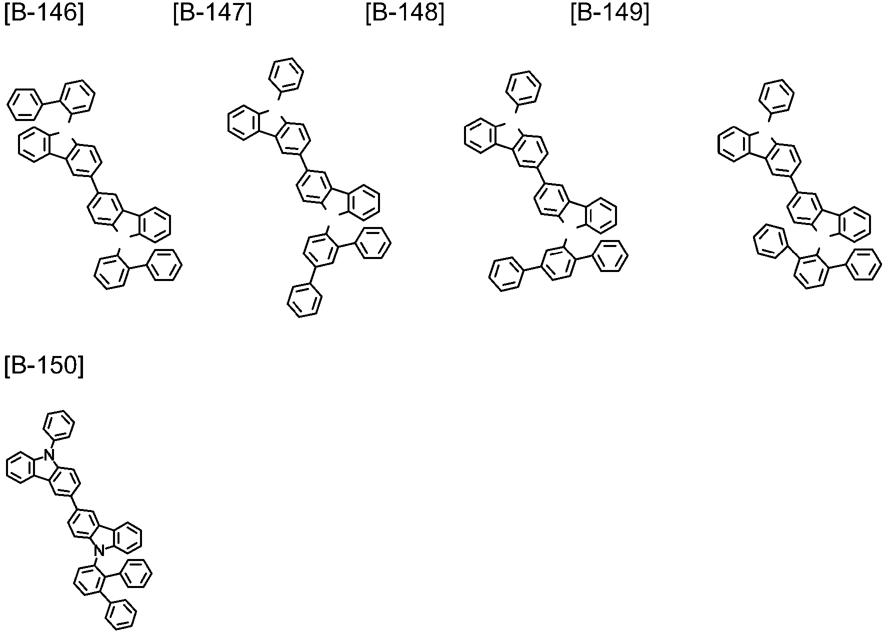

Additionally, examples of Compound B-1 to Compound B-150 listed in Group 2 in which at least one hydrogen is replaced with deuterium are shown below, but the present invention is not limited thereto.

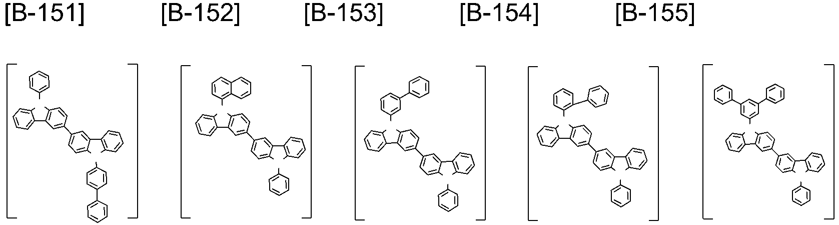

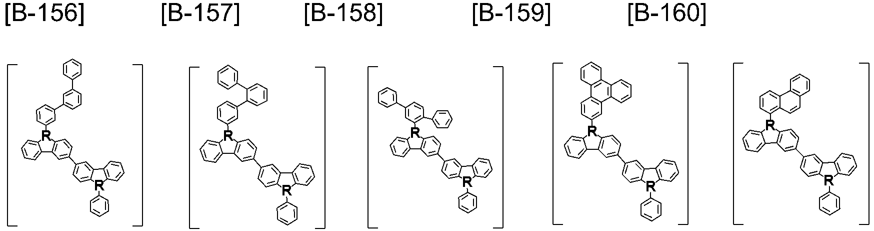

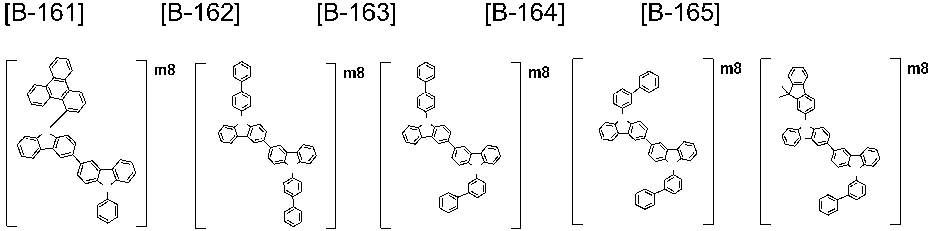

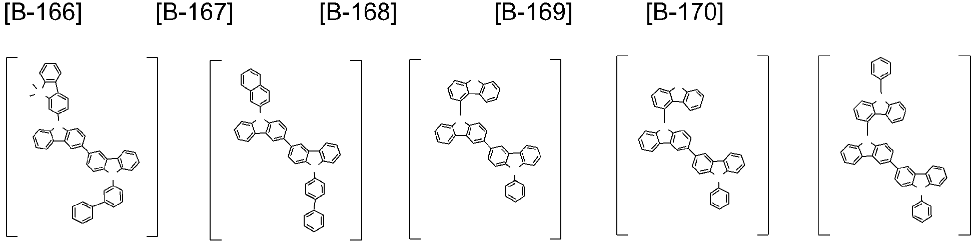

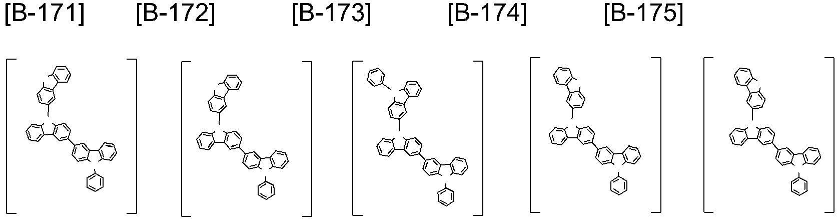

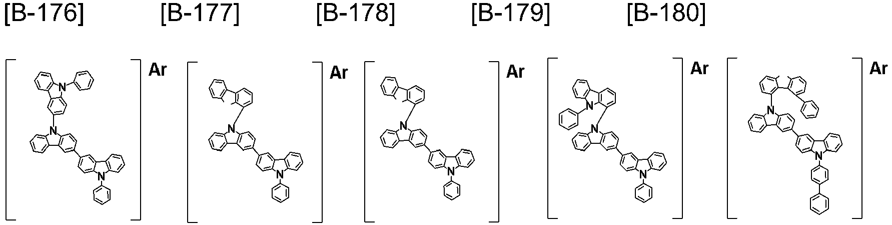

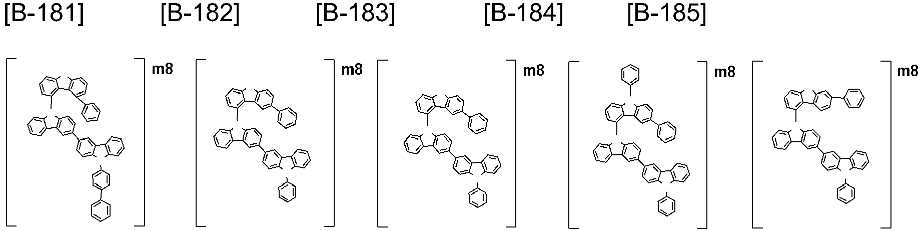

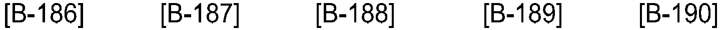

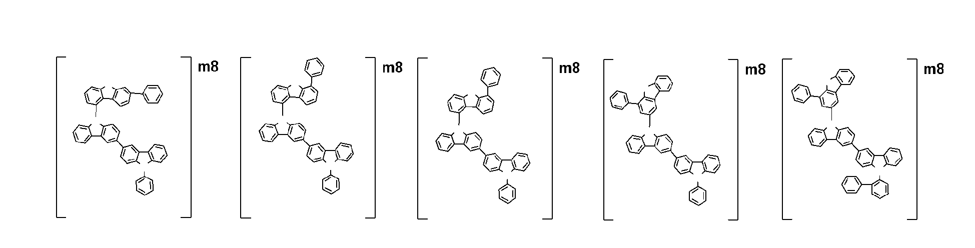

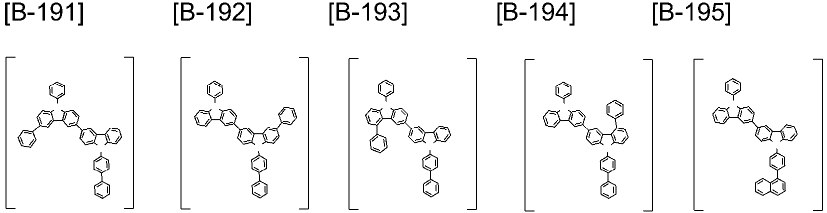

(Dn refers to the number of deuterium substitutions and indicates a structure substituted with one or more deuteriums)

The most specific structures for Compound B-151 to Compound B-195 of Group 2 are presented below as examples according to the position and substitution degree of deuterium substitution, which is not to limit the scope of rights to compounds not shown below.

The scope of the present invention is determined by the claims, and when deuterium is substituted, it is not limited to the compounds exemplified below, and the position of deuterium substitution and the substitution degree of deuterium substitution may include all changeable ranges within the range of Compound B-1 to Compound B-195.

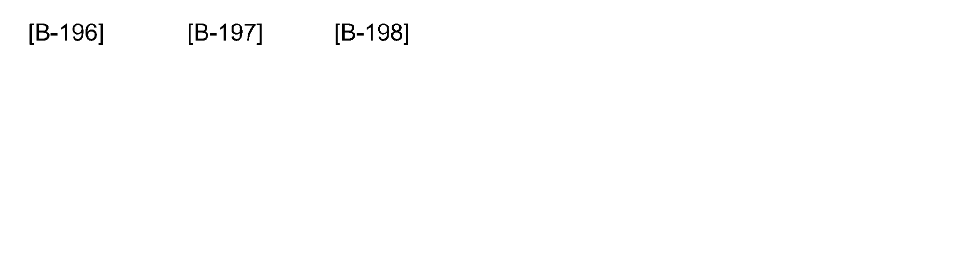

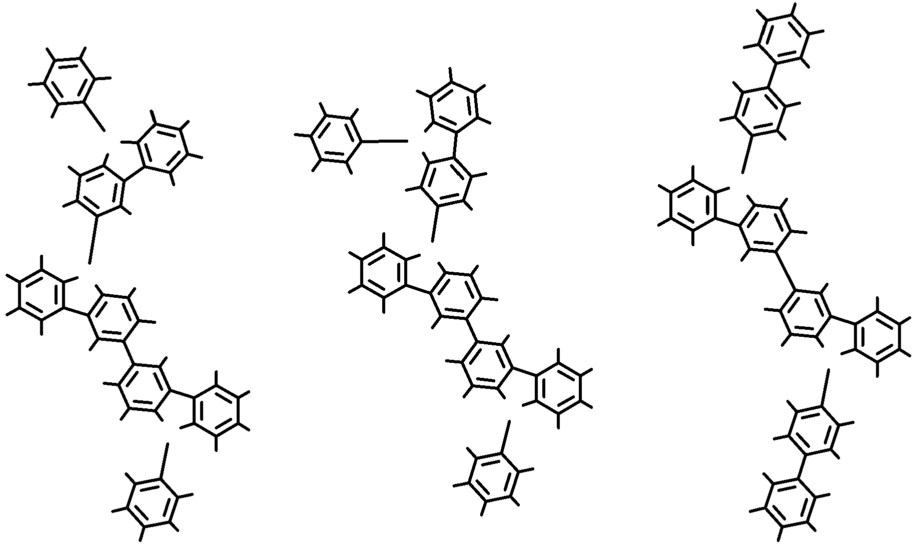

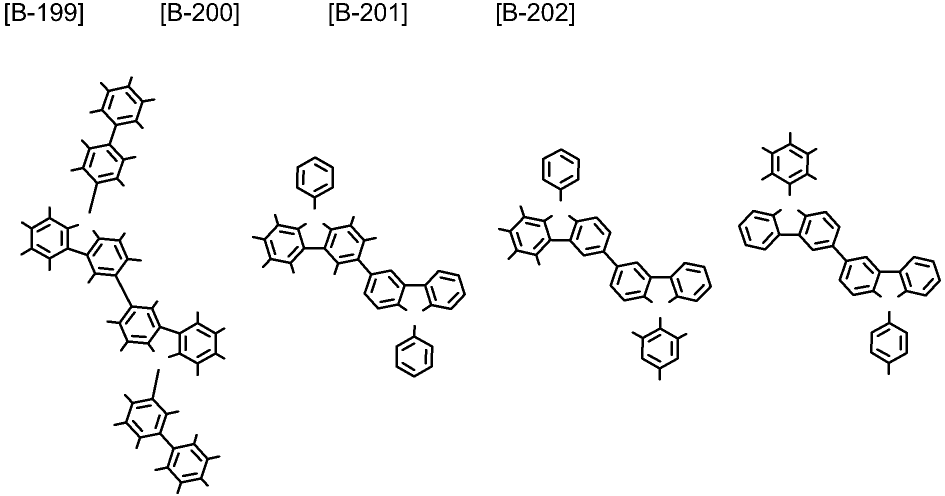

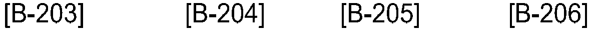

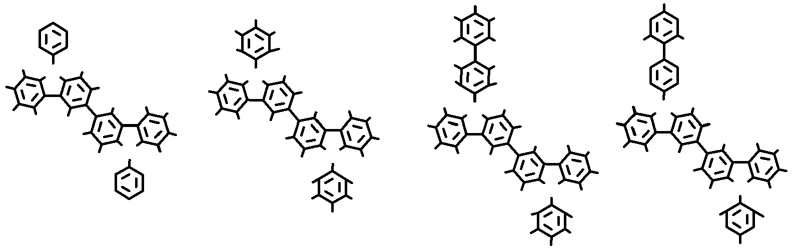

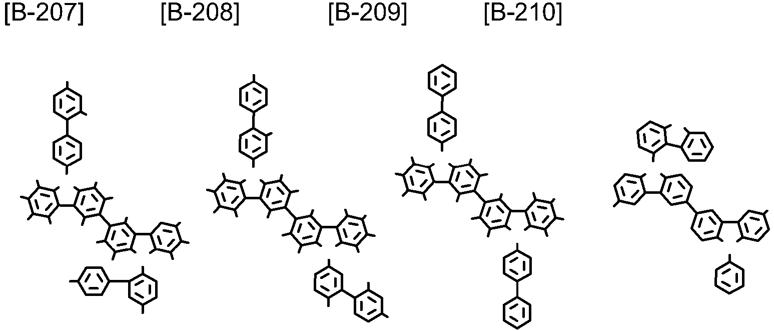

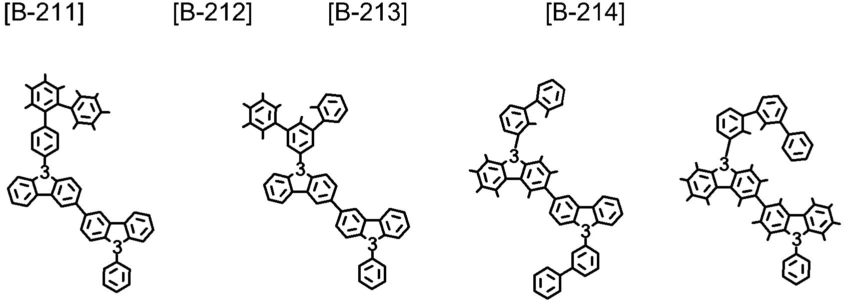

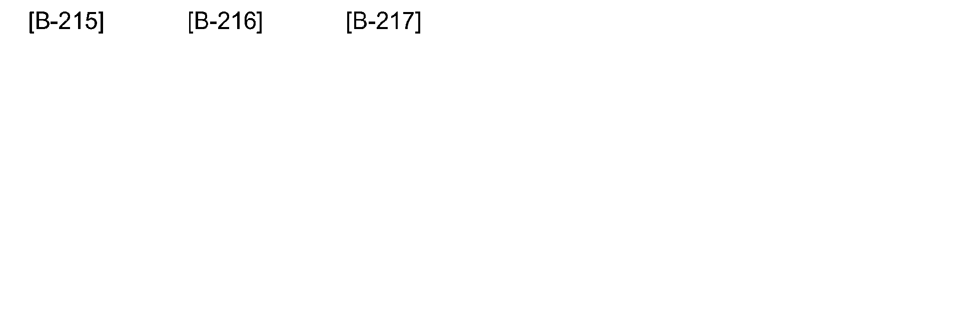

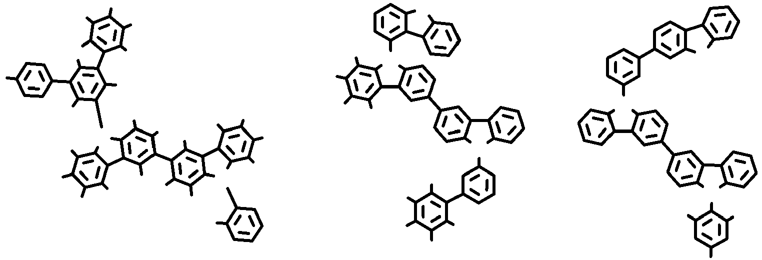

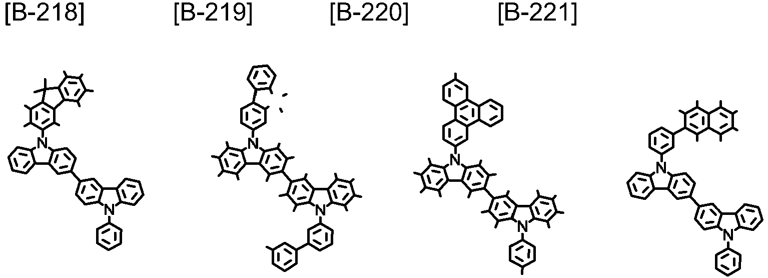

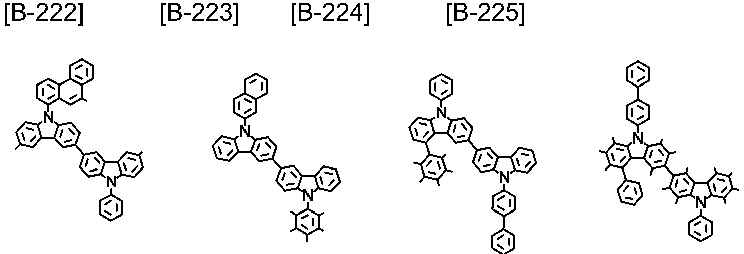

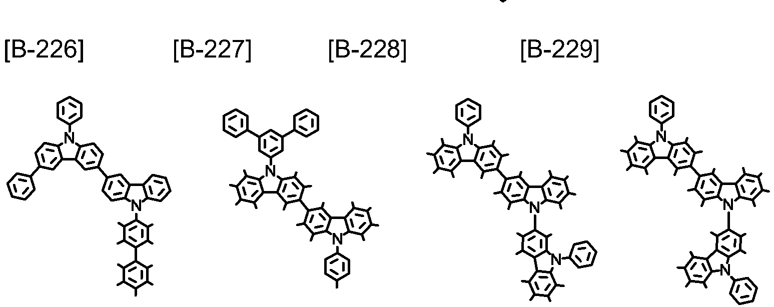

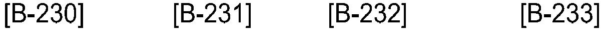

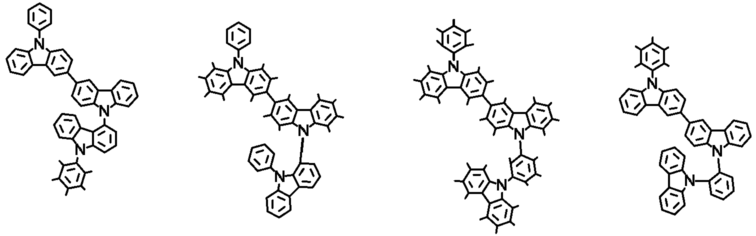

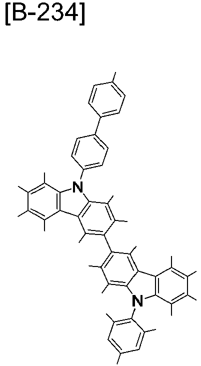

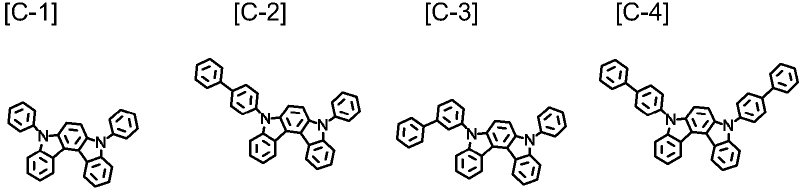

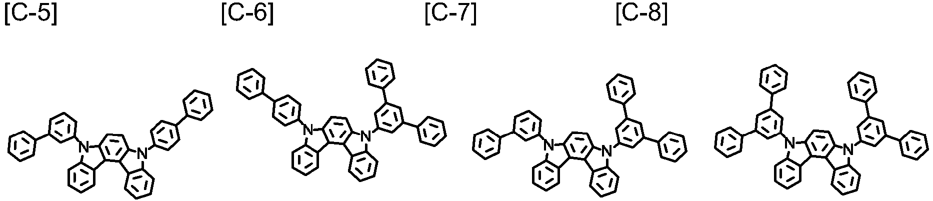

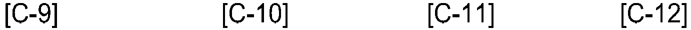

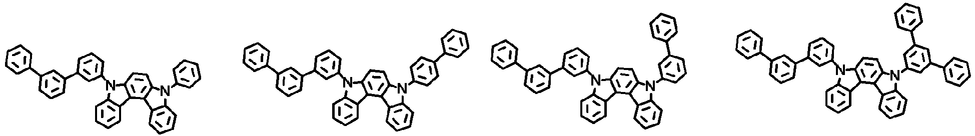

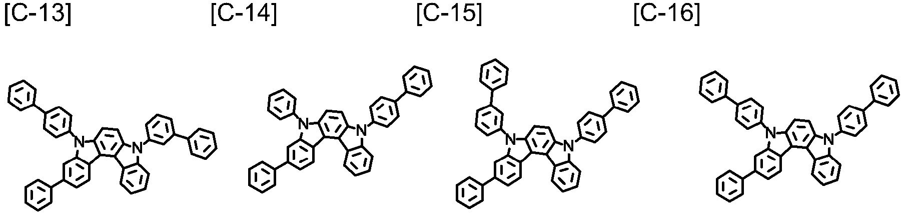

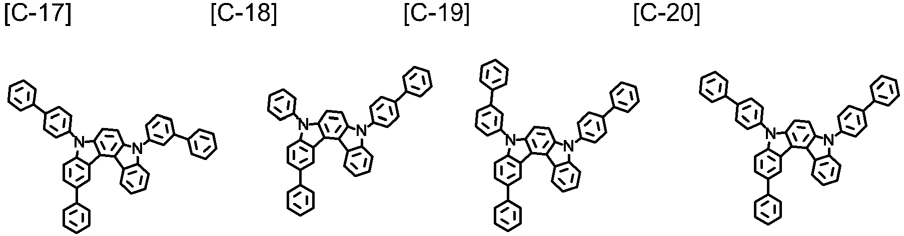

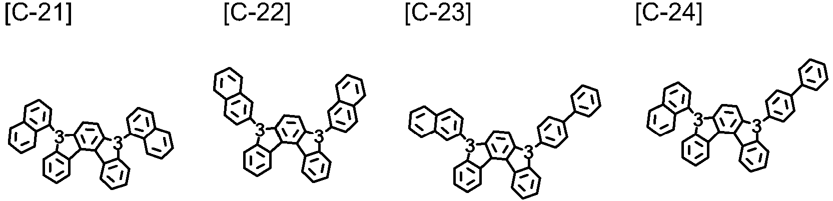

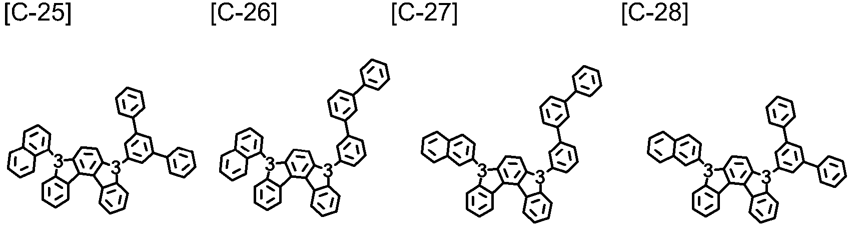

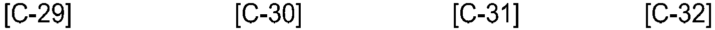

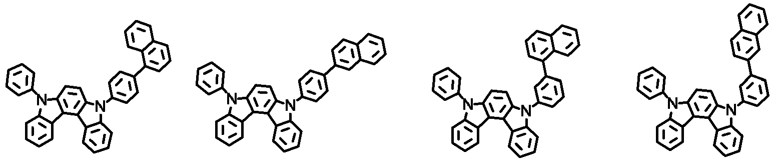

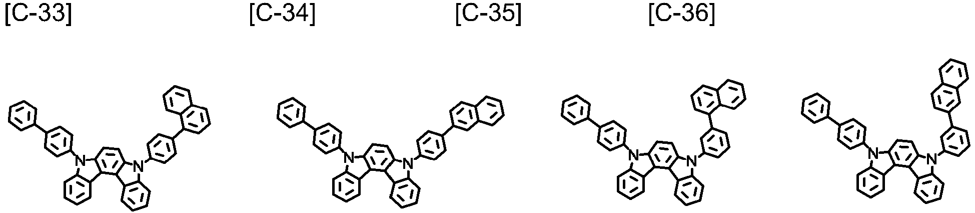

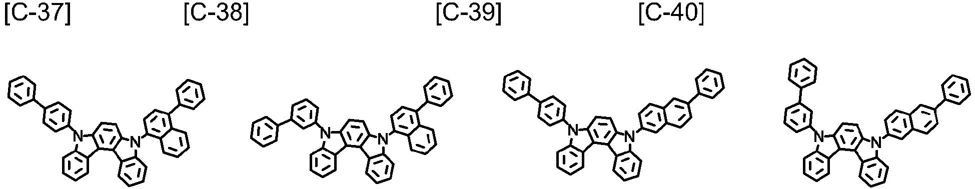

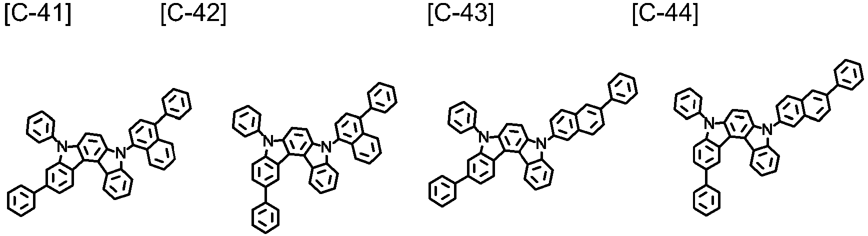

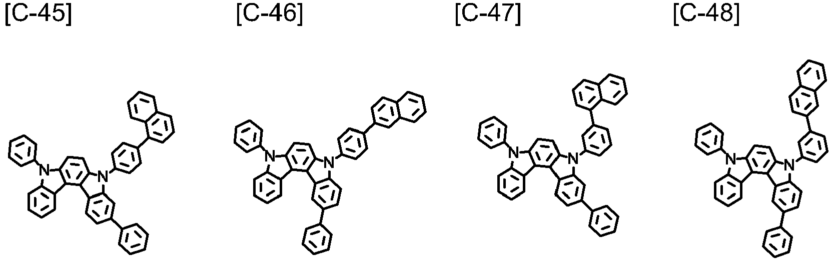

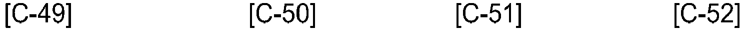

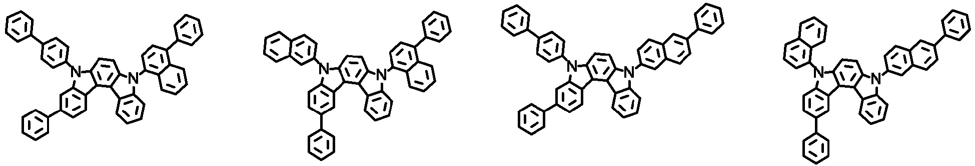

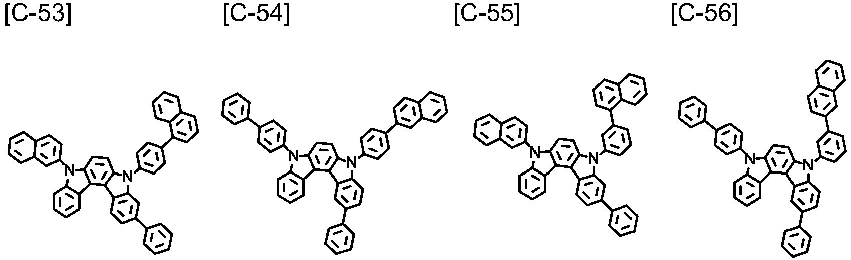

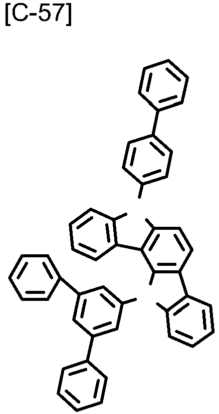

Additionally, examples of Compound C-1 to Compound C-57 listed in Group 2 in which at least one hydrogen is replaced with deuterium are shown below, but the present disclosure is not limited thereto.

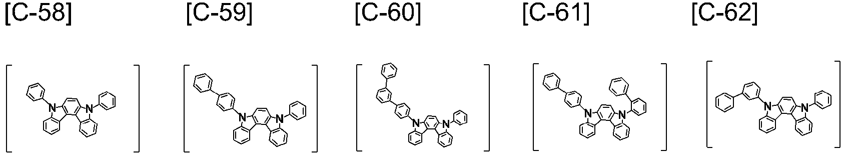

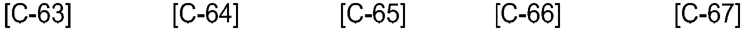

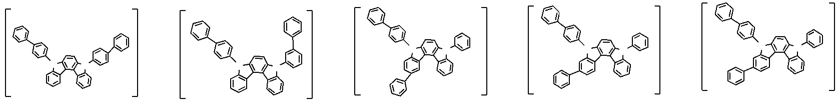

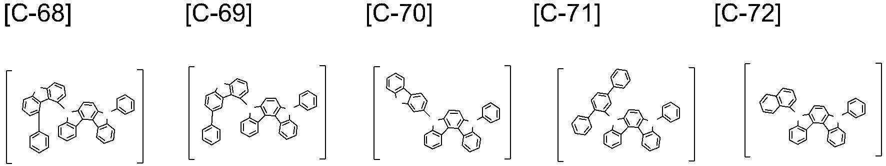

### (Dn refers to the number of deuterium substitutions and indicates a structure substituted with one or more deuteriums)

The most specific structures for Compound C-58 to Compound C-72 of Group 2 are presented below as examples according to the position and substitution degree of deuterium substitution, which is not to limit the scope of rights to compounds not shown below.

The scope of the present invention is determined by the claims, and when deuterium is substituted, it is not limited to the compounds exemplified below, and the position of deuterium substitution and the substitution degree of deuterium substitution may include all changeable ranges within the range of Compound C-1 to Compound C-72.

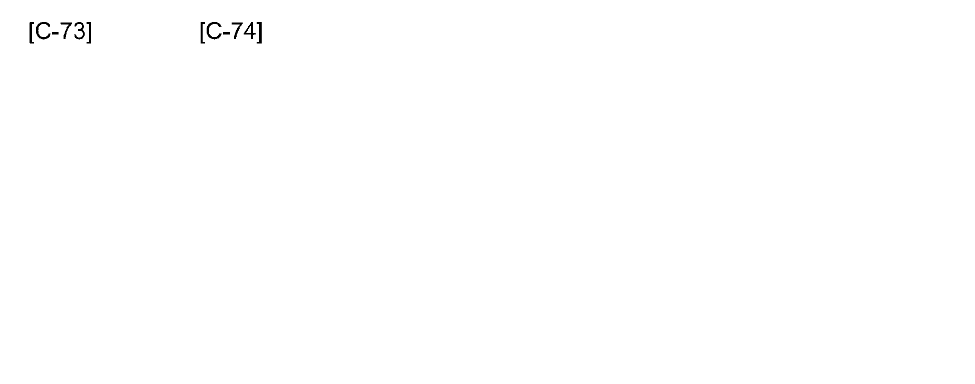

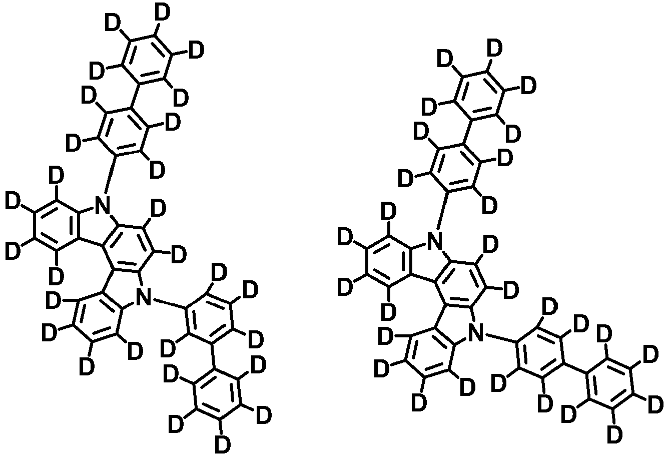

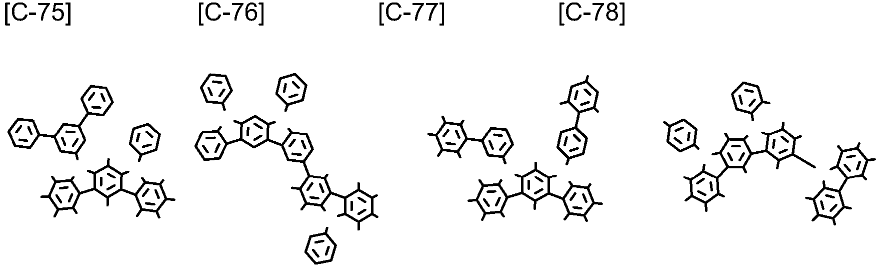

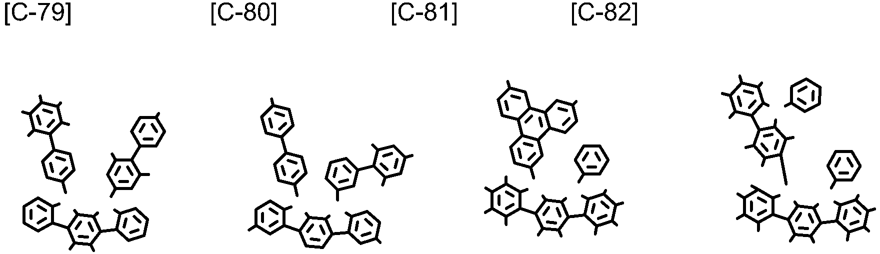

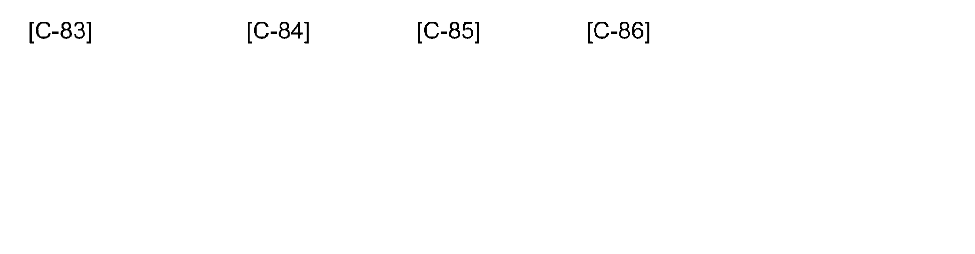

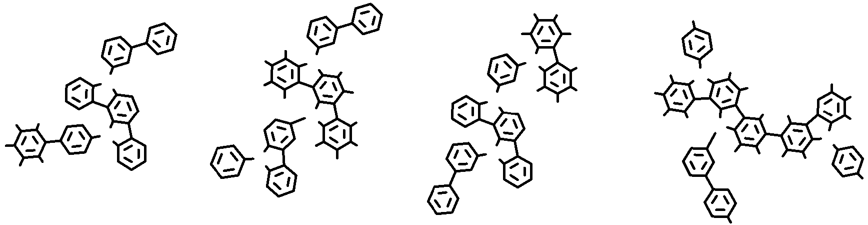

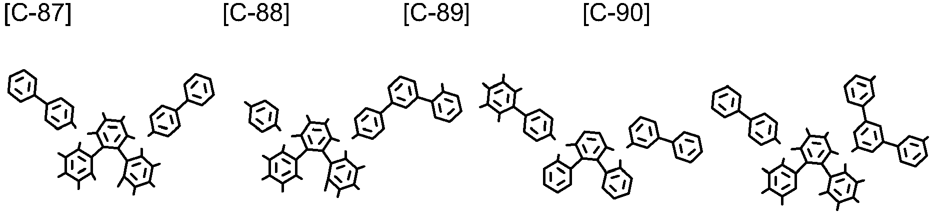

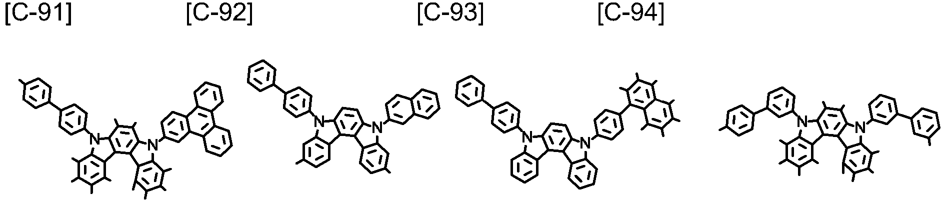

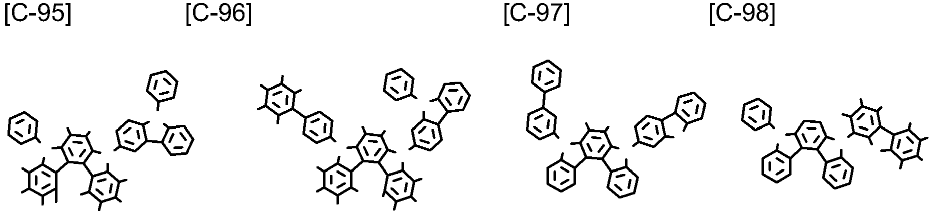

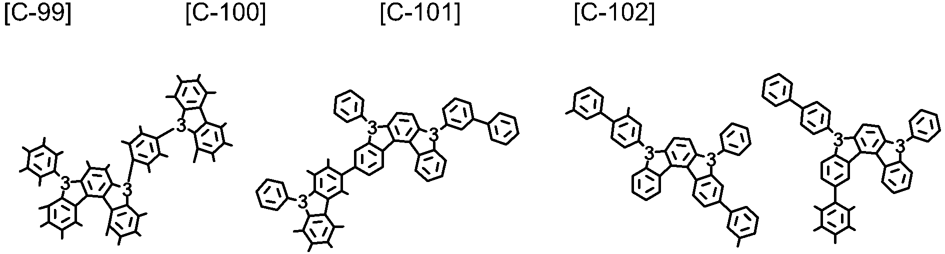

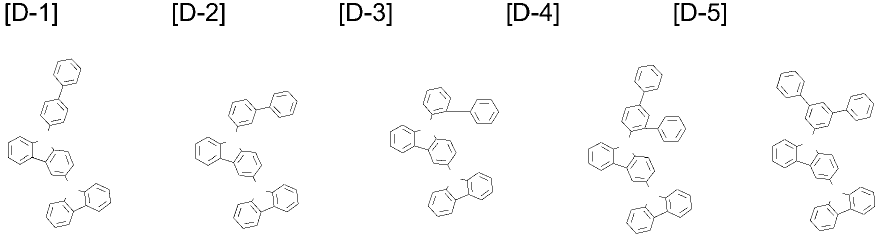

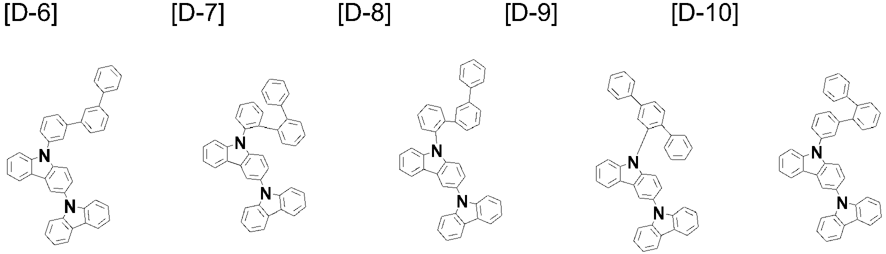

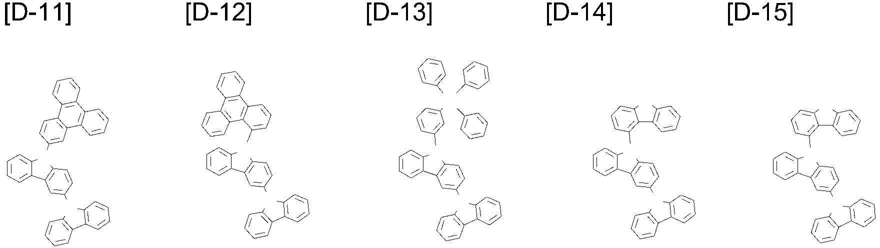

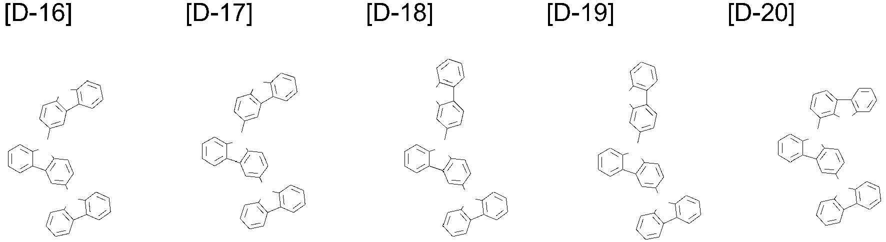

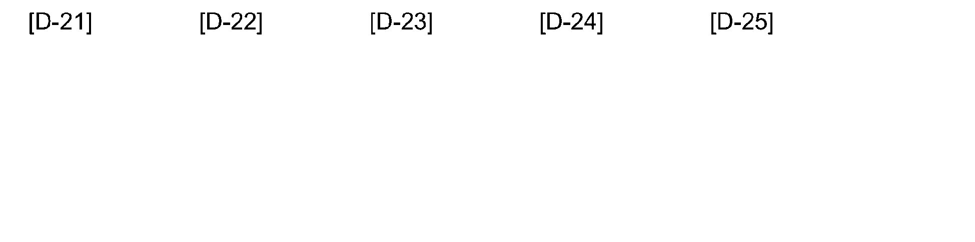

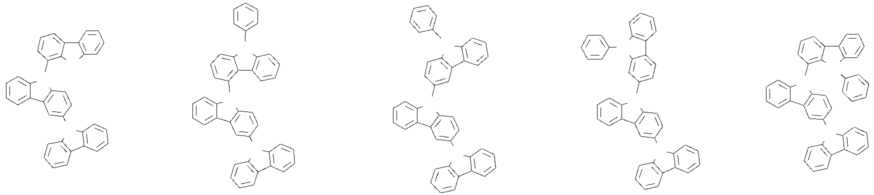

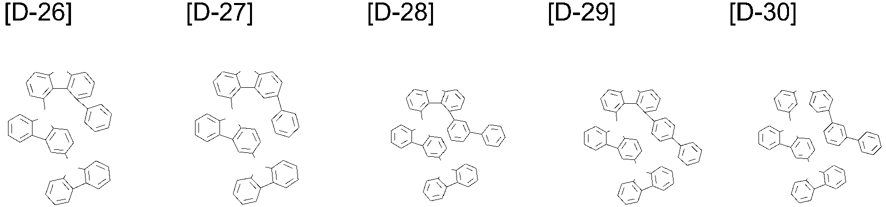

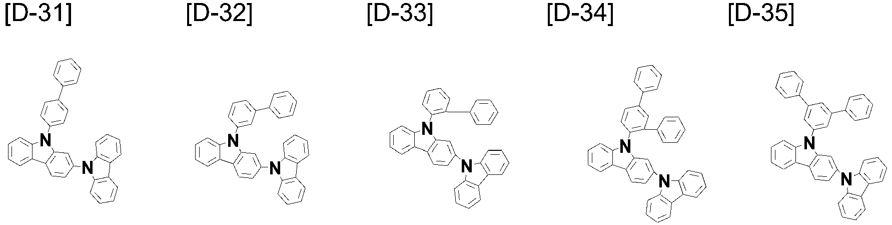

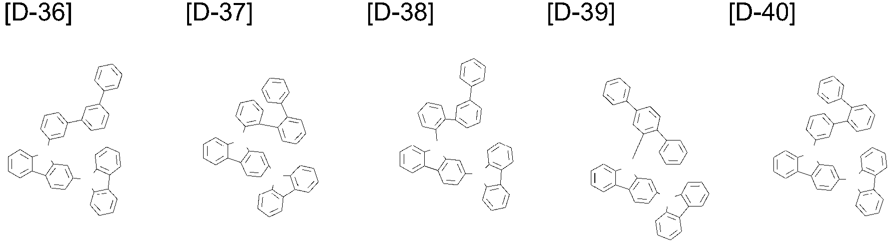

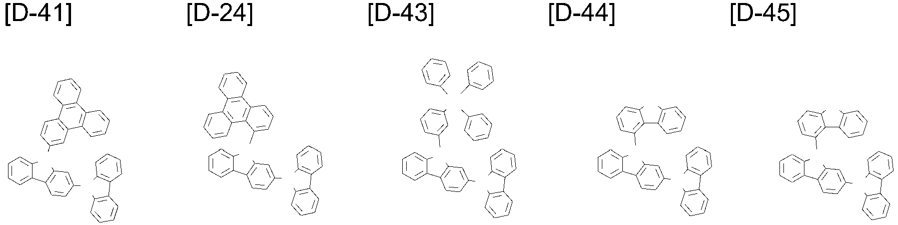

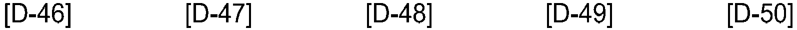

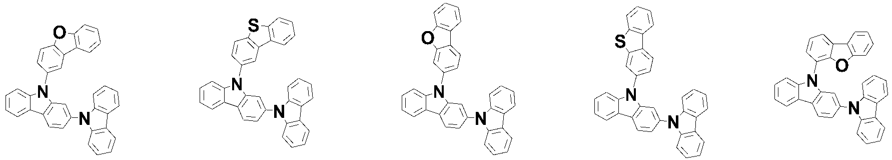

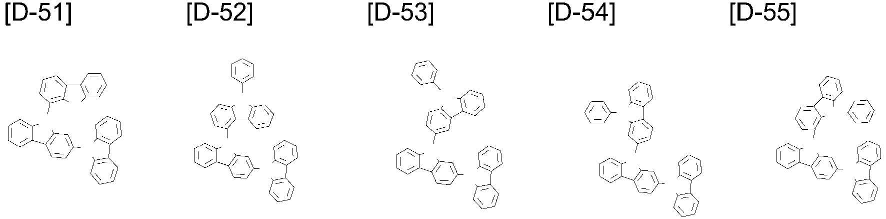

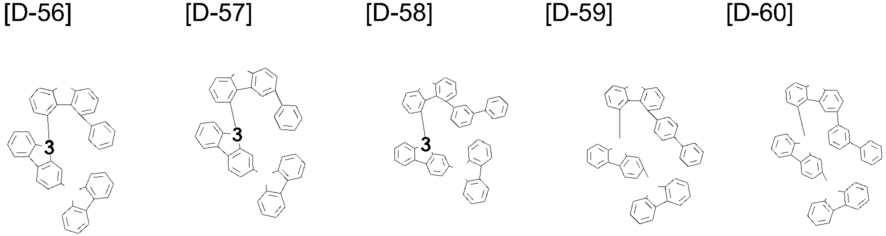

Additionally, examples of Compound D-1 to Compound D-60 listed in Group 2 in which at least one hydrogen is replaced with deuterium are shown below, but the present invention is not limited thereto.

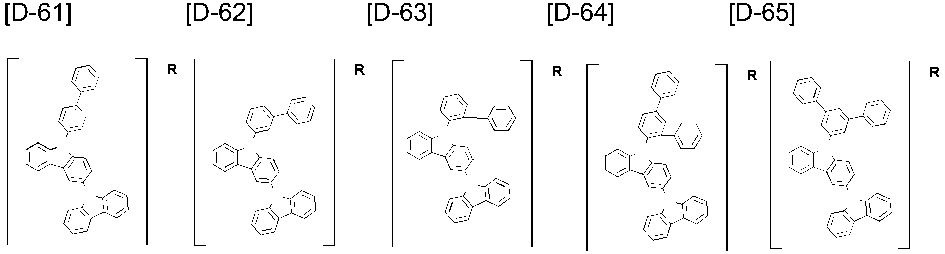

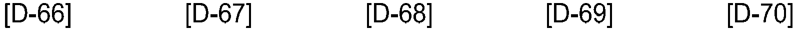

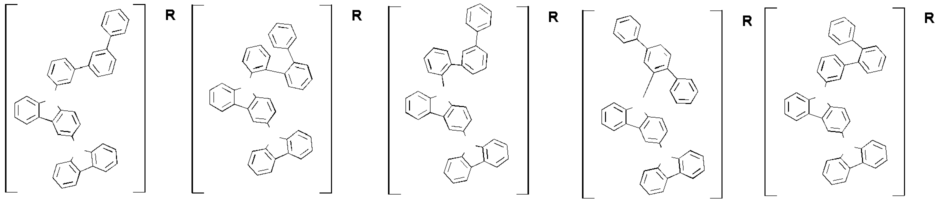

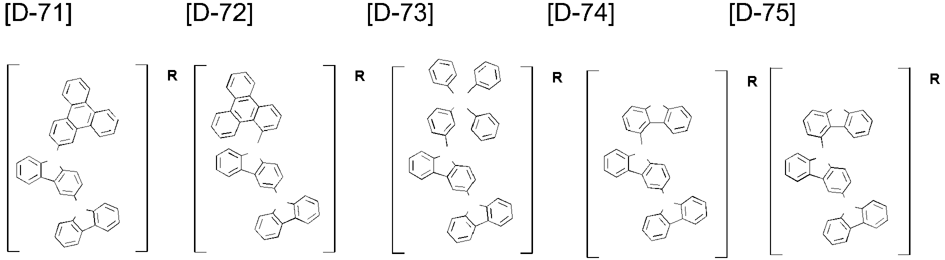

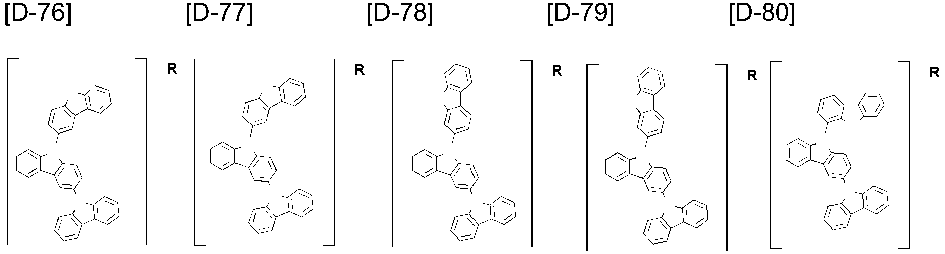

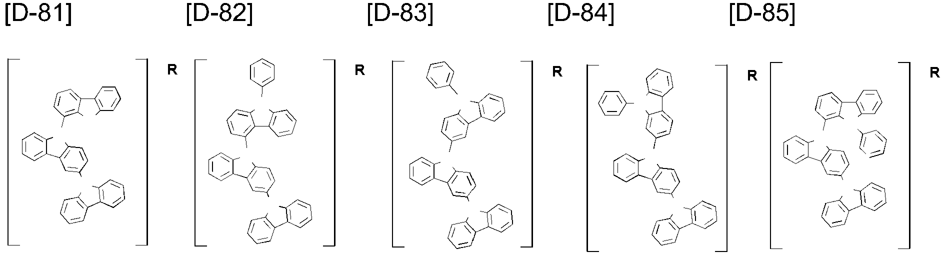

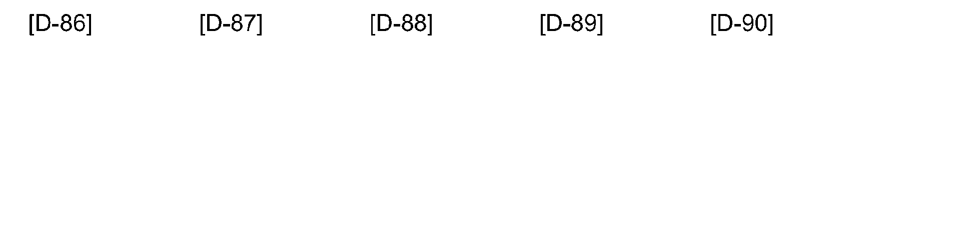

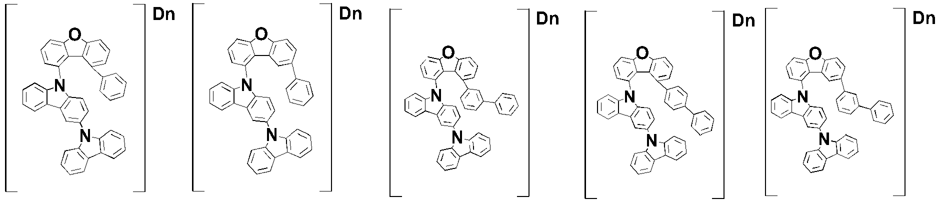

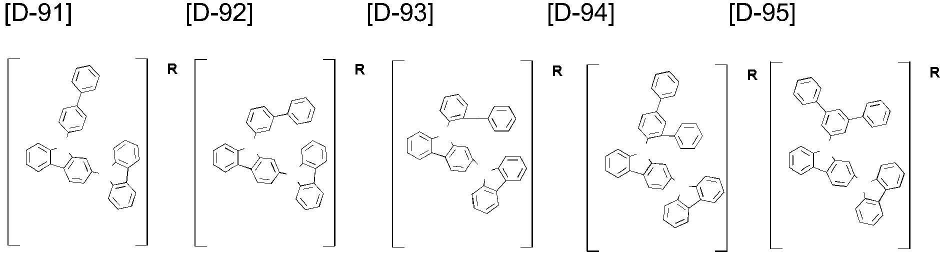

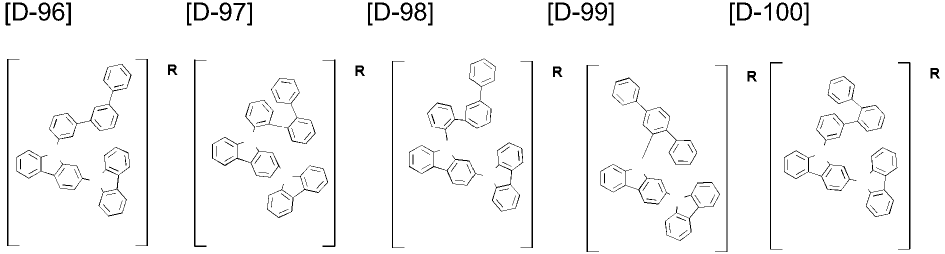

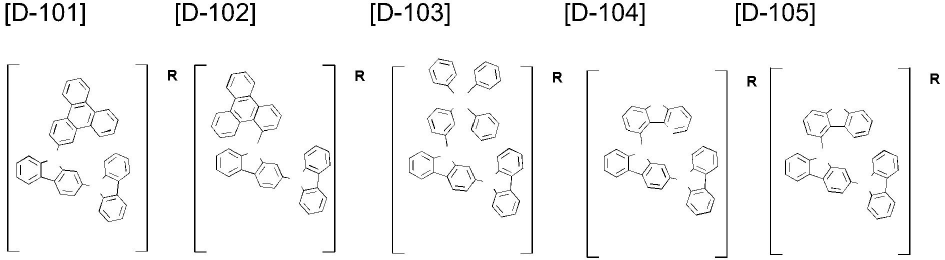

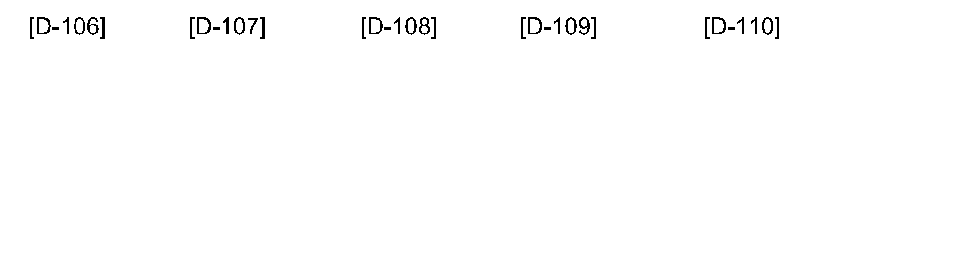

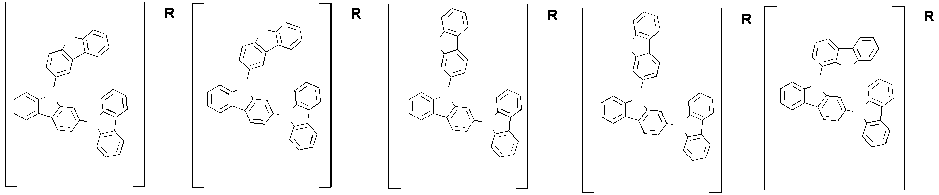

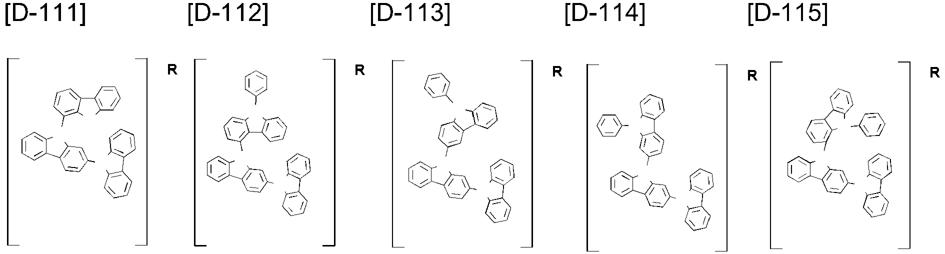

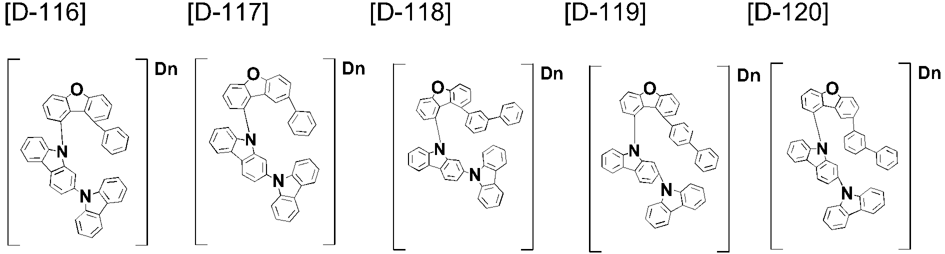

### (Dn refers to the number of deuterium substitutions and indicates a structure substituted with one or more deuteriums)

The first compound and the second compound may be included in a weight ratio of, for example, 1:99 to 99:1. Within the above range, bipolar characteristics may be realized by adjusting an appropriate weight ratio using electron transport capability of the first compound and hole transport capability of the second compound, thereby improving efficiency and life-span. Within the above range, they may be included in a weight ratio of, for example, about 10:90 to 90:10, about 20:80 to 80:20, and for example, they may be included in a weight ratio of, for example, about 20:80 to about 70:30, about 20:80 to about 60:40, and about 30:70 to about 60:40. As a specific example, they may be included in a weight ratio of 40:60, 50:50, or 60:40.

In a most specific embodiment, the second compound may be one represented by a combination of Chemical Formula 3 and Chemical Formula 4.

For example, the second compound may be one represented by Chemical Formula 3C.

Hereinafter, an organic optoelectronic device to which the compound for the organic optoelectronic device or the composition for the organic optoelectronic device described above is applied is described.

The organic optoelectronic device may be a suitable device to convert electrical energy into photoenergy and vice versa, e.g., an organic photoelectric device, an organic light emitting diode, an organic solar cell, or an organic photoconductor drum.

Herein, an organic light emitting diode as one example of an organic optoelectronic device is described referring to drawings.

FIG. 1 is a cross-sectional view showing an organic light emitting diode according to an embodiment.

Referring to FIG. 1, an organic light emitting diode 100 according to an embodiment includes an anode 120 and a cathode 110 facing each other and an organic layer 105 disposed between the anode 120 and cathode 110.

The anode 120 may be made of a conductor having a large work function to help hole injection, and may be for example a metal, a metal oxide and/or a conductive polymer. The anode 120 may be, for example a metal such as nickel, platinum, vanadium, chromium, copper, zinc, gold, and the like or an alloy thereof; a metal oxide such as zinc oxide, indium oxide, indium tin oxide (ITO), indium zinc oxide (IZO), and the like; a combination of a metal and an oxide such as ZnO and Al or SnO₂ and Sb; a conductive polymer such as poly(3-methylthiophene), poly(3,4-(ethylene-1,2-dioxy)thiophene) (PEDOT), polypyrrole, and polyaniline, but is not limited thereto.

The cathode 110 may be made of a conductor having a low work function to facilitate electron injection, for example, and may be made of, for example, a metal, a metal oxide, and/or a conductive polymer. Examples of the cathode 110 include for example, a metal such as magnesium, calcium, sodium, potassium, titanium, indium, yttrium, lithium, gadolinium, aluminum, silver, tin, lead, cesium, and barium or an alloy thereof; and a multi-layered structure material such as LiF/Al, LiO₂/Al, LiF/Ca, LiF/Al, and BaF₂/Ca, but is not limited thereto.

The organic layer 105 may include the aforementioned compound for the organic optoelectronic device or composition for the organic optoelectronic device.

The organic layer 105 includes a light emitting layer 130, the light emitting layer 130 includes a host and a dopant, the host may include the aforementioned compound for the organic optoelectronic device or composition for the organic optoelectronic device, and the dopant may be, for example, a phosphorescent dopant, for example, a red, green, or blue phosphorescent dopant, and for example, a red or green phosphorescent dopant.

The dopant is a material mixed with the compound or composition for an organic optoelectronic device in a small amount to cause light emission, and may be generally a material such as a metal complex that emits light by multiple excitation into a triplet or more. The dopant may be, for example an inorganic, organic, or organic/inorganic compound, and one or more types thereof may be used.

Examples of the dopant may be a phosphorescent dopant and examples of the phosphorescent dopant may be an organic metal compound including Ir, Pt, Os, Ti, Zr, Hf, Eu, Tb, Tm, Fe, Co, Ni, Ru, Rh, Pd, or a combination thereof. The phosphorescent dopant may be, for example a compound represented by Chemical Formula Z, but is not limited thereto.

[Chemical Formula Z] L⁸MX

In Chemical Formula Z, M is a metal, and L⁸ and X are the same or different and are ligands to form a complex compound with M.

The M may be for example Ir, Pt, Os, Ti, Zr, Hf, Eu, Tb, Tm, Fe, Co, Ni, Ru, Rh, Pd, or a combination thereof, and L⁸ and X may be for example a bidentate ligand.

Examples of ligands represented by L⁸ and X may be selected from the chemical formulas listed in Group A, but are not limited thereto.

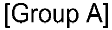

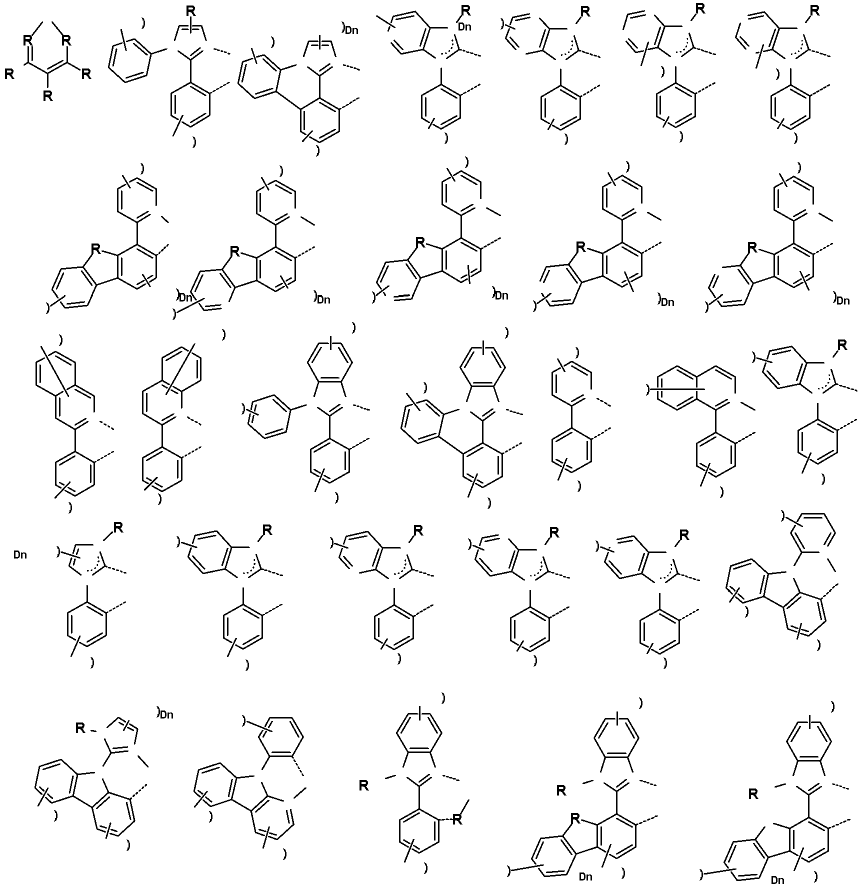

In Group A,
R³⁰⁰ to R³⁰² are each independently hydrogen, deuterium, a C1 to C30 alkyl group that is substituted or unsubstituted with a halogen, a C6 to C30 aryl group that is substituted or unsubstituted with a C1 to C30 alkyl, or a halogen, and
R³⁰³ to R³²⁴ are each independently hydrogen, deuterium, a halogen, a substituted or unsubstituted C1 to C30 alkyl group, a substituted or unsubstituted C1 to C30 alkoxy group, a substituted or unsubstituted C3 to C30 cycloalkyl group, a substituted or unsubstituted C2 to C30 alkenyl group, a substituted or unsubstituted C6 to C30 aryl group, a substituted or unsubstituted C1 to C30 heteroaryl group, a substituted or unsubstituted C1 to C30 amino group, a substituted or unsubstituted C6 to C30 arylamino group, SF₅, a trialkylsilyl group having a substituted or unsubstituted C1 to C30 alkyl group, a dialkylarylsilyl group having a substituted or unsubstituted C1 to C30 alkyl group and a C6 to C30 aryl group, or a triarylsilyl group having a substituted or unsubstituted C6 to C30 aryl group.

In an embodiment, the dopant may be an iridium complex, and may be represented by Chemical Formula 6-1 or Chemical Formula 6-2.

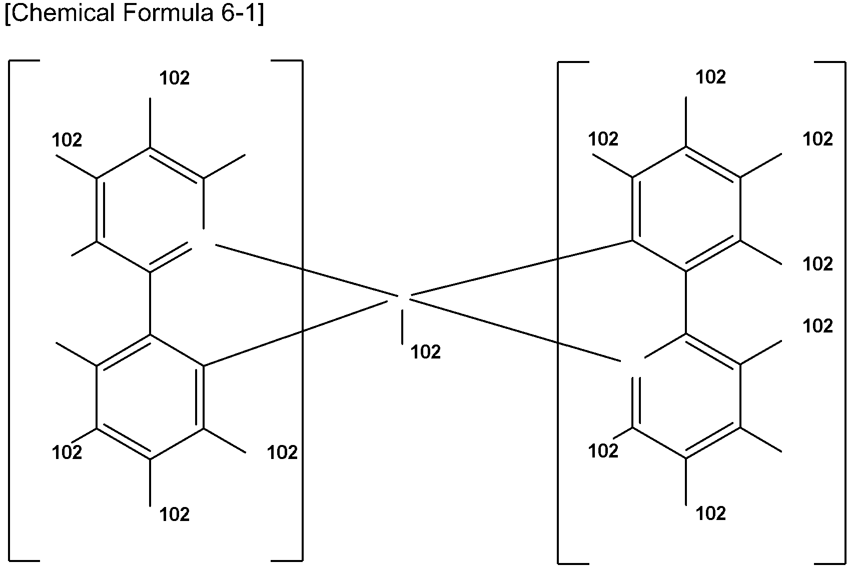

In Chemical Formula 6-1,
R¹⁰¹ to R¹¹⁶ are each independently hydrogen, deuterium, a substituted or unsubstituted C1 to C10 alkyl group, a substituted or unsubstituted C6 to C20 aryl group, or -SiR¹³²R¹³³R¹³⁴,
R¹³² to R¹³⁴ are each independently a substituted or unsubstituted C1 to C6 alkyl group,
at least one of R¹⁰¹ to R¹¹⁶ is a functional group represented by Chemical Formula V-1,
L¹⁰⁰ is a bidentate ligand of a monovalent anion, and is a ligand that coordinates to iridium through a lone pair of carbons or heteroatoms, and
m21 and m22 are each independently any one of integers of 0 to 3, and m21 + m22 is any one of integers from 1 to 3, 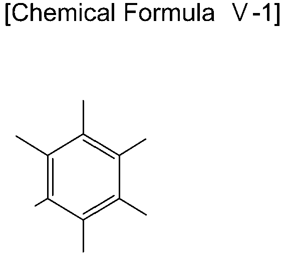
wherein, in Chemical Formula V-1,
R¹³⁵ to R¹³⁹ are each independently hydrogen, deuterium, a substituted or unsubstituted C1 to C10 alkyl group, a substituted or unsubstituted C6 to C20 aryl group, or -SiR¹³²R¹³³R¹³⁴, and
* refers to a portion linked to a carbon atom. 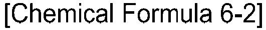 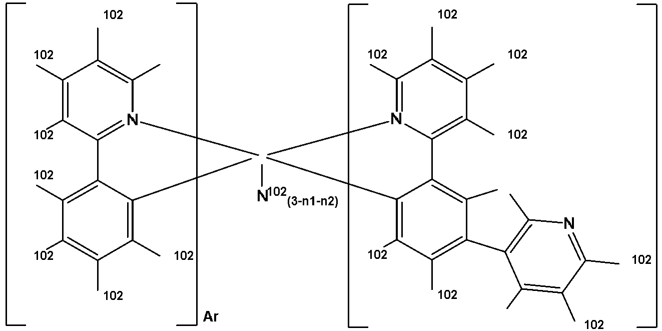
wherein, in Chemical Formula 4-2,
R¹⁰¹ to R¹¹⁷ are each independently hydrogen, deuterium, a substituted or unsubstituted C1 to C10 alkyl group, a substituted or unsubstituted C6 to C20 aryl group, or -SiR¹³³R¹³⁴R¹³⁵,
R¹³³ to R¹³⁵ are each independently a substituted or unsubstituted C1 to C6 alkyl group,
L¹⁰⁰ is a bidentate ligand of a monovalent anion, and is a ligand that coordinates to iridium through a lone pair of carbons or heteroatoms, and
n1 and n2 are each independently any integer from 0 to 3, and n1 + n2 is any one of integers from 1 to 3.

In another embodiment, the dopant may be a platinum complex, for example represented by Chemical Formula Z-1.

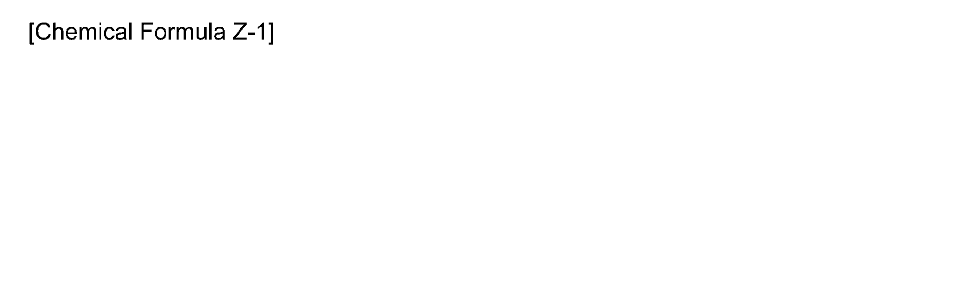

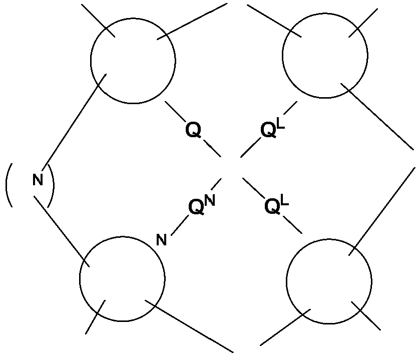

In Chemical Formula Z-1, rings A, B, C, and D independently represent a 5-membered or 6-membered carbocyclic or heterocyclic ring;
R^{A}, R^{B}, R^{C}, and R^{D} independently represent mono-, di-, tri-, or tetra-substitution, or unsubstitution;
L^{B}, L^{C}, and L^{D} are each independent selected from a direct bond, BR, NR, PR, O, S, Se, C=O, S=O, SO₂, CRR', SiRR', GeRR', and a combination thereof;
when nA is 1, L^{E} is selected from a direct bond, BR, NR, PR, O, S, Se, C=O, S=O, SO₂, CRR', SiRR', GeRR', and a combination thereof; when nA is 0, L^{E} does not exist; and
R^{A}, R^{B}, R^{C}, R^{D}, R, and R' are each independently selected from hydrogen, deuterium, a halogen, alkyl group, a cycloalkyl group, a heteroalkyl group, arylalkyl group, an alkoxy group, aryloxy group, an amino group, a silyl group, an alkenyl group, a cycloalkenyl group, a heteroalkenyl group, an alkynyl group, aryl group, a heteroaryl group, an acyl group, a carbonyl group, a carboxylic acid group, an ester group, a nitrile group, an isonitrile group, a sulfanyl group, a sulfinyl group, a sulfonyl group, a phosphino group, and a combination thereof; any adjacent R^{A}, R^{B}, R^{C}, R^{D}, R, and R' are optionally linked to each other to provide a ring; X^{B}, X^{C}, X^{D}, and X^{E} are each independently selected from carbon and nitrogen; and Q¹, Q², Q³, and Q⁴ each represent oxygen or a direct bond.

The platinum complex may be represented, for example, by Chemical Formula 7-1 or Chemical Formula 7-2.

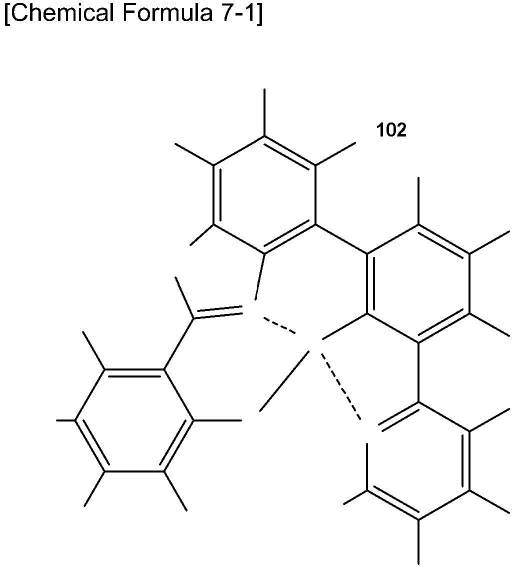

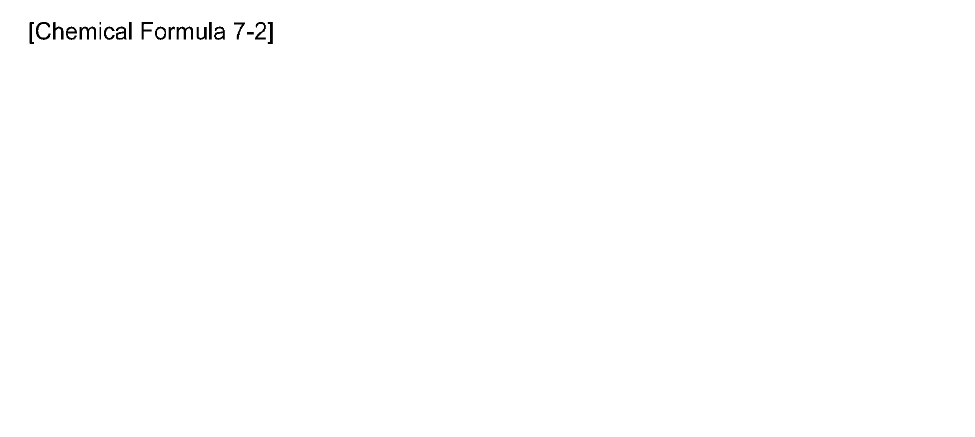

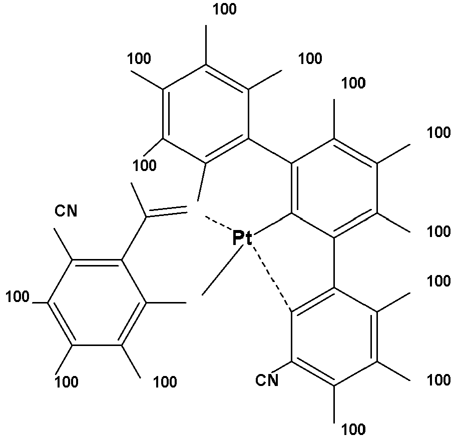

In Chemical Formula 7-1 and Chemical Formula 7-2,
X¹⁰⁰ is selected from O, S, and NR¹³²,
R¹¹⁸ to R¹³² are each independently hydrogen, deuterium, a substituted or unsubstituted C1 to C10 alkyl group, a substituted or unsubstituted C6 to C20 aryl group, or -SiR¹³³R¹³⁴R¹³⁵,
R¹³³ to R¹³⁵ are each independently a substituted or unsubstituted C1 to C6 alkyl group,
at least one of R¹¹⁸ to R¹³² is -SiR¹³³R¹³⁴R¹³⁵ or a tert-butyl group, and
R¹³³ to R¹³⁵ are each independently a substituted or unsubstituted C1 to C6 alkyl group.

The organic layer may further include a charge transport region in addition to the light emitting layer.

The charge transport region may be, for example, a hole transport region 140.

The hole transport region 140 may further increase hole injection and/or hole mobility between the anode 120 and the light emitting layer 130 and block electrons.

Specifically, the hole transport region 140 may include a hole transport layer between the anode 120 and the light emitting layer 130, and a hole transport auxiliary layer between the light emitting layer 130 and the hole transport layer, and at least one of compounds listed in Group B below may be included in at least one layer of the hole transport layer and the hole transport auxiliary layer.

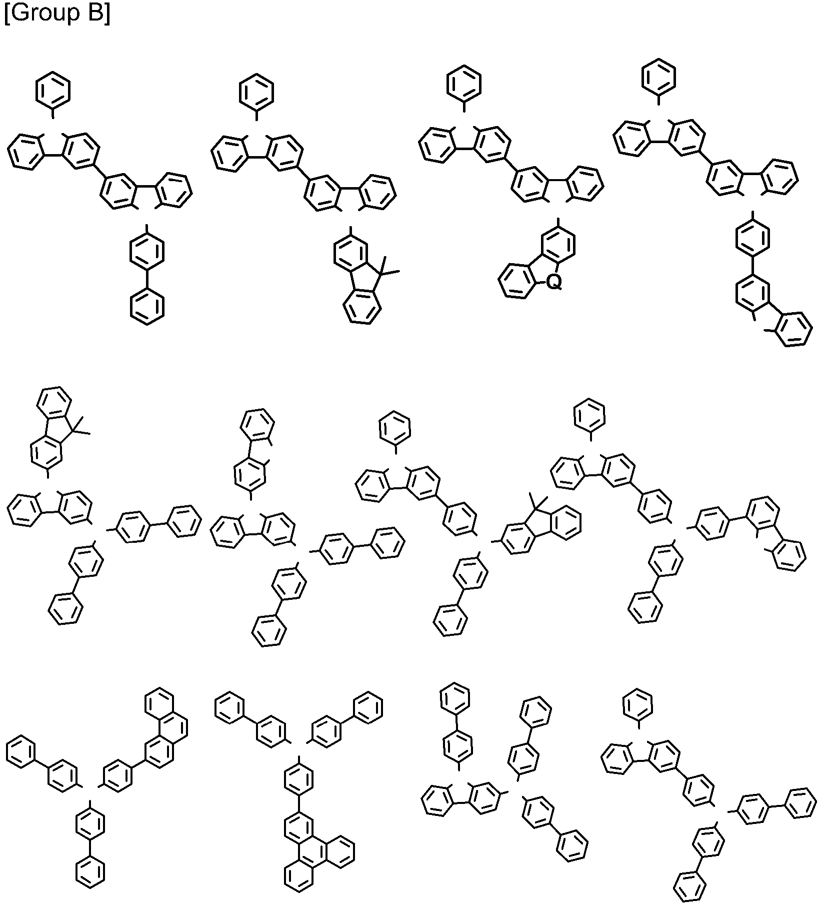

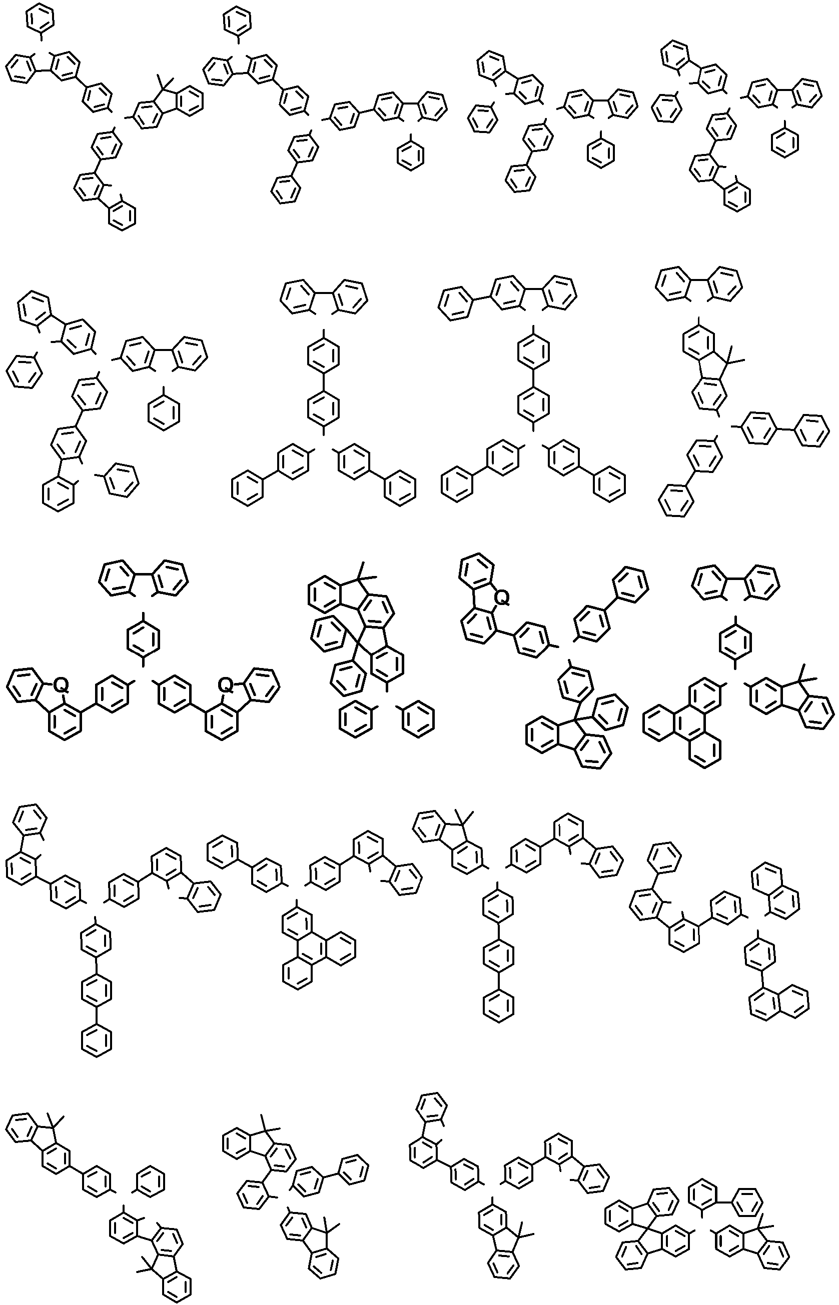

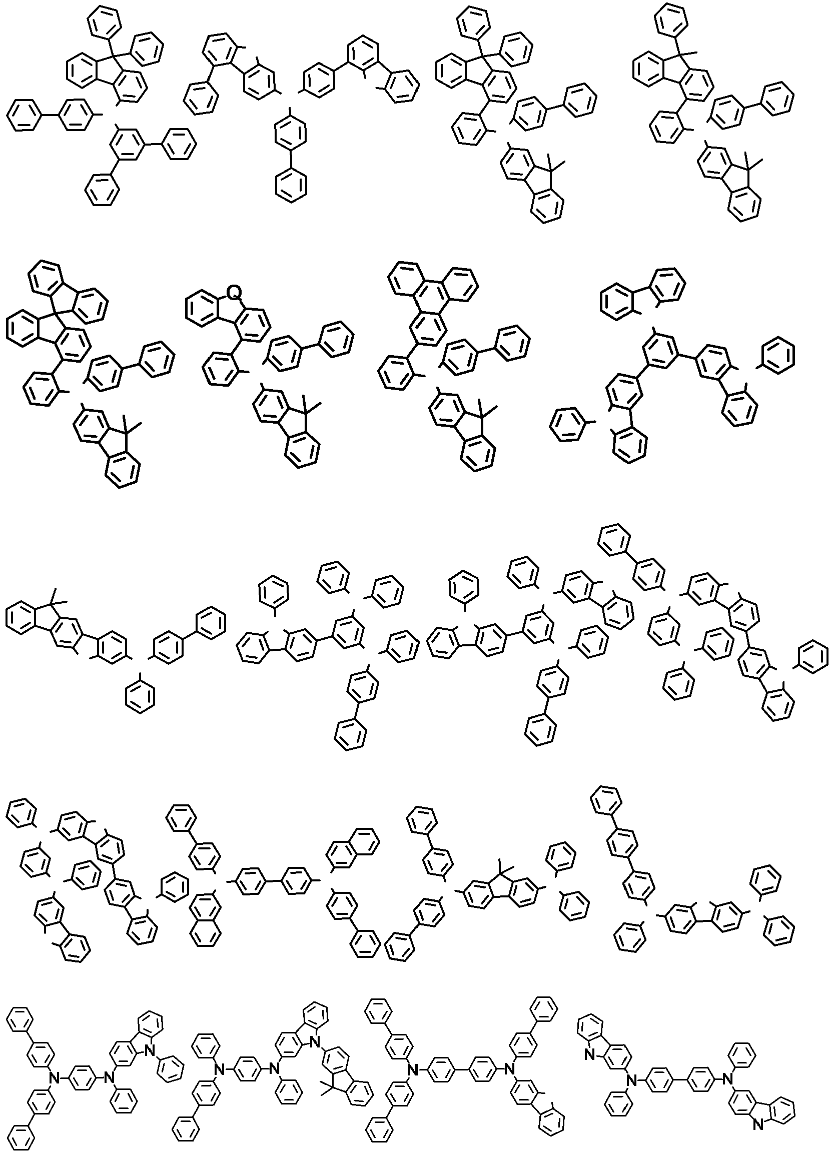

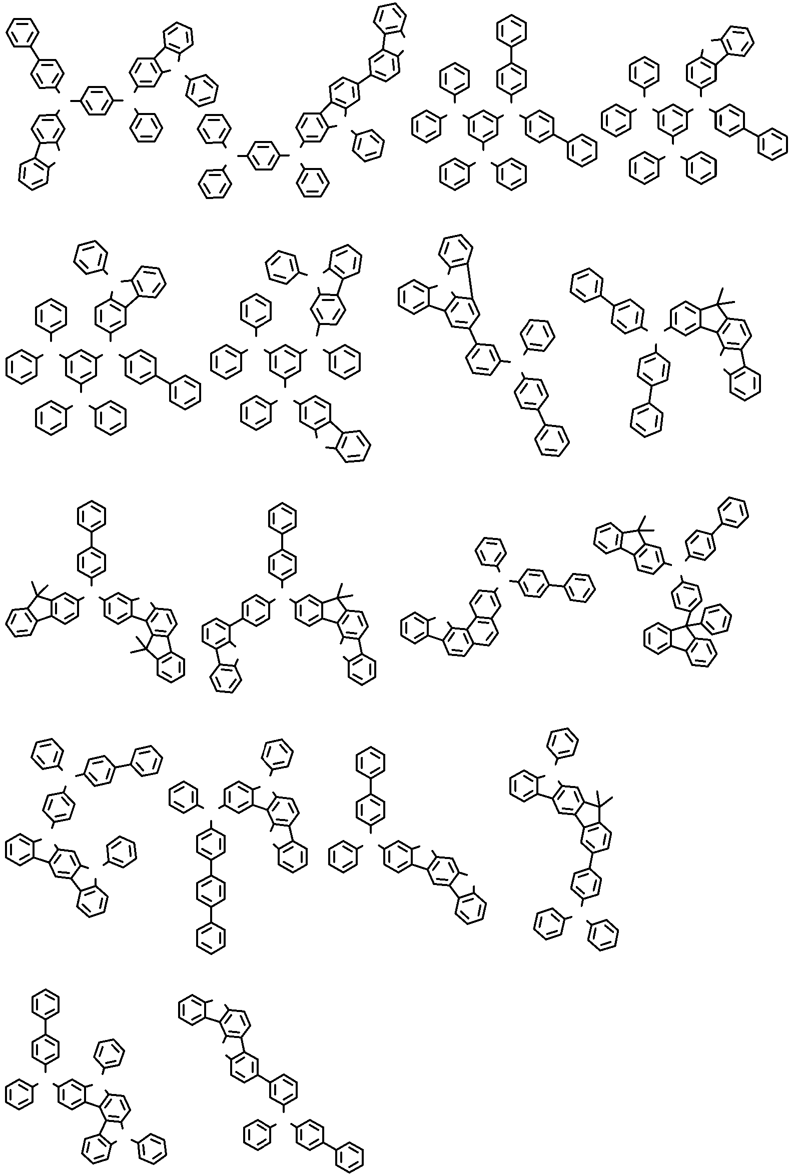

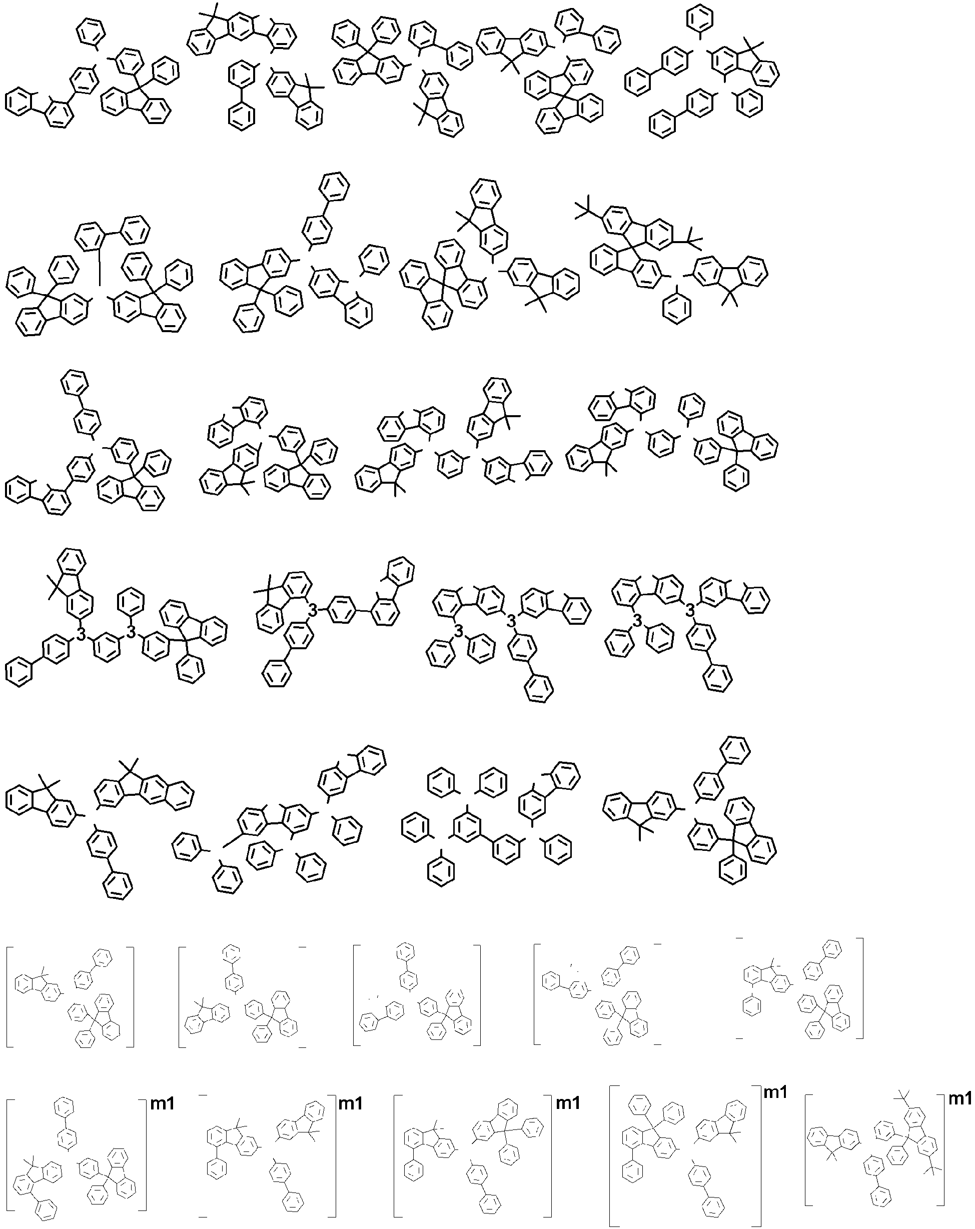

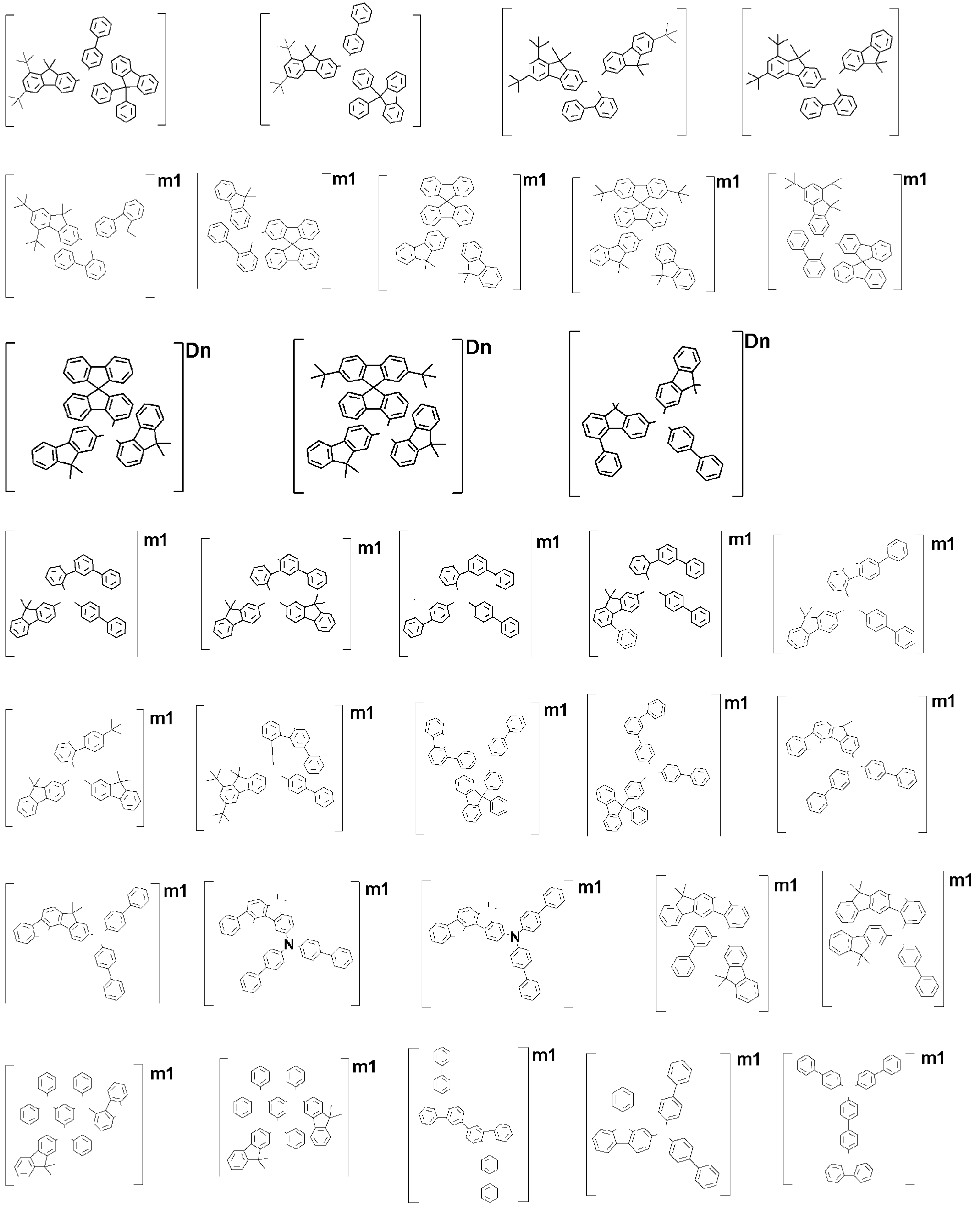

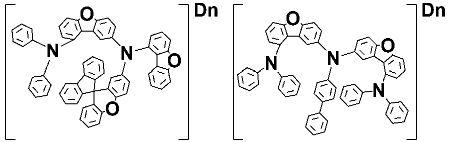

### (Dn refers to the number of deuterium substitutions and indicates a structure substituted with one or more deuteriums)

In addition to the compounds described above, known compounds and compounds having structures similar thereto described in US5061569A, JP1993-009471A, WO1995-009147A1, JP1995-126615A, JP1998-095973A, and the like may also be used in the hole transport region 140.

Additionally, the charge transport region may be, for example, an electron transport region 150.

The electron transport region 150 may further increase electron injection and/or electron mobility between the cathode 110 and the light emitting layer 130 and block holes.

Specifically, the electron transport region 150 may include an electron transport layer between the cathode 110 and the light emitting layer 130, and an electron transport auxiliary layer between the light emitting layer 130 and the electron transport layer, and at least one of the compounds listed in Group C below may be included in at least one layer of the electron transport layer and the electron transport auxiliary layer.

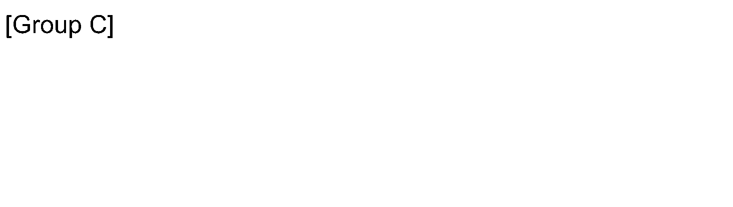

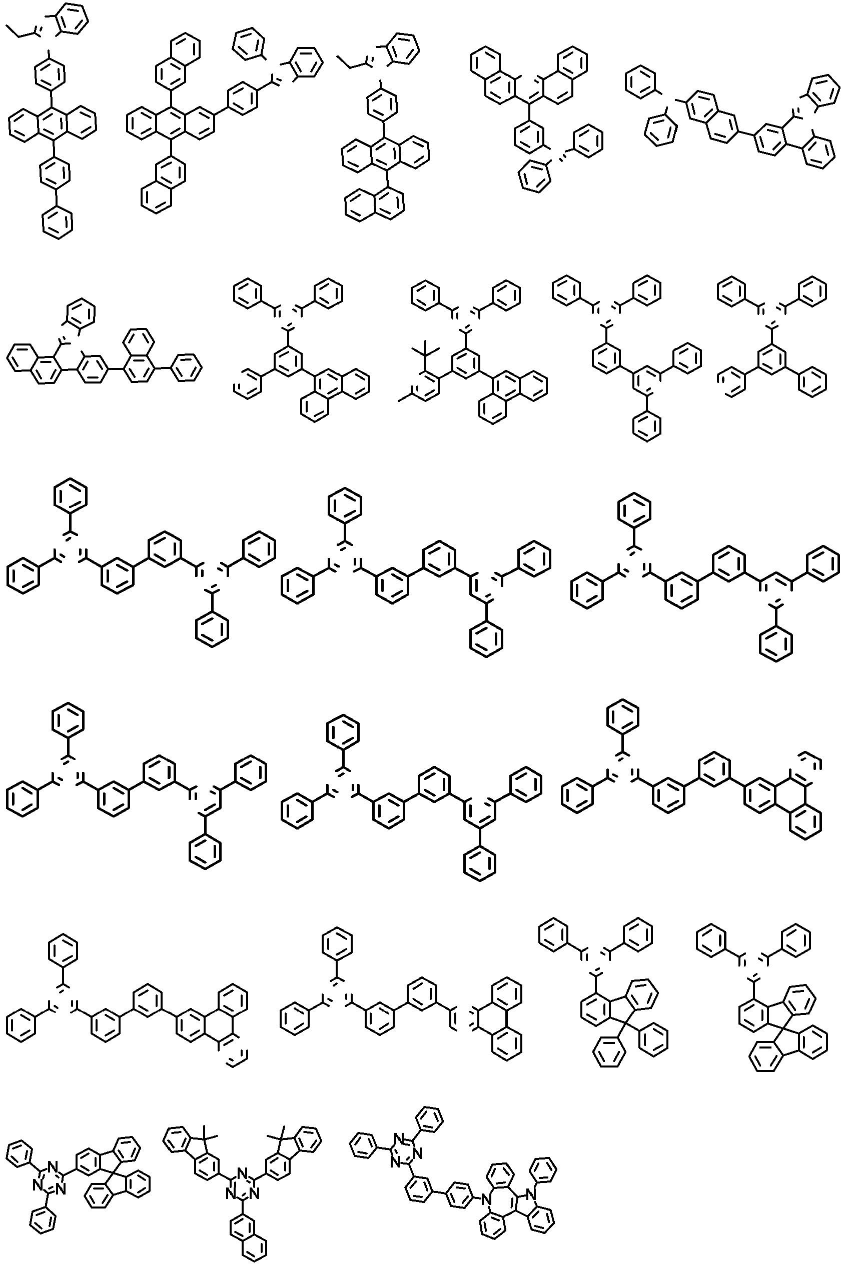

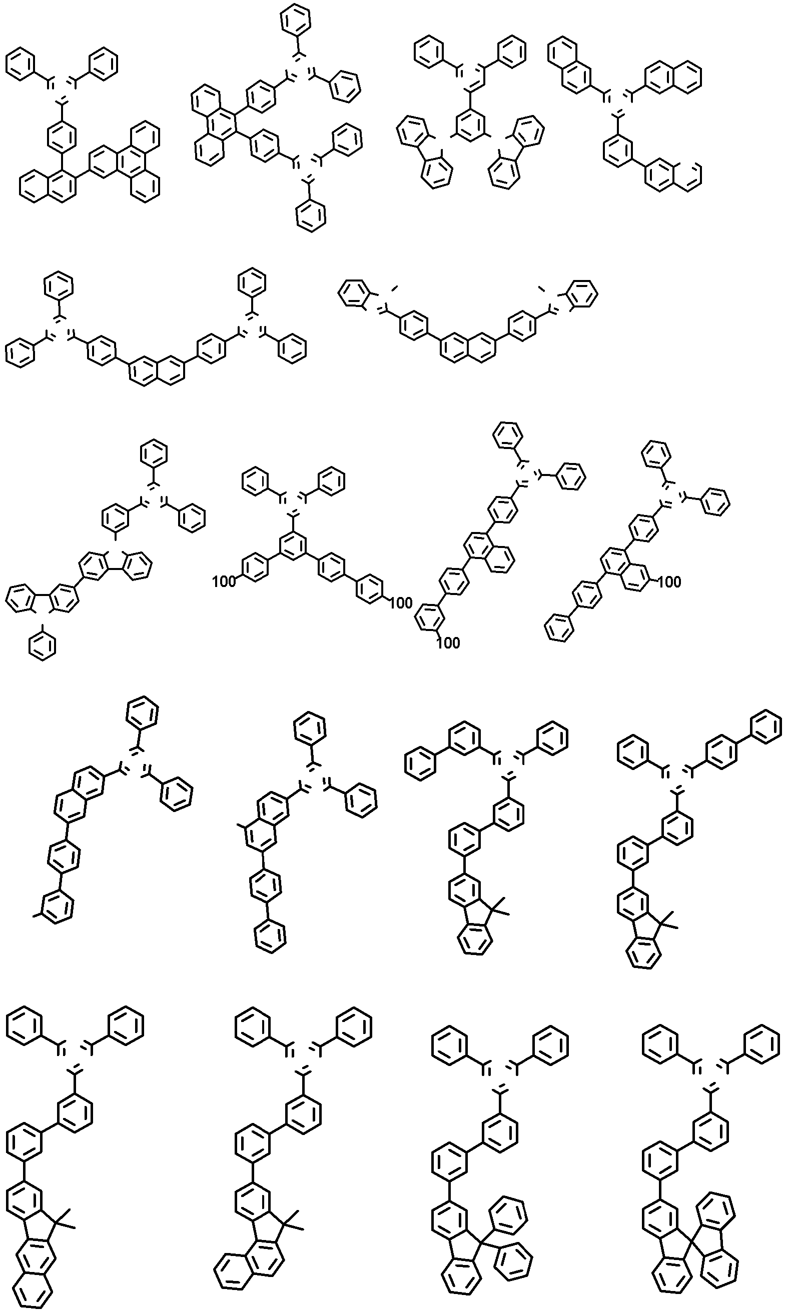

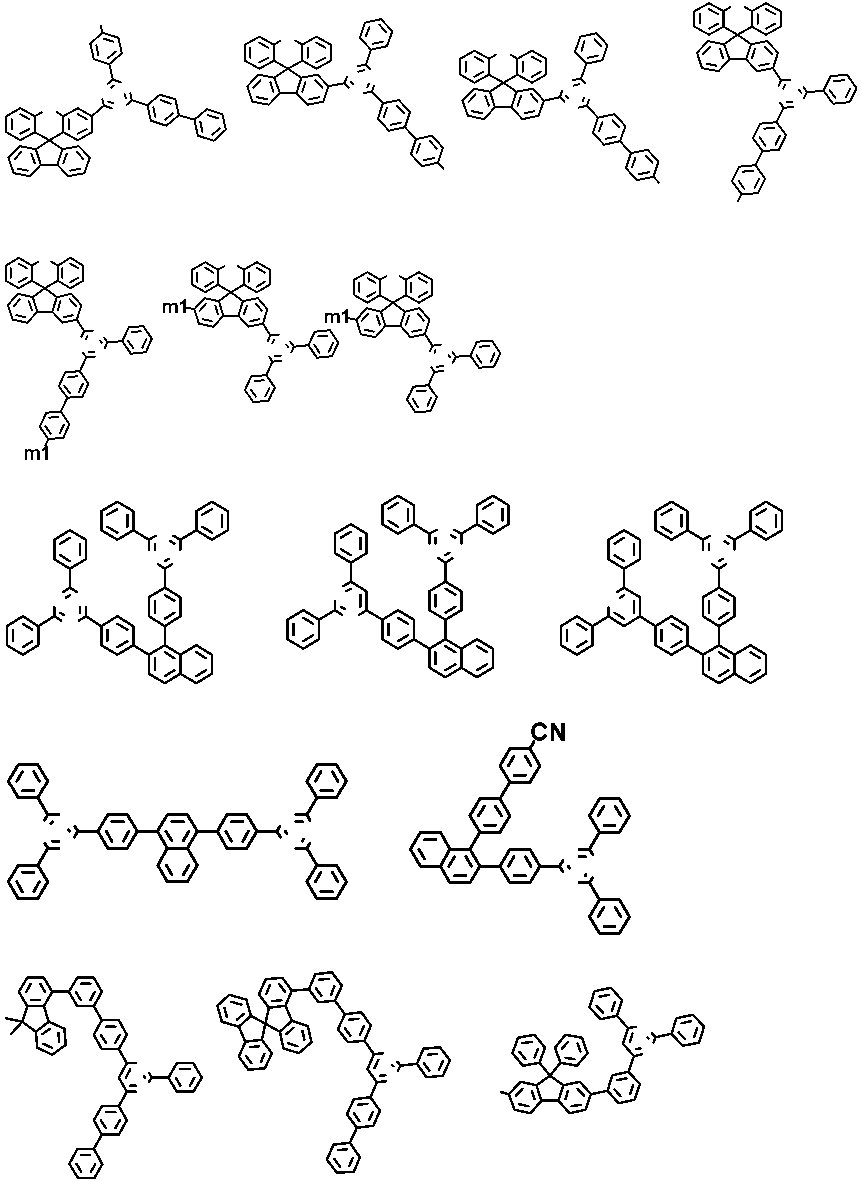

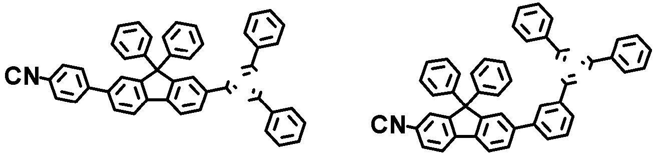

An embodiment may be an organic light emitting diode including a light emitting layer as an organic layer.

Another embodiment may be an organic light emitting diode including a light emitting layer and a hole transport region as an organic layer.

Another embodiment may be an organic light emitting diode including a light emitting layer and an electron transport region as an organic layer.

An organic light emitting diode according to an embodiment of the present invention may include a hole transport region 140 and an electron transport region 150 in addition to a light emitting layer 130 as an organic layer 105 as shown in FIG. 1.

Meanwhile, the organic light emitting diode may additionally include an electron injection layer (not shown), a hole injection layer (not shown), and the like, in addition to the light emitting layer as the aforementioned organic layer.

The organic light emitting diode 100 may be manufactured by forming an anode or a cathode on a substrate, and then forming an organic layer by a dry film method such as vacuum deposition, sputtering, plasma plating and ion plating, and forming a cathode or an anode thereon.

The aforementioned organic light emitting diode may be applied to an organic light emitting display device.

### [Mode for Invention]

Hereinafter, the embodiments are illustrated in more detail with reference to examples. However, these examples are exemplary, and the present scope is not limited thereto.

Hereinafter, starting materials and reactants used in Examples and Synthesis Examples were purchased from Sigma-Aldrich Co. Ltd., TCI Inc., Tokyo chemical industry, or P&H tech as far as there is no particular comment or were synthesized by known methods.

### (Synthesis of Compound for Organic Optoelectronic Device)

### Synthesis of First Compound

### Synthesis Example 1: Synthesis of Compound A-2

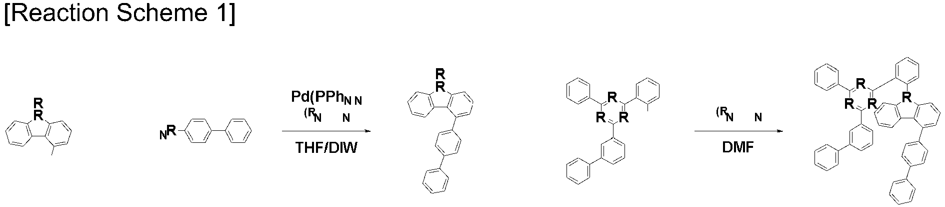

### a) Synthesis of Intermediate A-2-1

4-bromo-9H-carbazole (g, mmol), 4-biphenylboronic acid (33.4 g, 165.3 mmol), Pd(PPh₃)₄ (9.1 g, 7.9 mmol), and potassium carbonate (65.3 g, 472.3 mmol) are put in 800 mL of tetrahydrofuran : distilled water mixed solution and stirred under reflux for 12 hours. When the reaction is completed, purification by column chromatography (dichloromethane : n-hexane) was performed to obtain 38.8 g of Intermediate A-2-1 (Yield: 67.7 %).

### b) Synthesis of Compound A-2

Intermediate A-2-1 (3.0 g, 9.3 mmol), 2-(2-fluorophenyl)-9-phenyl-9H-carbazole (3.5 g, 102. mmol), and potassium carbonate (3.9 g, 27.9 mmol) are put in 500 mL of N,N-dimethylformamide and stirred under reflux for 12 hours. When the reaction is completed, purification by column chromatography (dichloromethane : n-hexane) was performed to obtain 4.6 g of Compound A-2 (Yield: 76.5 %).

### Synthesis Example 2 to Synthesis Example 10

A compound was synthesized through the final reaction (nucleophilic aromatic substitution) of Synthesis Example 1 using Reactant 1 and Reactant 2 as starting materials instead of 2-(2-fluorophenyl)-9-phenyl-9H-carbazole and Intermediate A-2-1.

Reactant 1 and Reactant 2 were synthesized through the same process as Synthesis Example 1 unless otherwise specified, and the deuterium-substituted structure was synthesized through the same process as Synthesis Example 1 after Reactant 1 underwent a deuterium substitution reaction according to the following Synthesis Example 11.

**(Table 1)**

| Synthesis Example | Reactant 1 | Reactant 2 | Product | Yield |
|---|---|---|---|---|
| Synthesis Example 2 | 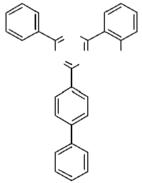 | 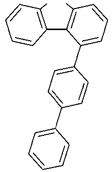 | 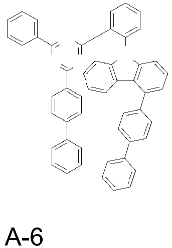 | 85.2 % |
| Synthesis Example 3 | 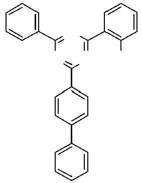 | 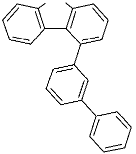 | 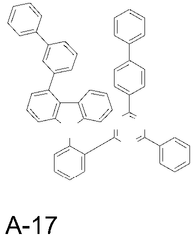 | 79.6 % |
| Synthesis Example 4 | 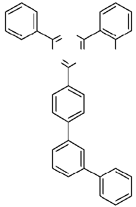 | 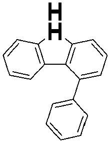 | 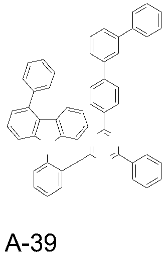 | 93.9 % |
| Synthesis Example 5 | 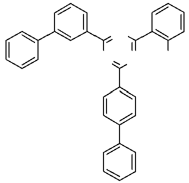 | 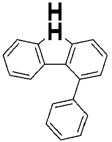 | 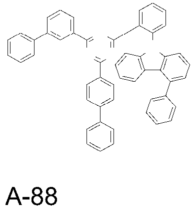 | 72.5 % |
| Synthesis Example 6 | 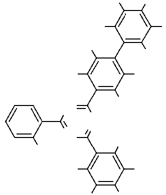 | 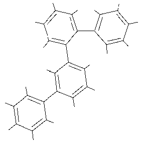 | 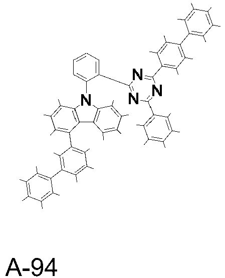 | 74.5 % |
| Synthesis Example 7 | 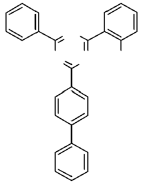 | 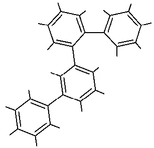 | 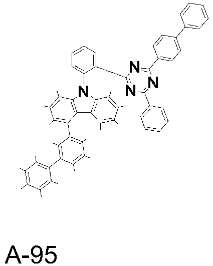 | 80.0 % |
| Synthesis Example 8 | 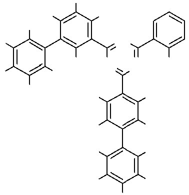 | 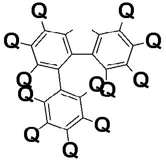 | 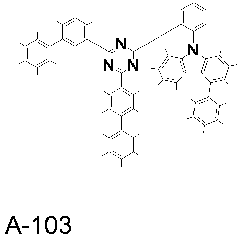 | 81.2 % |
| Synthesis Example 9 | 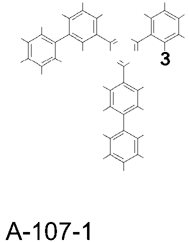 | 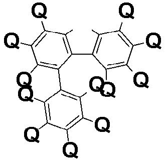 | 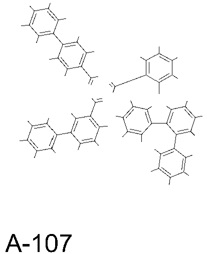 | 79.8 % |
| Synthesis Example 10 | 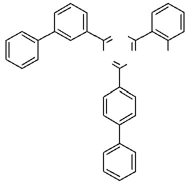 | 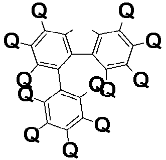 | 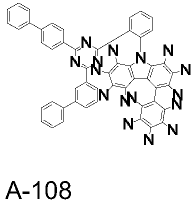 | 81.2 % |
| Comparative Synthesis Example 1 | 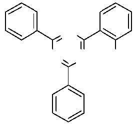 | 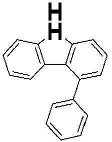 | 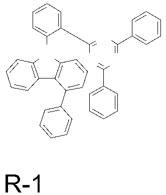 | 69.5 % |
| Comparative Synthesis Example 2 | 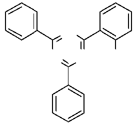 | 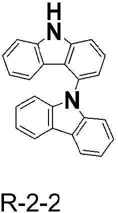 | 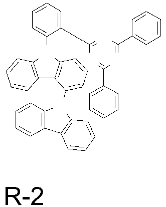 | 75.5 % |
| Comparative Synthesis Example 3 | 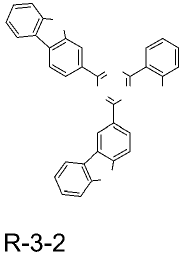 | 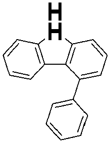 | 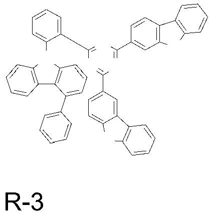 | 89.4 % |
| Comparative Synthesis Example 4 | 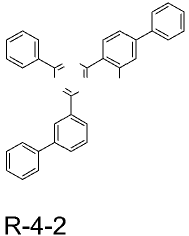 | 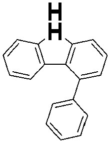 | 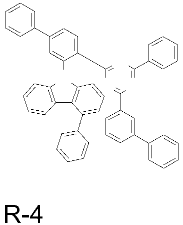 | 63.2 % |
| Comparative Synthesis Example 5 | 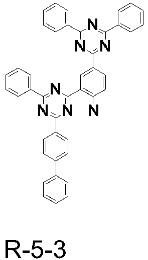 | 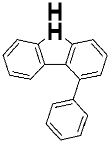 | 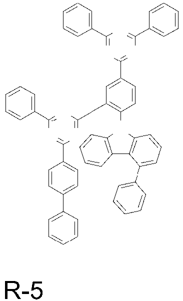 | 49.9 % |
| Comparative Synthesis Example 6 | 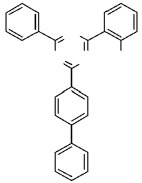 | 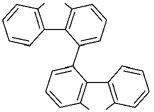 | 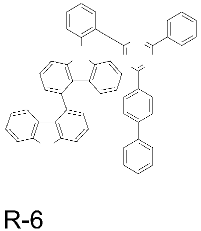 | 67.8 % |

### Synthesis Example 11: Synthesis of Compound A-107-1 (Deuterium Substitution Reaction)

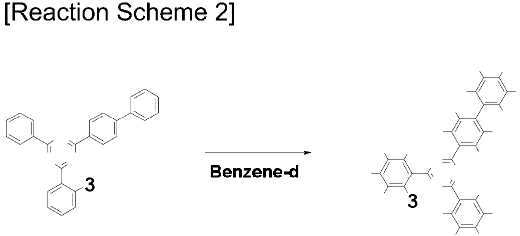

### a) Synthesis of Reactant A-107-1

2-([1,1'-biphenyl]-4-yl)-4-(2-fluorophenyl)-6-phenyl-1,3,5-triazine (30.0 g, 74 mmol), trifluoromethanesulfonic acid (55.8 g, 372 mmol), and benzene-d₆ (200 g, 2.38 mol) are put and stirred at 80°C for 24 hours. Purified water is added and neutralized with saturated K₃PO₄ solution. The organic layer was extracted and concentrated, and purified by recrystallization with a mixed solution of dichloromethane and n-hexane to obtain 27.9 g of Reactant A-107-1 (Yield: 88.9 %).

### Synthesis Example 12: Synthesis of Compound R-2-2

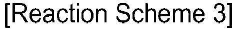

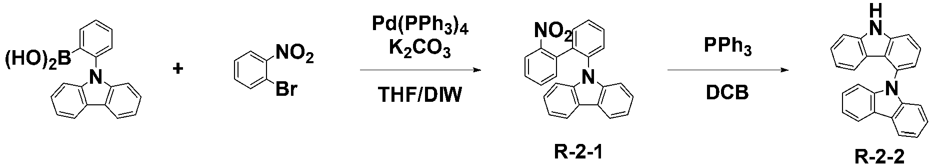

### a) Synthesis of Intermediate R-2-1

After synthesizing in the same manner as the synthesis method of Intermediate A-2-1 of Synthesis Example 1 using (2-(9H-carbazol-9-yl)phenyl)boronic acid and 1-bromo-2-nitrobenzene as starting materials, 51.5 g (Yield: 78.9 %) of Intermediate R-2-1 was obtained by purification by recrystallization with a mixed solution of dichloromethane and n-hexane.

### b) Synthesis of Compound R-2-2

Intermediate R-2-1 (51.5 g, 141 mmol) and triphenylphosphine (111.2 g, 424 mmol) are put in 700 mL of 1,2-dichlorobenzene and stirred under reflux for 16 hours. When the reaction is completed, purification by column chromatography (dichloromethane : n-hexane) was performed to obtain 27.4 g of Compound R-2-2 (Yield: 58.4 %).

### Synthesis Example 13: Synthesis of Compound R-3-2

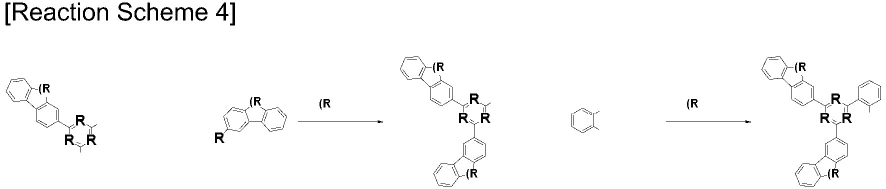

### a) Synthesis of Intermediate R-3-1

Synthesis was performed in the same manner as the synthesis method of Intermediate A-2-1 of Synthesis Example 1 using 2,4-dichloro-6-(dibenzo[b,d]furan-3-yl)-1,3,5-triazine and dibenzo[b,d] furan-2-ylboronic acid as starting materials. 25.0 g of Intermediate R-3-1 (Yield: 70.5 %) was obtained by purification by recrystallization with toluene.

### b) Synthesis of Compound R-3-2

Synthesis was performed in the same manner as the synthesis method of Intermediate A-2-1 of Synthesis Example 1 using Intermediate R-3-1 and 2-fluorophenylboronic acid as starting materials. 15.4 g of Compound R-3-2 (Yield: 88.3 %) was obtained by purification by recrystallization with a mixed solution of dichloromethane and n-hexane.

### Synthesis Example 14: Synthesis of Compound R-4-2

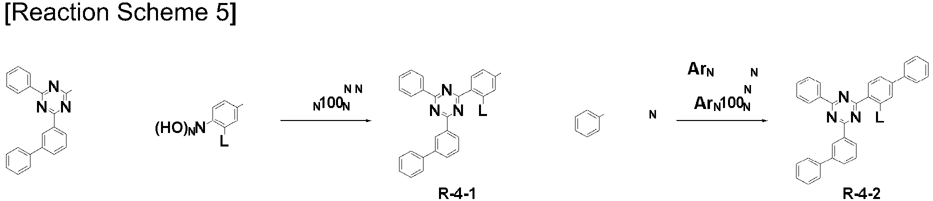

### a) Synthesis of intermediate R-4-1

Synthesis was performed in the same manner as the synthesis method of Intermediate A-2-1 of Synthesis Example 1 using 2-([1,1'-biphenyl]-3-yl)-4-chloro-6-phenyl-1,3,5-triazine and (4-chloro-2-fluorophenyl)boronic acid as starting materials. 35.5 g of Intermediate R-3-1 (Yield: 80.0 %) was obtained by purification by recrystallization with a toluene : acetone mixed solution.

### b) Synthesis of compound R-4-2

Intermediate R-4-1 (35.5 g, 81 mmol), phenylboronic acid (10.4 g, 85 mmol), Pd₂(dba)₃ (2.2 g, 2.4 mmol), P(t-Bu)₃ solution (1.5 g, 7.3 mmol), and cesium carbonate (52.8 g, 162 mmol) are dissolved in 270 mL of dioxane and stirred under reflux at 110 °C for 12 hours. When the reaction is completed, the solvent was removed by rotary evaporation, and 26.9 g of compound R-4-2 (Yield: 69.3 %) was obtained by purification by recrystallization with toluene.

### Synthesis Example 15: Synthesis of Compound R-5-3

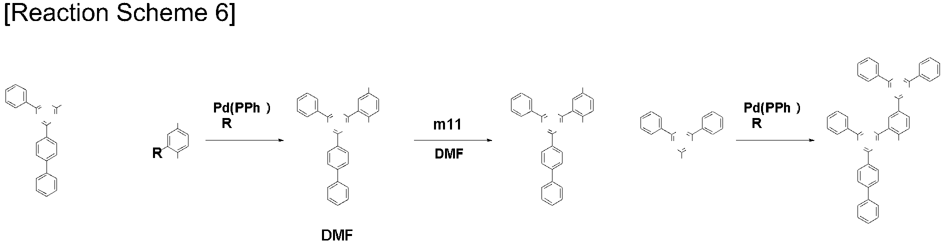

### a) Synthesis of intermediate R-5-1

Synthesis was performed in the same manner as the synthesis method of Intermediate A-2-1 of Synthesis Example 1 using 2-([1,1'-biphenyl]-4-yl)-4-chloro-6-phenyl-1,3,5-triazine and (5-chloro-2-fluorophenyl)boronic acid as starting materials. 25.0 g of Intermediate R-5-1 (Yield: 68.7 %) was obtained by purification by recrystallization with a toluene : acetone mixed solution.

### b) Synthesis of intermediate R-5-2

Intermediate R-5-1 (25.0 g, 57 mmol), bis(pinacolato)diboron (18.8 g, 74 mmol), potassium acetate (16.8 g, 171 mmol), tricyclohexylphosphine (3.2 g, 11 mmol), and [1,1'-bis(diphenylphosphino)ferrocene]-dichloropalladium(II) (2.3 g, 2.9 mmol) were put in 285 mL of N,N-dimethylformamide and stirred under reflux at 150°C for 12 hours. When the reaction is completed, the solution is put in an excess of DIW to form a precipitate. The precipitate was filtered, dissolved by boiling in toluene, and filtered through silica gel. After all solvent of the filtered solution was removed, 23.8 g of intermediate R-5-2 (78.9 %) was obtained by recrystallization with a mixed solution of dichloromethane and n-hexane.

### c) Synthesis of Compound R-5-3

Synthesis was performed in the same manner as the synthesis method of Intermediate A-2-1 of Synthesis Example 1 using intermediate R-5-2 and 2-chloro-4,6-diphenyl-1,3,5-triazine as starting materials. 16.5 g of Compound R-5-3 (Yield: 78.5 %) was obtained by purification by recrystallization with monochlorobenzene.

### Synthesis of Second Compound

### Synthesis Example 16: Synthesis of Compound B-136

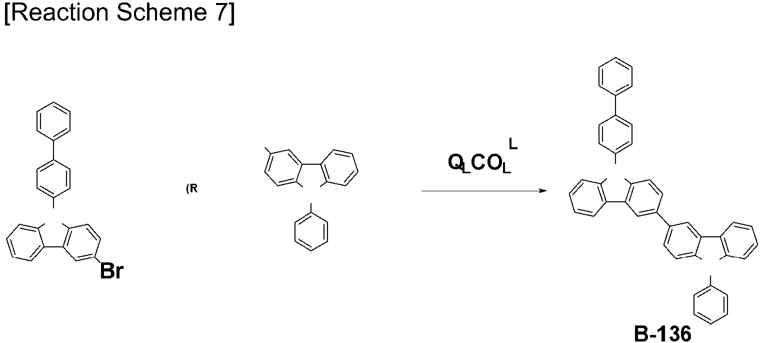

Synthesis was performed in the same manner as the synthesis method of Intermediate A-2-1 of Synthesis Example 1 using 9-([1,1'-biphenyl]-4-yl)-3-bromo-9H-carbazole and 9-phenyl-3-(4,4,5,5-tetramethyl-1,3,2-dioxaborolan-2-yl)-9H-carbazole as starting materials. 20.2 g of Compound B-136 (Yield: 62.1 %) was obtained by purification by recrystallization with a toluene : acetone mixed solution.

### Synthesis Example 17: Synthesis of Compound C-4

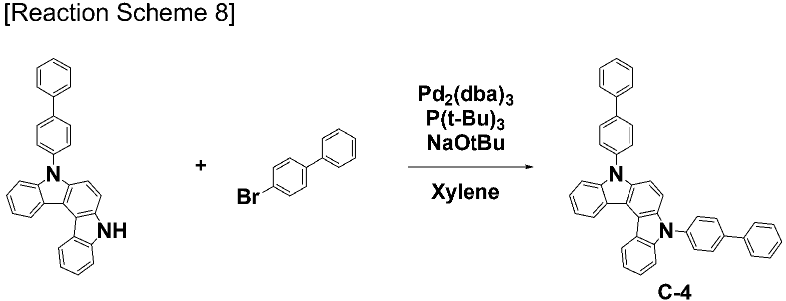

5-([1,1'-biphenyl]-4-yl)-5,8-dihydroindolo[2,3-c]carbazole (20.0 g, 49 mmol), 4-bromobiphenyl (12.0 g, 51 mmol), sodium t-butoxide (7.1 g, 73 mmol), and tri-tert-butylphosphine (1.5 g, 7 mmol) are dissolved in 250 ml of xylene, and Pd₂(dba)₃ (2.2 g, 2 mmol) is put therein, followed by being stirred under reflux for 12 hours under nitrogen atmosphere. After the reaction was finished, extraction was performed with xylene and distilled water, and then the organic layer was dried/filtered with anhydrous magnesium sulfate and the filtrate was concentrated under reduced pressure. Thereafter, purification by column chromatography with dichloromethane : n-hexane was performed to obtain 24.0 g of Compound C-4 (Yield: 87.3%).

### Synthesis Example 18: Synthesis of Compound C-5

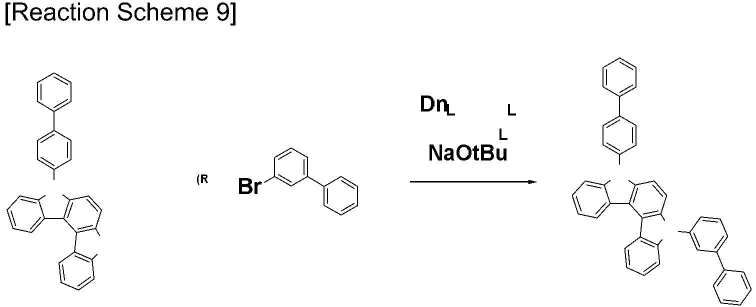

### a) Synthesis of Compound C-5

Synthesis was performed in the same manner as the synthesis method of Compound C-4 of Synthesis Example 17 using 5-([1,1'-biphenyl]-4-yl)-5,8-dihydroindolo[2,3-c]carbazole and 3-bromobiphenyl as starting materials. Purification by column chromatography with dichloromethane : n-hexane was performed to obtain 10.6 g of Compound C-5 (Yield: 69.2 %).

### Synthesis Example 19: Synthesis of Compound D-1

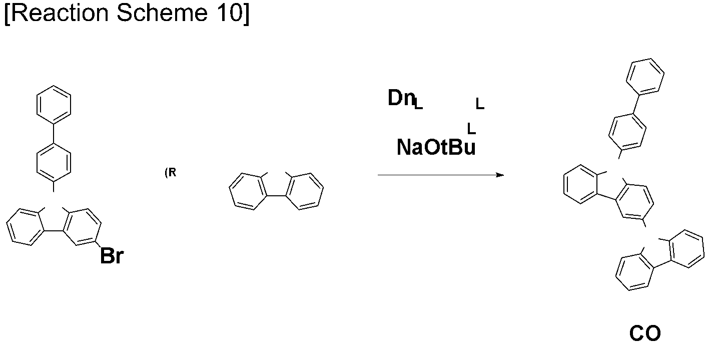

### a) Synthesis of Compound D-1

Synthesis was performed in the same manner as the synthesis method of Compound C-4 of Synthesis Example 17 using 9-([1,1'-biphenyl]-4-yl)-3-bromo-9H-carbazole and 9H-carbazole as starting materials. Purification by column chromatography with dichloromethane : n-hexane was performed to obtain 8.7 g of Compound D-1 (Yield: 45.2 %).

### Example 1: Manufacture of Green Organic Light Emitting Diode (Single Host)

A glass substrate coated with a thin film of ITO (indium tin oxide) was ultrasonically cleaned with distilled water. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. The prepared ITO transparent electrode was used as an anode, Compound A doped with 3% NDP-9 (Novaled GmbH) was vacuum-deposited on the ITO substrate to form a 100 A-thick hole injection layer, and Compound A is deposited on the hole injection layer to a thickness of 1350 Å to form a hole transport layer. Compound B was deposited on the hole transport layer to a thickness of 350 Å to form a hole transport auxiliary layer. On the hole transport auxiliary layer, Compound A-6 was used as a host and PhGD was doped at 7 wt% as a dopant to form a light emitting layer with a thickness of 400 Å by vacuum deposition. Subsequently, Compound C was deposited on the light emitting layer to a thickness of 50 Å to form an electron transport auxiliary layer, and Compound D and Liq were simultaneously vacuum-deposited in a weight ratio of 1:1 to form an electron transport layer with a thickness of 300 Å. LiQ (15 Å) and Al (1200 Å) were sequentially vacuum-deposited on the electron transport layer to form a cathode, thereby manufacturing an organic light emitting diode.

It was manufactured with a structure of ITO/Compound A (3% NDP-9 doping, 100 Å)/Compound A (1350 Å)/Compound B (350 Å)/EML [Host (Compound A-6) : PhGD = 93 wt% : 7 wt%] (400 Å)/Compound C (50 Å)/Compound D:LiQ (300 Å)/LiQ (15 A)/Al (1200 Å).
Compound A: N-(biphenyl-4-yl)-9,9-dimethyl-N-(4-(9-phenyl-9H-carbazol-3-yl)phenyl)-9H-fluoren-2-amine
Compound B: N-[4-(4-Dibenzofuranyl)phenyl]-N-[4-(9-phenyl-9H-fluoren-9-yl)phenyl][1,1'-biphenyl]-4-amine
Compound C: 2,4-Diphenyl-6-(4',5',6'-triphenyl[1,1":2',1":3",1‴:3‴,1ʺʺ-quinquephenyl]-3""-yl)-1,3,5-triazine
Compound D: 2-(1,1'-Biphenyl-4-yl)-4-(9,9-diphenylfluoren-4-yl)-6-phenyl-1,3,5-triazine

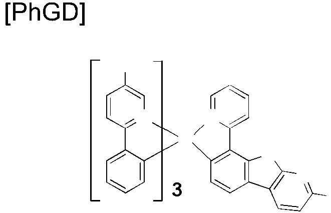

### Examples 2 to 9 and Comparative Examples 1 to 6

Organic light emitting diodes were manufactured in the same method as in Example 1 except for changing to compositions listed in Table 2 below.

### Example 10: Manufacture of Green Organic Light Emitting Diode (Mixed Host)

A glass substrate coated with a thin film of ITO (indium tin oxide) was ultrasonically cleaned with distilled water. After washing with the distilled water, the glass substrate was washed with a solvent such as isopropyl alcohol, acetone, methanol, and the like ultrasonically and dried and then, moved to a plasma cleaner, cleaned by using oxygen plasma for 10 minutes, and moved to a vacuum depositor. The prepared ITO transparent electrode was used as an anode, Compound E doped with 3% NDP-9 (Novaled GmbH) was vacuum-deposited on the ITO substrate to form a 100 A-thick hole injection layer, and Compound E is deposited on the hole injection layer to a thickness of 1350 Å to form a hole transport layer. Compound F was deposited on the hole transport layer to a thickness of 320 Å to form a hole transport auxiliary layer. On the hole transport auxiliary layer, Compound A-6 and Compound B-136 were simultaneously used as hosts in a weight ratio of 3:7 and PtGD was doped at 15 wt% as a dopant to form a light emitting layer with a thickness of 380 Å by vacuum deposition. Subsequently, Compound G was deposited on the light emitting layer to a thickness of 50 Å to form an electron transport auxiliary layer, and Compound H and Liq were simultaneously vacuum-deposited in a weight ratio of 1:1 to form an electron transport layer with a thickness of 300 Å. LiQ (15 Å) and Al (1200 Å) were sequentially vacuum-deposited on the electron transport layer to form a cathode, thereby manufacturing an organic light emitting diode.

It was manufactured with a structure of ITO/Compound E (3% NDP-9 doping, 100Å)/Compound E (1350 Å)/Compound F (320 Å)/EML [Host (Compound A-6 : Compound B-136 = 3 : 7 wt%/wt%) : PtGD = 85 wt% : 15 wt%] (380 Å)/Compound G (50 Å)/Compound H: LiQ (300 Å)/LiQ (15 Å)/Al (1200 Â).
Compound E: N-(9,9-diphenyl-9H-fluoren-2-yl)-N,9-diphenyl-9H-carbazol-2-amine
Compound F: 9,9-dimethyl-N-[3-(9-phenyl-9H-fluoren-9-yl)phenyl]-4-(4-phenylphenyl)-9H-fluoren-2-amine
Compound G: 4-{4-[4-(9,9-dimethyl-9H-fluoren-4-yl)phenyl]phenyl}-2-phenyl-6-(4-phenylphenyl)pyrimidine
Compound H: 2-(4-{1-[4-(diphenyl-1,3,5-triazin-2-yl)phenyl]naphthalene-2-yl}-4,6-diphenyl-1,3,5-triazine

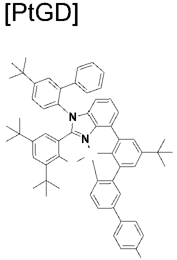

### Examples 11 to 20 and Comparative Examples 7 to 16

Organic light emitting diodes were manufactured in the same method as in Example 4 except for changing to compositions listed in Table 3 below.

### Evaluation

Driving voltage, luminous efficiency, and life-span characteristics of the organic light emitting diodes according to Examples 1 to 20 and Comparative Examples 1 to 16 were evalused.

Specific measurement methods are as follows, and the results are shown in Table 2 to Table 3.

### (1) Measurement of Current Density Change Depending on Voltage Change

The obtained organic light emitting diodes were measured regarding a current value flowing in the unit diode, while increasing the voltage from 0 V to 10 V using a current-voltage meter (Keithley 2400), and the measured current value was divided by area to provide the results.

### (2) Measurement of Luminance Change Depending on Voltage Change

Luminance was measured by using a luminance meter (Minolta Cs-1000A), while the voltage of the organic light emitting diodes was increased from 0 V to 10 V.

### (3) Measurement of Luminous Efficiency

The luminance and the current density measured from (1) and (2) above and a voltage were used to calculate the current efficiency(cd/A) at the same current density (10 mA/cm²).

Luminous efficiency values of Examples 1 to 9 and Comparative Examples 1 to 6 were calculated as relative values based on Comparative Example 1 and listed in Table 3 below.

The luminous efficiency values of Examples 10 to 20 and Comparative Examples 7 to 16 were calculated as relative values based on Comparative Example 8 and listed in Table 2 below.

### (4) Measurement of Life-span

The results were obtained by maintaining the luminance (cd/m²) at 24000 cd/m² and measuring the time for the current efficiency (cd/A) to decrease to 97%.

The life-span measurement values of Examples 1 to 9 and Comparative Examples 1 to 6 were calculated as relative values based on Comparative Example 1 and listed in Table 2 below.

The life-span measurement values of Examples 10 to 20 and Comparative Examples 7 to 16 were calculated as relative values based on Comparative Example 8 and listed in Table 3 below.

### (5) Measurement of Driving Voltage

The driving voltage of each diode was measured at 15 mA/cm² using a current-voltage meter (Keithley 2400) to obtain results.

The driving voltages of Examples 1 to 9 and Comparative Examples 1 to 6 were calculated as relative values based on Comparative Example 1 and listed in Table 2 below.

The driving voltages of Examples 10 to 20 and Comparative Examples 7 to 16 were calculated as relative values based on Comparative Example 8 and listed in Table 3 below.

**(Table 2)**

| No. | Compound | Driving Voltage (%) | Luminous efficiency (%) |
|---|---|---|---|
| Example 1 | A-6 | 94 | 106 |
| Example 2 | A-17 | 94 | 106 |
| Example 3 | A-39 | 95 | 105 |
| Example 4 | A-88 | 94 | 107 |
| Example 5 | A-94 | 94 | 106 |
| Example 6 | A-95 | 94 | 106 |
| Example 7 | A-103 | 94 | 107 |
| Example 8 | A-107 | 94 | 107 |
| Example 9 | A-108 | 94 | 107 |
| Comparative Example 1 | R-1 | 100 | 100 |
| Comparative Example 2 | R-2 | 102 | 99 |
| Comparative Example 3 | R-3 | 97 | 95 |
| Comparative Example 4 | R-4 | 101 | 95 |
| Comparative Example 5 | R-5 | 94 | 80 |
| Comparative Example 6 | R-6 | 96 | 101 |

**(Table 3)**

| No. | Compound | | Driving voltage (%) | Luminous efficiency (%) | Life-span (%) |
|---|---|---|---|---|---|
| | First compound | Second compound | | | |
| Example 10 | A-6 | C-4 | 94 | 103 | 190 |
| Example 11 | A-6 | C-5 | 95 | 105 | 170 |
| Example 12 | A-17 | C-4 | 94 | 103 | 185 |
| Example 13 | A-39 | C-4 | 95 | 103 | 220 |
| Example 14 | A-88 | C-4 | 93 | 104 | 180 |
| Example 15 | A-94 | C-4 | 94 | 103 | 260 |
| Example 16 | A-95 | C-4 | 94 | 103 | 220 |
| Example 17 | A-103 | C-4 | 93 | 104 | 250 |
| Example 18 | A-107 | C-4 | 93 | 104 | 250 |
| Example 19 | A-108 | C-4 | 93 | 104 | 200 |
| Example 20 | A-108 | C-5 | 94 | 106 | 190 |
| Comparative Example 7 | R-1 | B-136 | 112 | 103 | 40 |
| Comparative Example 8 | R-1 | C-4 | 100 | 100 | 100 |
| Comparative Example 9 | R-1 | C-5 | 101 | 102 | 90 |
| Comparative Example 10 | R-1 | D-1 | 115 | 97 | 25 |
| Comparative Example 11 | R-2 | C-4 | 102 | 100 | 150 |
| Comparative Example 12 | R-3 | C-4 | 94 | 98 | 65 |
| Comparative Example 13 | R-4 | C-4 | 101 | 98 | 150 |
| Comparative Example 14 | R-5 | B-136 | 97 | 93 | 10 |
| Comparative Example 15 | R-5 | C-4 | 92 | 90 | 10 |
| Comparative Example 16 | R-6 | C-4 | 95 | 102 | 80 |

Referring to Table 1 and Table 2, it can be confirmed that the organic light emitting diodes according to Examples 1 to 20 exhibited significantly improved driving voltage, luminous efficiency, and life-span characteristics compared to the organic light emitting diodes according to Comparative Examples 1 to 16. In particular, it was confirmed that when biphenyl or more is introduced into triazine, an effect of reducing the driving voltage was exhibited, and when biphenyl or terphenyl is introduced into carbazole, life-span was improved.

While this disclosure has been described in connection with what is presently considered to be practical example embodiments, it is to be understood that the invention is not limited to the disclosed embodiments, but, on the contrary, is intended to cover various modifications and equivalent arrangements included within the spirit and scope of the appended claims.

## Claims

1. A compound for an organic optoelectronic device represented by Chemical Formula 1: 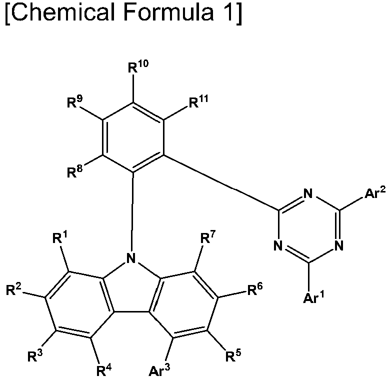 wherein, in Chemical Formula 1,
Ar¹ and Ar² are each independently a substituted or unsubstituted C6 to C30 aryl group,
Ar³ is a substituted or unsubstituted C6 to C30 aryl group,
at least one of Ar¹ to Ar³ is a substituted or unsubstituted C10 to C30 aryl group,
R¹ to R⁷ are each independently hydrogen, deuterium, a cyano group, a halogen, a substituted or unsubstituted C1 to C30 alkyl group, or a substituted or unsubstituted C6 to C30 aryl group, and
R⁸ to R¹¹ are each independently hydrogen, deuterium, a cyano group, a halogen, or a substituted or unsubstituted C1 to C30 alkyl group.

2. The compound for the organic optoelectronic device as claimed in claim 1, wherein
Ar¹ and Ar² are each independently a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, or a substituted or unsubstituted triphenylene group, and
at least one of Ar¹ and Ar² is a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, or a substituted or unsubstituted triphenylene group.

3. The compound for the organic optoelectronic device as claimed in claim 1, wherein
Ar¹ and Ar² are each independently a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, or a substituted or unsubstituted triphenylene group, and
Ar³ is a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted fluorenyl group, or a substituted or unsubstituted triphenylene group.

4. The compound for the organic optoelectronic device as claimed in claim 1, wherein
the compound is one selected from compounds listed in Group 1: 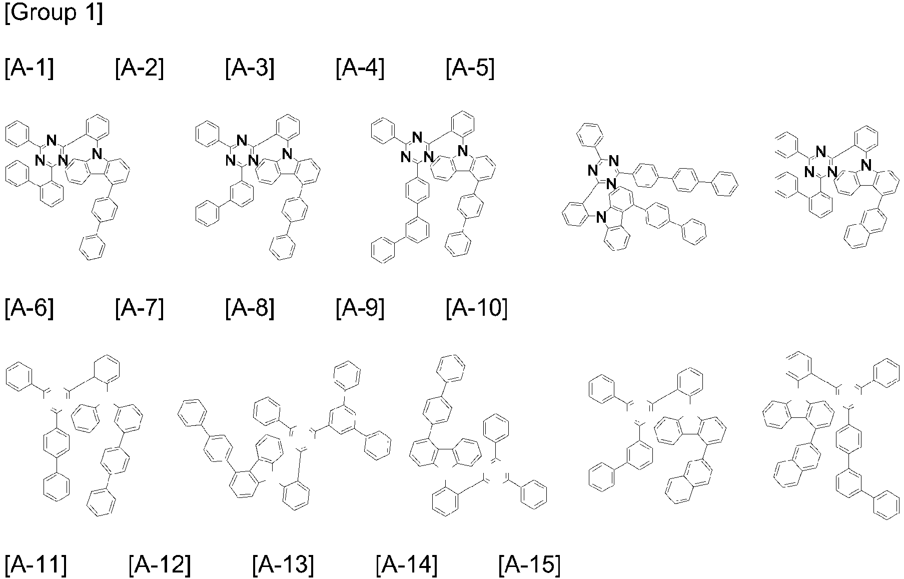 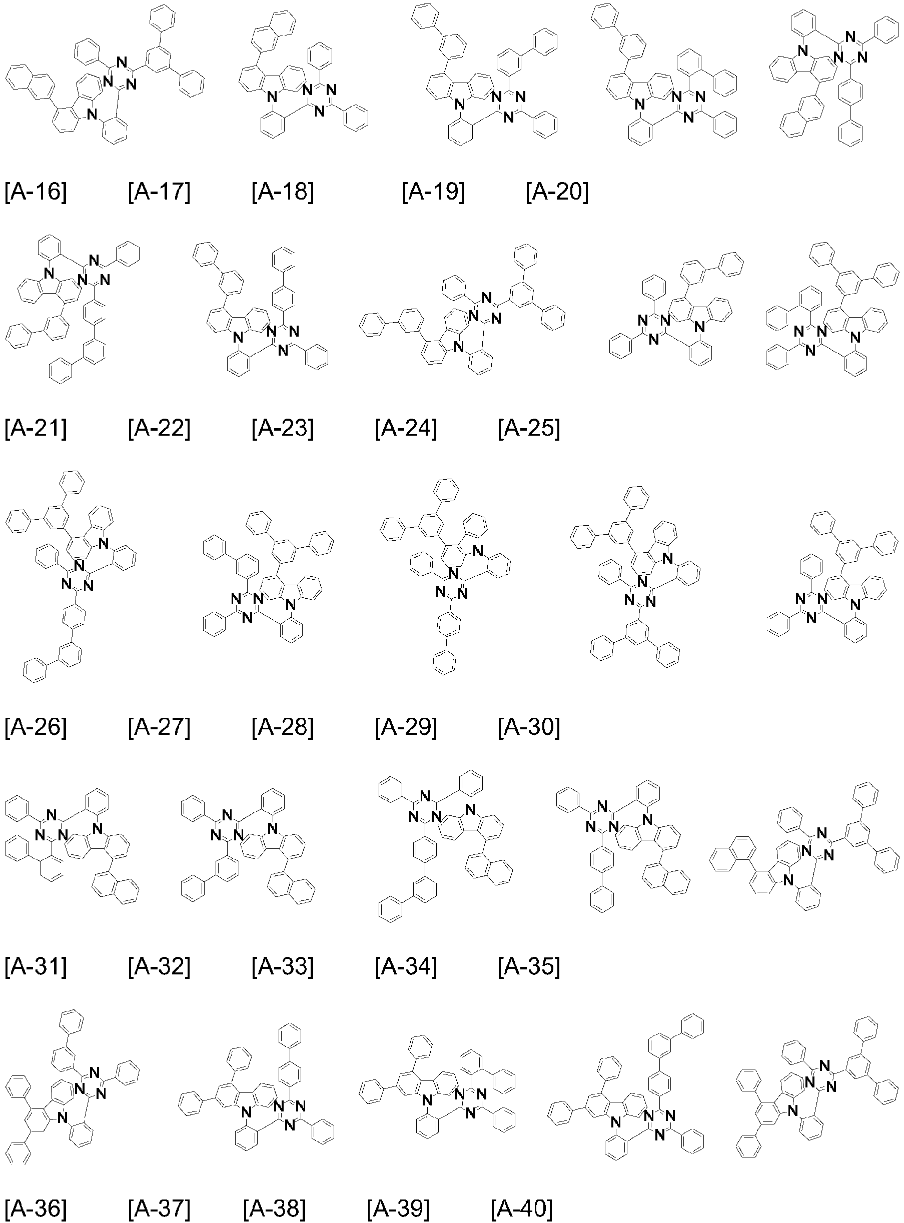 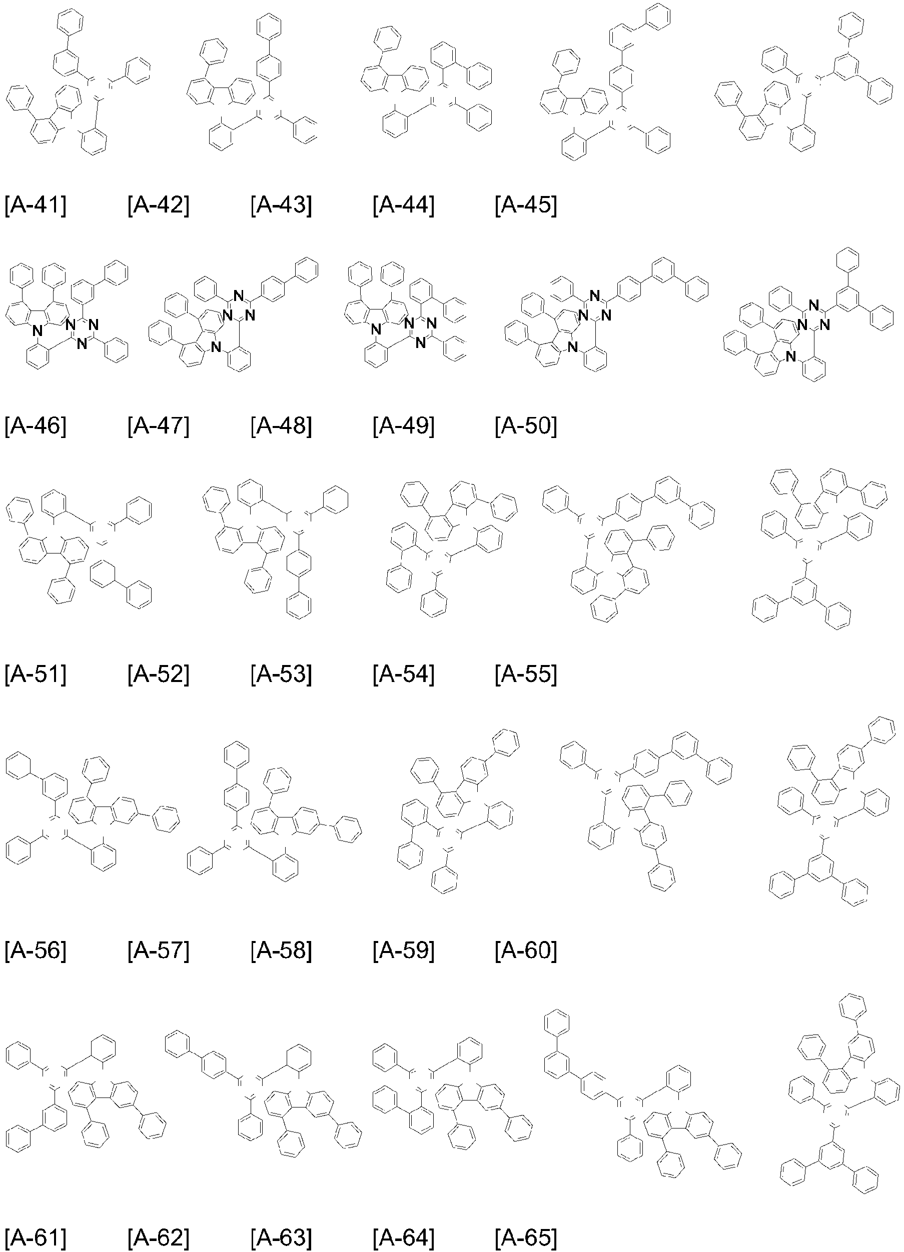 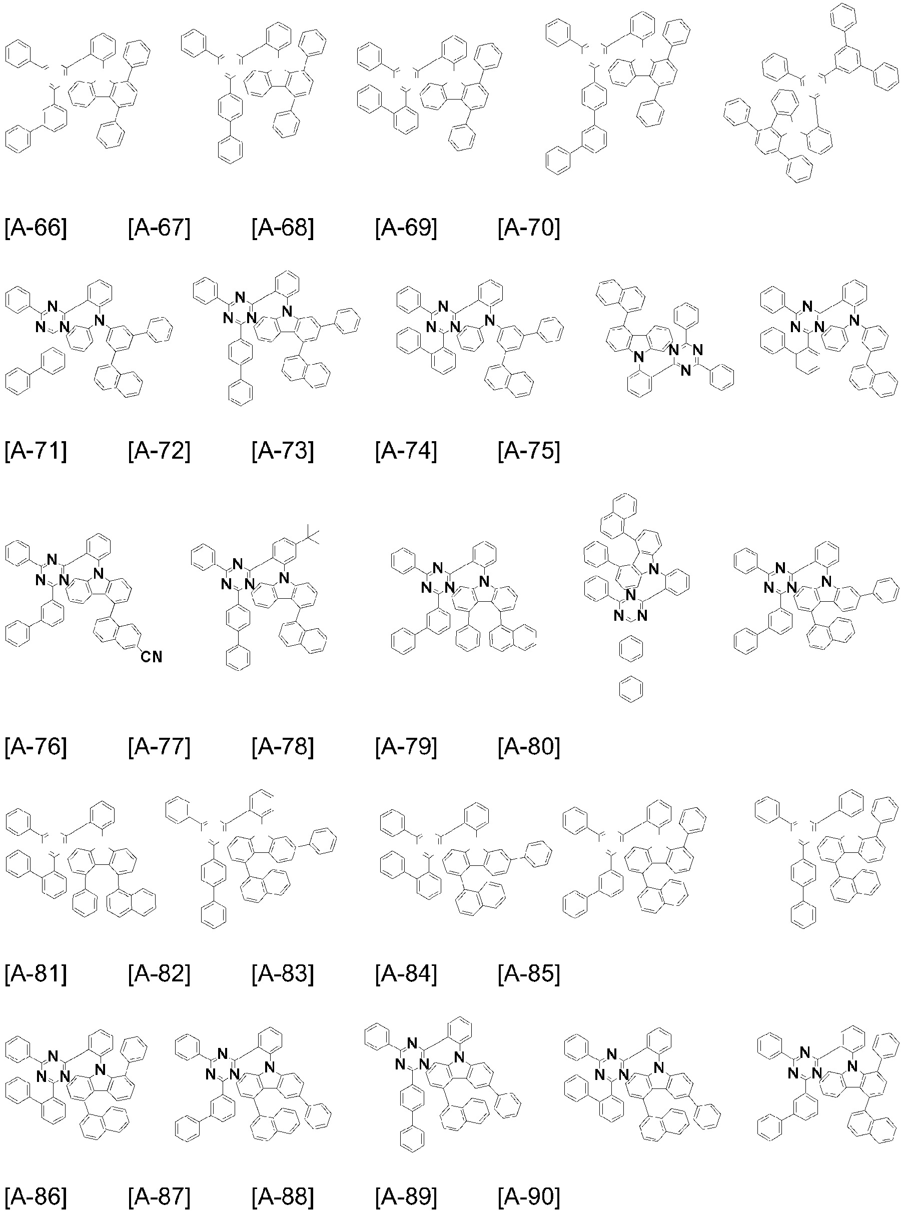 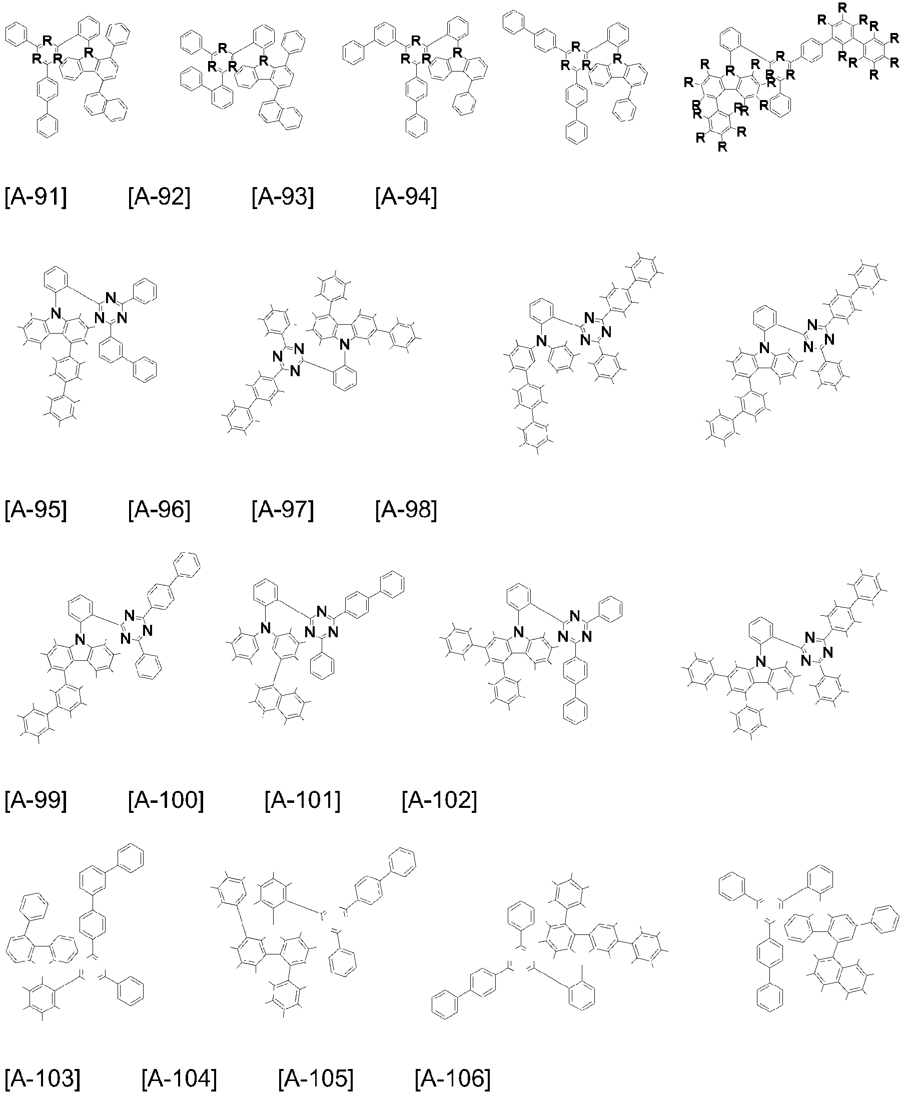 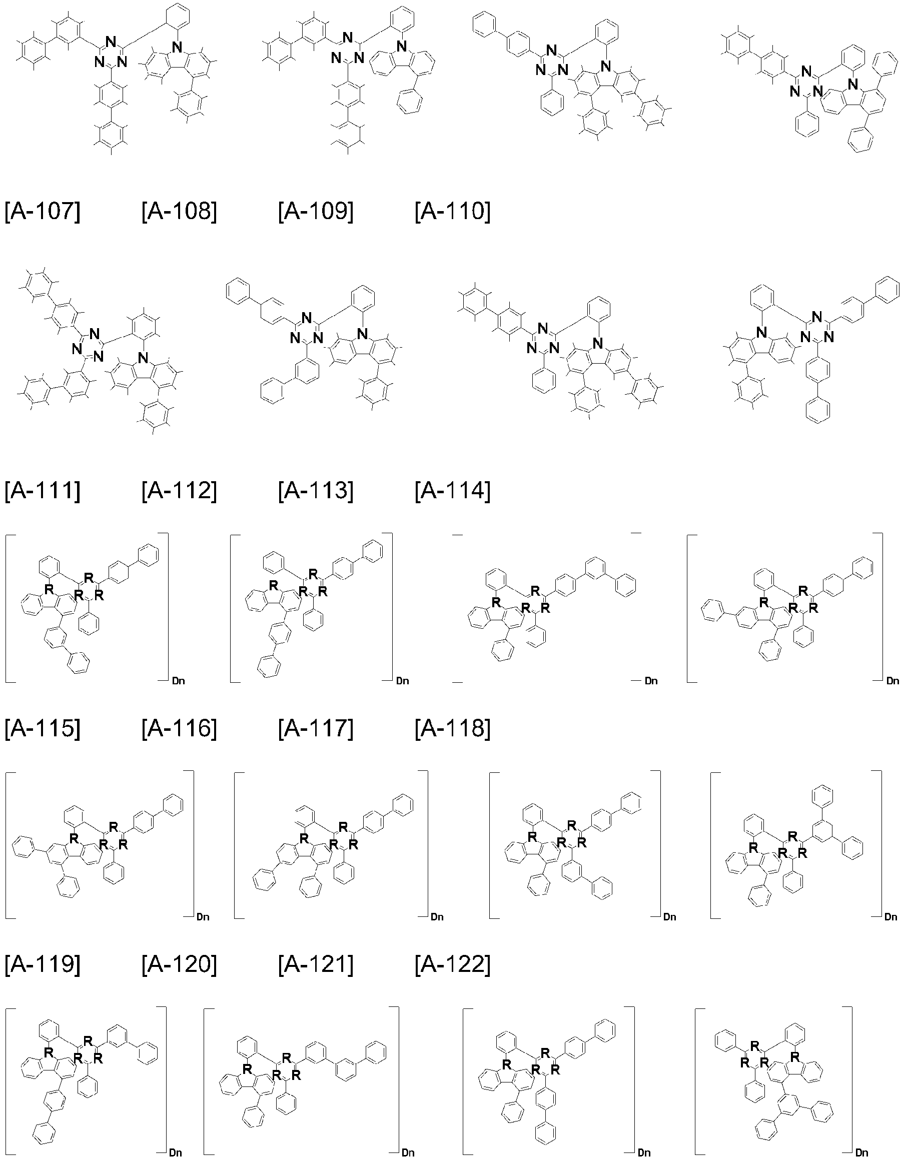
(Dn refers to the number of deuterium substitutions and indicates a structure substituted with one or more deuteriums).

5. A composition for an organic optoelectronic device, comprising
a first compound and a second compound,
wherein the first compound is the compound for the organic optoelectronic device according to claim 1, and
the second compound is represented by Chemical Formula 2; a combination of Chemical Formula 3 and Chemical Formula 4; or Chemical Formula 5: 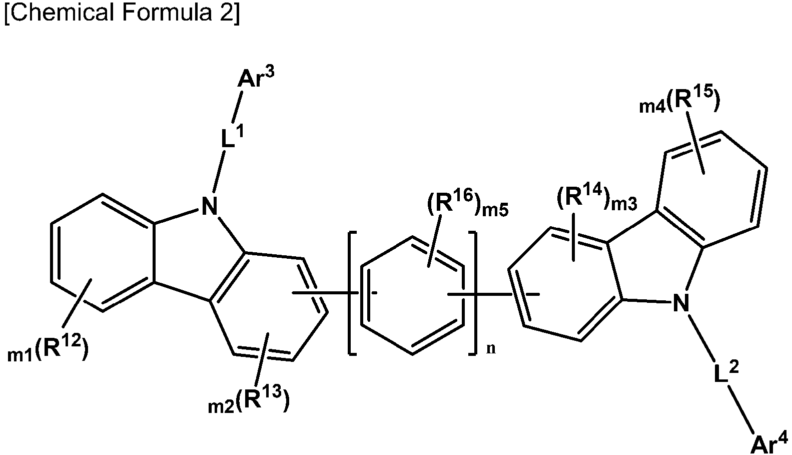
wherein, in Chemical Formula 2,
R¹² to R¹⁶ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar³ and Ar⁴ are each independently a substituted or unsubstituted C6 to C20 aryl group or a substituted or unsubstituted C2 to C30 heterocyclic group,
L¹ and L² are each independently a single bond or a substituted or unsubstituted C6 to C20 arylene group,
m1, m4, and m5 are each independently one of integers from 1 to 4,
m2 and m3 are each independently one of integers from 1 to 3, and
n is one of integers from 0 to 2; 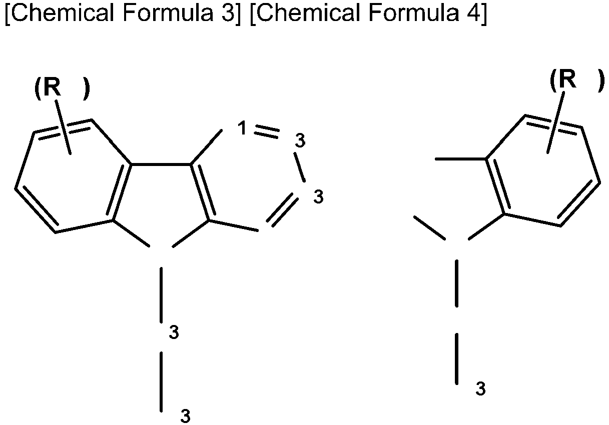
wherein, in Chemical Formula 3 and Chemical Formula 4,
among a₁* to a₄* in Chemical Formula 3, two adjacent ones are each a linking carbon (C) that is linked to *s in Chemical Formula 4,
among a₁* to a₄* of Chemical Formula 3, the remaining two that are not linked to Chemical Formula 4 are C-L^{a}-R^{a},
L^{a}, L³, and L⁴ are each independently a single bond or a substituted or unsubstituted C6 to C20 arylene group,
R^{a}, R¹⁷, and R¹⁸ are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁵ and Ar⁶ are each independently a substituted or unsubstituted C6 to C20 aryl group or a substituted or unsubstituted C2 to C30 heterocyclic group, and
m6 and m7 are each independently one of integers from 1 to 4; 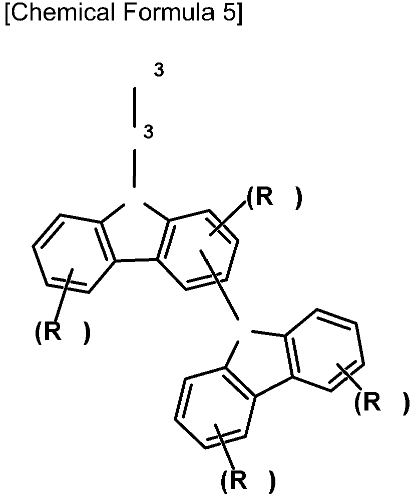
wherein, in Chemical Formula 5,
L⁵ is each independently a single bond or a substituted or unsubstituted C6 to C20 arylene group,
R¹⁹ to R²² are each independently hydrogen, deuterium, a cyano group, a halogen, a substituted or unsubstituted amine group, a substituted or unsubstituted C1 to C30 alkyl group, a substituted or unsubstituted C6 to C30 aryl group, or a substituted or unsubstituted C2 to C30 heterocyclic group,
Ar⁷ is a substituted or unsubstituted C6 to C20 aryl group or a substituted or unsubstituted C2 to C30 heterocyclic group,
m8, m10, and m11 are each independently one of integers from 1 to 4, and
m9 is one of integers from 1 to 3.

6. The composition for the organic optoelectronic device as claimed in claim 5, wherein
Chemical Formula 2 is represented by Chemical Formula 2-8: 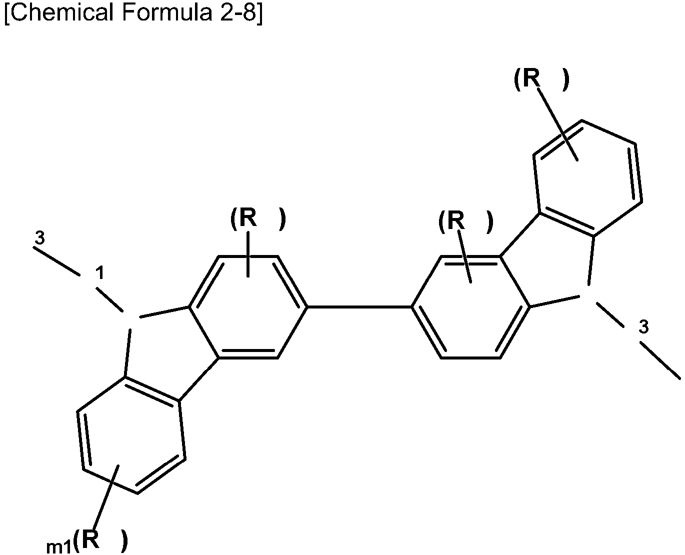
wherein, in Chemical Formula 2-8,
R¹² to R¹⁵ are each independently hydrogen, deuterium, or a substituted or unsubstituted C6 to C12 aryl group,
m1 and m4 are each independently one of integers from 1 to 4,
m2 and m3 are each independently one of integers from 1 to 3, and
L¹-Ar³ and L²-Ar⁴ are each independently one of the substituents listed in Group I , 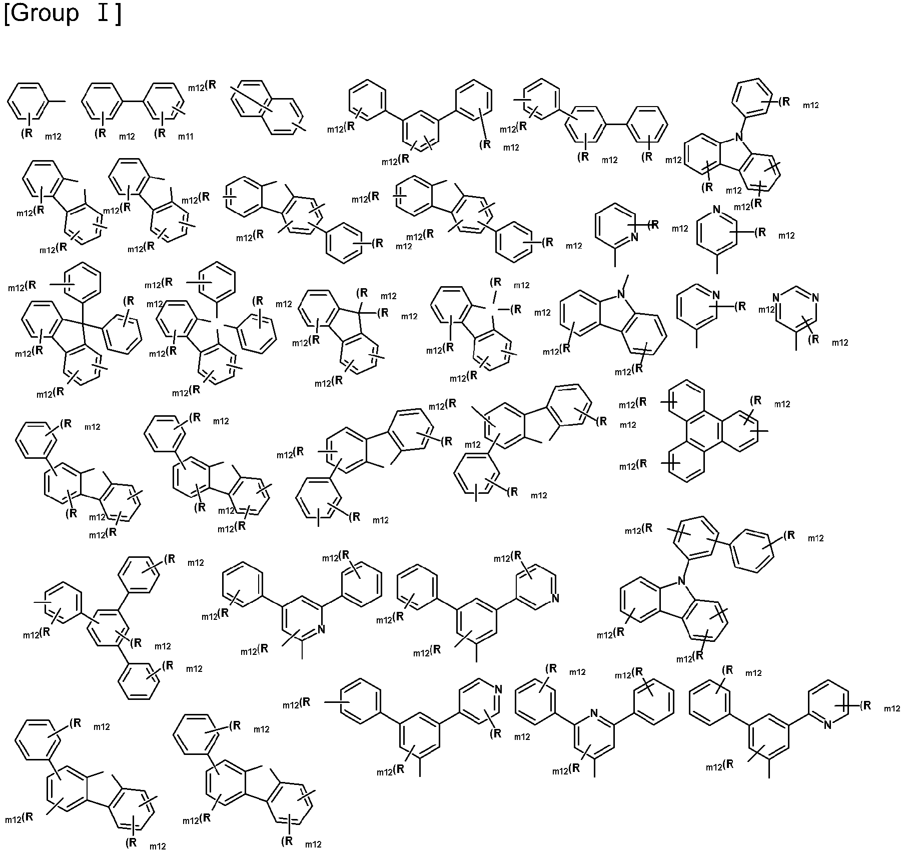
wherein, in Group I,
R²³ to R²⁷ are each independently hydrogen, deuterium, a cyano group, a C1 to C10 alkyl group, or a C6 to C12 aryl group,
m12 is one of integers from 1 to 5,
m13 is one of integers from 1 to 4,
m14 is one of integers from 1 to 3,
m15 is an integer of 1 or 2,
m16 is one of integers from 1 to 7, and
* is a linking point.

7. The composition for the organic optoelectronic device as claimed in claim 5, wherein
the combination of Chemical Formula 3 and Chemical Formula 4is represented by Chemical Formula 3C: 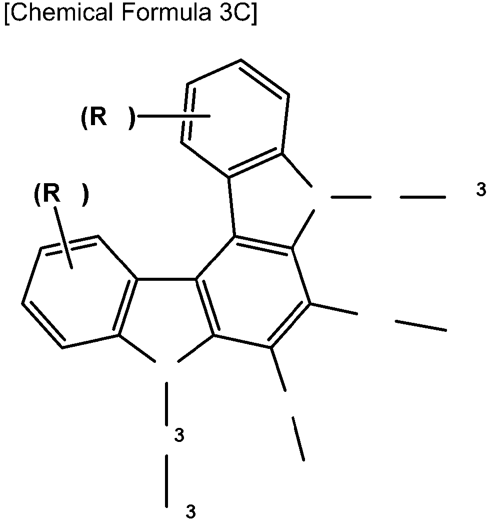
wherein, in Chemical Formula 3C,
L^{a3} and L^{a4} are a single bon,
R¹⁷, R¹⁸, R^{a3}, and R^{a4} are each independently hydrogen, deuterium or C6 to C12 aryl group,
m11 and m12 are each independently one of integers from 1 to 4, and
L³-Ar⁵ and L⁴-Ar⁶ are each independently one of the substituents listed in Group I , 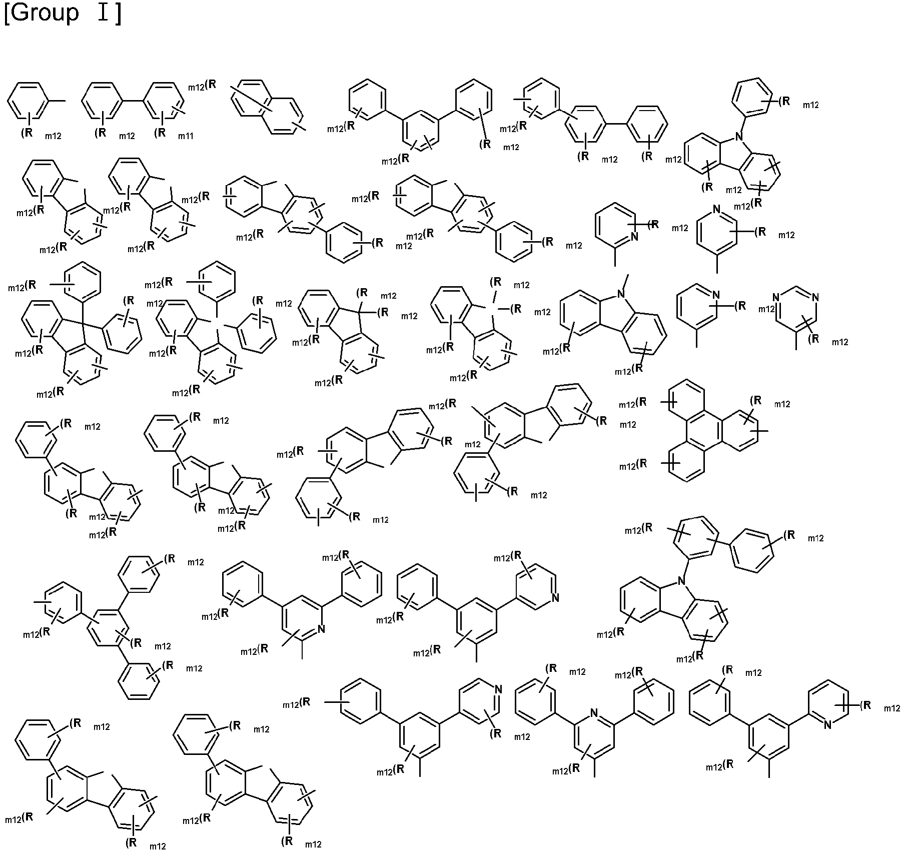
wherein, in Group I,
R²³ to R²⁷ are each independently hydrogen, deuterium, a cyano group, a C1 to C10 alkyl group, or a C6 to C12 aryl group,
m12 is one of integers from 1 to 5,
m13 is one of integers from 1 to 4,
m14 is one of integers from 1 to 3,
m15 is an integer of 1 or 2,
m16 is one of integers from 1 to 7, and
* is a linking point.

8. An organic optoelectronic device, comprising
an anode and a cathode facing each other, and
at least one organic layer positioned between the anode and the cathode,
wherein the organic layer comprises
the compound for the organic optoelectronic device as claimed in any one of claims 1 to 4; or
the composition for the organic optoelectronic device as claimed in any one of claims 5 to 7.

9. The organic optoelectronic device as claimed in claim 8, wherein
the organic layer comprises a light emitting layer, and
the light emitting layer comprises the compound for the organic optoelectronic device or the composition for the organic optoelectronic device.

10. A display device comprising the organic optoelectronic device as claimed in claim 8.
